# EUROPEAN PATENT APPLICATION

(11) **EP 4 752 125 A1**
(43) Date of publication of application: **03.06.2026**
(21) Application number: 24845187.4
(22) Date of filing: 28.05.2024
(51) Int. Cl.: C07C 17/093, C07C 22/04, C07C 23/02, C07C 23/20, C07C 41/01, C07C 43/174, C07C 67/307, C07C 69/635, C07C 247/10, C07C 269/06, C07C 271/14, C07C 309/80, C07C 309/84, C07C 311/01, C07C 319/14, C07C 321/28, C07D 211/38, C07D 285/15

(54) **AMINO GROUP ELIMINATING AGENT, REACTION PRODUCT PRODUCTION METHOD, REACTION PRECURSOR, REACTION PRECURSOR PRODUCTION METHOD, AND METHOD FOR PRODUCING OPTICALLY ACTIVE REACTION PRODUCT**

(30) Priority: 24.07.2023 JP 2023120055
(71) Applicant: Mitsubishi Materials Electronic Chemicals Co., Ltd., Akita-shi Akita 010-8585 (JP)
(72) Inventor: NEAGARI WATABE Yota, Akita-shi, Akita 010-8585 (JP); MATSUMURA Noriaki, Akita-shi, Akita 010-8585 (JP); KAMIYA Takeshi, Akita-shi, Akita 010-8585 (JP)
(74) Representative: Hoffmann Eitle
(86) International application number: PCT/JP2024/019601
(87) International publication number: WO 2025/022805

(57) **Abstract**

An amino group eliminating agent containing a compound (a) represented by the following Formula (a), which is used to produce a reaction product obtained by reacting an organic compound having an amino group with the amino group eliminating agent and additionally reacting the organic compound with a nucleophile:

Y¹-X¹-Rf-X²-Y² ··· (a)

in Formula (a), Rf is a substituted or unsubstituted divalent hydrocarbon group containing at least one fluorine atom; X¹ and X² are each independently a carbonyl group or a sulfonyl group; Y¹ and Y² are each independently a halogen atom or the like.

## Description

### TECHNICAL FIELD

The present invention relates to an amino group eliminating agent, a reaction product production method, a reaction precursor, a reaction precursor production method, and an optically active reaction product production method.

Priority is claimed on Japanese Patent Application No. 2023-120055, filed July 24, 2023, the content of which is incorporated herein by reference.

### BACKGROUND ART

Primary alkylamines having an amino group (NH₂ group) on an alkyl carbon atom are commonly found in natural products such as amino acids and alkaloids, as well as in commercially available organic compounds and compound libraries used in pharmaceutical synthesis, and are known as readily available compounds. In addition, structures having an amino group on an alkyl group are frequently observed in pharmaceuticals, agricultural chemicals, electronic materials, functional materials, and their raw materials and intermediates.

In the synthesis of a desired organic compound, reactions that convert a target functional group into a desired functional group are extremely useful. However, reactions that directly convert an amino group on an alkyl carbon atom into another functional group are generally more challenging than reactions that convert halogen or hydroxy groups. This difficulty is attributable, for example, to the high bond dissociation energy of C-N bonds and the inherent basicity of amines.

For example, as a classical reaction method for converting an amino group into another functional group, the Sandmeyer reaction is known. In this reaction, an amino group is converted into a diazonium salt, and a substituent such as chlorine, bromine, or iodine is then introduced. However, this reaction proceeds efficiently with aromatic amines, but it is difficult to apply to aliphatic amines.

Several functionalization reactions for aliphatic amines have been developed (Non Patent Document 1), including methods of converting an amino group into a pyridinium salt and then functionalizing it (Non Patent Documents 2 and 3), and methods of converting an aliphatic amine into an isodiazene through a reaction with an anomeric amide, then eliminating nitrogen molecules, and functionalizing it have been reported (Non Patent Documents 4 and 5). In addition, reagents such as trifluoromethanesulfonyl azide and fluorosulfuryl azide, which convert an amino group into an azide group, have also been developed (Non Patent Documents 6 and 7).

### CITATION LIST

### Non Patent Documents

Non Patent Document 1: Kathleen J. Berger; Mark D. Levin. Organic & Biomolecular Chemistry (2020), 19, 11-36.
Non Patent Document 2: Namek F. EWEISS; Alan R. KATRITZKY; Pai-Lin NIE, Christopher A. RAMSDEN. Synthesis (1977), 9, 634-635.
Non Patent Document 3: Felix J. R. Klauck; Michael J. James; Frank Glorius. Angewwandte Chemie International Edition (2017), 56(40), 12336-12339.
Non Patent Document 4: Jiang-Hao Xue; Yin Li; Dong-Hang Tan; Fang-Hai Tu; Yuan Liu; Qingjiang Li; Honggen Wang. iScience (2023), 26(c), 106255.
Non Patent Document 5: Balu D. Dherange; Mingbin Yuan; Christopher B. Kelly; Christopher A. Reiher; Cristina Grosanu; Kathleen J. Berger; Osvaldo Gutierrez; Mark D. Levin. Journal of the American Chemical Society (2023), 145(a), 17-24.
Non Patent Document 6: C. J. Cavender; V. J. Shiner Jr. The Journal of Organic Chemistry (1972), 37(22), 3567-3569.
Non Patent Document 7: Genyi Meng; Taijie Guo; Tiancheng Ma; Jiong Zhang; Yucheng Shen; Karl Barry Sharpless; Jiajia Dong. Nature (2019), 574, 86-89.

### SUMMARY OF INVENTION

### Technical Problem

Functionalization reactions of primary alkylamines are useful approaches for converting an amino group, but, for example, the reactions via pyridinium salts in Non Patent Documents 2 and 3 may require a high temperature of 200 to 300°C and an expensive transition metal catalyst. In the methods using anomeric amides in Non Patent Documents 4 and 5, since some anomeric amides are prone to decomposition, there is a safety concern during scale-up. In the reactions that convert an amino group into an azide group in Non Patent Documents 6 and 7, it is necessary to synthesize trifluoromethanesulfonyl azide or fluorosulfuryl azide in advance.

The present invention has been made in view of the above circumstances, and an object of the present invention is to provide an amino group eliminating agent, a reaction product production method, a reaction precursor, a reaction precursor production method, and an optically active reaction product production method, which are used to efficiently produce a reaction product by reacting an organic compound having an amino group with an amino group eliminating agent, and additionally reacting the organic compound with a nucleophile.

### Solution to Problem

The inventors of the present invention conducted extensive studies regarding the above problems, and as a result, found that a reaction product can be efficiently produced by reacting an organic compound having an amino group with a specific amino group eliminating agent, and additionally reacting the organic compound with a nucleophile, and completed the present invention.

The present invention includes the following aspects.
[1] An amino group eliminating agent containing a compound (a) represented by the following Formula (a), which is used to produce a reaction product obtained by reacting an organic compound having an amino group with the amino group eliminating agent and additionally reacting the organic compound with a nucleophile:

   Y¹-X¹-Rf-X²-Y² ··· (a)

   in Formula (a), Rf is a substituted or unsubstituted divalent hydrocarbon group containing at least one fluorine atom; X¹ and X² are each independently a carbonyl group or a sulfonyl group; and Y¹ and Y² are each independently a halogen atom, a substituted or unsubstituted alkylcarbonyloxy group, a substituted or unsubstituted arylcarbonyloxy group, a substituted or unsubstituted alkylsulfonyloxy group, or a substituted or unsubstituted arylsulfonyloxy group.
[2] The amino group eliminating agent according to [1], wherein the organic compound is represented by the following Formula (b): 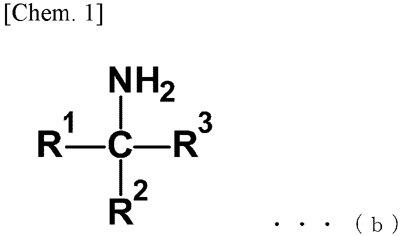 in Formula (b), R¹, R² and R³ are each independently a hydrogen atom, a polar group, a substituted or unsubstituted saturated hydrocarbon group, a substituted or unsubstituted unsaturated hydrocarbon group, or a substituted or unsubstituted aromatic hydrocarbon group, and at least two of R¹, R² and R³ may be bonded to each other to form a ring.
[3] The amino group eliminating agent according to [1] or [2],
   wherein the nucleophile is used to substitute the amino group, which has reacted with the amino group eliminating agent, with a chemical species, and
   the chemical species includes at least one selected from the group consisting of a hydrogen atom, a hydroxy group, a halogen atom, a substituted or unsubstituted alkoxy group, a substituted or unsubstituted aryloxy group, an azide group, a substituted or unsubstituted amino group, a substituted or unsubstituted aromatic heterocyclic group, a substituted or unsubstituted alkylthio group, a substituted or unsubstituted arylthio group, a cyano group, a substituted or unsubstituted phosphino group, a substituted or unsubstituted amide group, a substituted or unsubstituted sulfonamide group, and a substituted or unsubstituted hydrocarbon group.
[4] The amino group eliminating agent according to any one of [1] to [3], wherein the reaction product is represented by the following Formula (d): 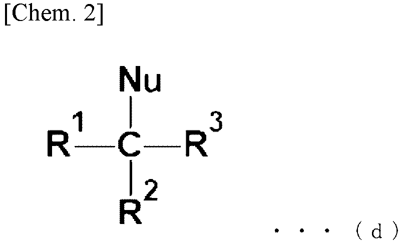 in Formula (d), R¹, R² and R³ are each independently a hydrogen atom, a polar group, a substituted or unsubstituted saturated hydrocarbon group, a substituted or unsubstituted unsaturated hydrocarbon group, or a substituted or unsubstituted aromatic hydrocarbon group, and at least two of R¹, R² and R³ may be bonded to each other to form a ring; and Nu is a hydrogen atom, a hydroxy group, a halogen atom, a substituted or unsubstituted alkoxy group, a substituted or unsubstituted aryloxy group, an azide group, a substituted or unsubstituted amino group, a substituted or unsubstituted aromatic heterocyclic group, a substituted or unsubstituted alkylthio group, a substituted or unsubstituted arylthio group, a cyano group, a substituted or unsubstituted phosphino group, a substituted or unsubstituted amide group, a substituted or unsubstituted sulfonamide group, or a substituted or unsubstituted hydrocarbon group.
[5] A reaction product production method, including
   reacting an organic compound having an amino group with an amino group eliminating agent containing a compound (a) represented by the following Formula (a) and additionally reacting the organic compound with a nucleophile:

   Y¹-X¹-Rf-X²-Y² ··· (a)

   in Formula (a), Rf is a substituted or unsubstituted divalent hydrocarbon group containing at least one fluorine atom; X¹ and X² are each independently a carbonyl group or a sulfonyl group; and Y¹ and Y² are each independently a halogen atom, a substituted or unsubstituted alkylcarbonyloxy group, a substituted or unsubstituted arylcarbonyloxy group, a substituted or unsubstituted alkylsulfonyloxy group, or a substituted or unsubstituted arylsulfonyloxy group.
[6] The reaction product production method according to [5], wherein the organic compound is represented by the following Formula (b): 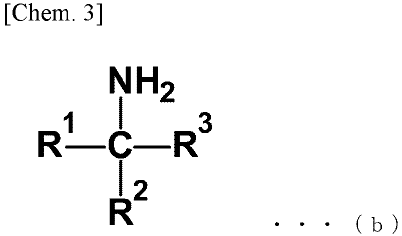 in Formula (b), R¹, R² and R³ are each independently a hydrogen atom, a polar group, a substituted or unsubstituted saturated hydrocarbon group, a substituted or unsubstituted unsaturated hydrocarbon group, or a substituted or unsubstituted aromatic hydrocarbon group, and at least two of R¹, R² and R³ may be bonded to each other to form a ring.
[7] The reaction product production method according to [5] or [6],
   wherein the nucleophile is used to substitute the amino group, which has reacted with the amino group eliminating agent, with a chemical species, and
   the chemical species includes at least one selected from the group consisting of a hydrogen atom, a hydroxy group, a halogen atom, a substituted or unsubstituted alkoxy group, a substituted or unsubstituted aryloxy group, an azide group, a substituted or unsubstituted amino group, a substituted or unsubstituted aromatic heterocyclic group, a substituted or unsubstituted alkylthio group, a substituted or unsubstituted arylthio group, a cyano group, a substituted or unsubstituted phosphino group, a substituted or unsubstituted amide group, a substituted or unsubstituted sulfonamide group, and a substituted or unsubstituted hydrocarbon group.
[8] The reaction product production method according to any one of [5] to [7], wherein the reaction product is represented by the following Formula (d): 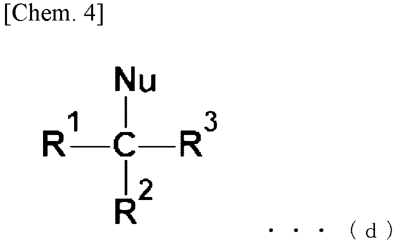 in Formula (d), R¹, R² and R³ are each independently a hydrogen atom, a polar group, a substituted or unsubstituted saturated hydrocarbon group, a substituted or unsubstituted unsaturated hydrocarbon group, or a substituted or unsubstituted aromatic hydrocarbon group, and at least two of R¹, R² and R³ may be bonded to each other to form a ring; and Nu is a hydrogen atom, a hydroxy group, a halogen atom, a substituted or unsubstituted alkoxy group, a substituted or unsubstituted aryloxy group, an azide group, a substituted or unsubstituted amino group, a substituted or unsubstituted aromatic heterocyclic group, a substituted or unsubstituted alkylthio group, a substituted or unsubstituted arylthio group, a cyano group, a substituted or unsubstituted phosphino group, a substituted or unsubstituted amide group, a substituted or unsubstituted sulfonamide group, or a substituted or unsubstituted hydrocarbon group.
[9] A reaction precursor containing a compound (e) represented by the following Formula (e) or a compound (f) represented by the following Formula (f), which is used to obtain a reaction product by reacting with a nucleophile: 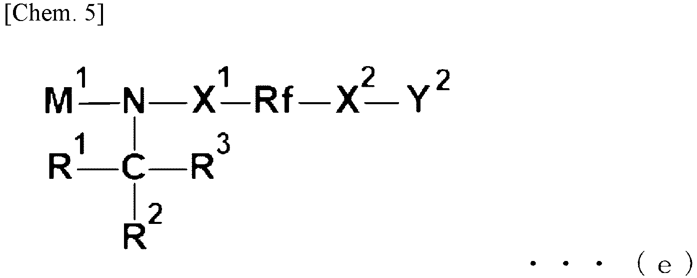 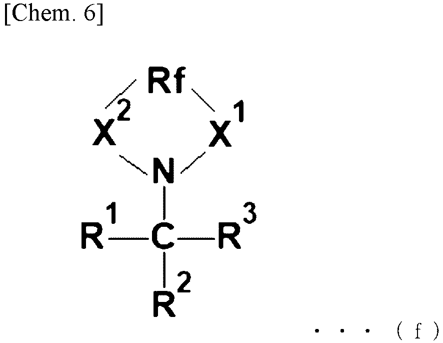 in Formulae (e) and (f), Rf is a substituted or unsubstituted divalent hydrocarbon group containing at least one fluorine atom; X¹ and X² are each independently a carbonyl group or a sulfonyl group; Y² is a halogen atom, a substituted or unsubstituted alkylcarbonyloxy group, a substituted or unsubstituted arylcarbonyloxy group, a substituted or unsubstituted alkylsulfonyloxy group, or a substituted or unsubstituted arylsulfonyloxy group; M¹ is a hydrogen atom or a counter ion; and R¹, R² and R³ are each independently a hydrogen atom, a polar group, a substituted or unsubstituted saturated hydrocarbon group, a substituted or unsubstituted unsaturated hydrocarbon group, or a substituted or unsubstituted aromatic hydrocarbon group, and at least two of R¹, R² and R³ may be bonded to each other to form a ring.
[10] The reaction precursor according to [9],
   wherein the nucleophile is used to substitute a tertiary amino group in Formulae (e) and (f) with a chemical species, and
   the chemical species includes at least one chemical species selected from the group consisting of a hydrogen atom, a hydroxy group, a halogen atom, a substituted or unsubstituted alkoxy group, a substituted or unsubstituted aryloxy group, an azide group, a substituted or unsubstituted amino group, a substituted or unsubstituted aromatic heterocyclic group, a substituted or unsubstituted alkylthio group, a substituted or unsubstituted arylthio group, a cyano group, a substituted or unsubstituted phosphino group, a substituted or unsubstituted amide group, a substituted or unsubstituted sulfonamide group, and a substituted or unsubstituted hydrocarbon group.
[11] The reaction precursor according to [9] or [10], wherein the reaction product is represented by the following Formula (d): 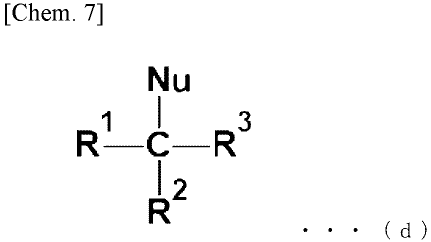 in Formula (d), R¹, R² and R³ are each independently a hydrogen atom, a polar group, a substituted or unsubstituted saturated hydrocarbon group, a substituted or unsubstituted unsaturated hydrocarbon group, or a substituted or unsubstituted aromatic hydrocarbon group, and at least two of R¹, R² and R³ may be bonded to each other to form a ring; and Nu is a hydrogen atom, a hydroxy group, a halogen atom, a substituted or unsubstituted alkoxy group, a substituted or unsubstituted aryloxy group, an azide group, a substituted or unsubstituted amino group, a substituted or unsubstituted aromatic heterocyclic group, a substituted or unsubstituted alkylthio group, a substituted or unsubstituted arylthio group, a cyano group, a substituted or unsubstituted phosphino group, a substituted or unsubstituted amide group, a substituted or unsubstituted sulfonamide group, or a substituted or unsubstituted hydrocarbon group.
[12] A method of producing a reaction precursor containing a compound (e) represented by the following Formula (e) or a compound (f) represented by the following Formula (f), which is used to obtain a reaction product by reacting with a nucleophile, including
   reacting an organic compound having an amino group with an amino group eliminating agent containing a compound (a) represented by the following Formula (a):

   Y¹-X¹-Rf-X²-Y² ··· (a)

   in Formula (a), Rf is a substituted or unsubstituted divalent hydrocarbon group containing at least one fluorine atom; X¹ and X² are each independently a carbonyl group or a sulfonyl group; and Y¹ and Y² are each independently a halogen atom, a substituted or unsubstituted alkylcarbonyloxy group, a substituted or unsubstituted arylcarbonyloxy group, a substituted or unsubstituted alkylsulfonyloxy group, or a substituted or unsubstituted arylsulfonyloxy group. 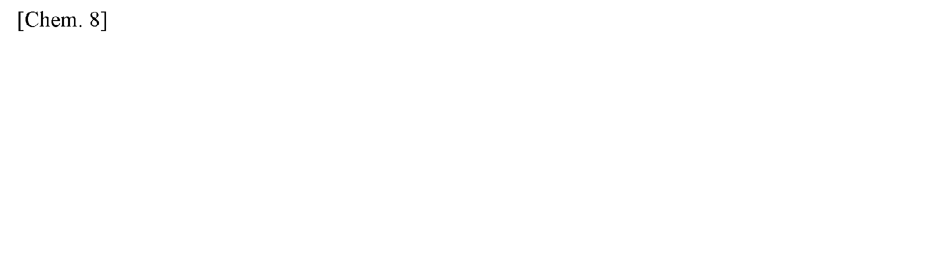 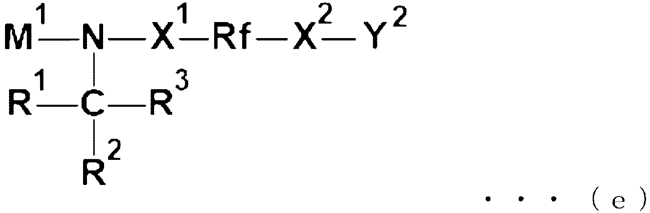 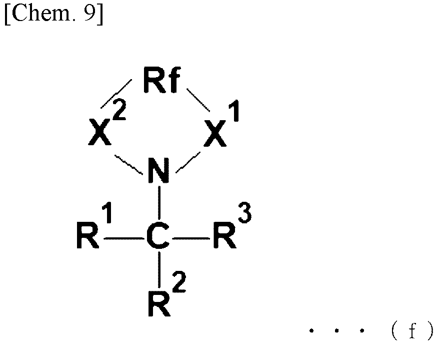 in Formulae (e) and (f), Rf is a substituted or unsubstituted divalent hydrocarbon group containing at least one fluorine atom; X¹ and X² are each independently a carbonyl group or a sulfonyl group; Y² is a halogen atom, a substituted or unsubstituted alkylcarbonyloxy group, a substituted or unsubstituted arylcarbonyloxy group, a substituted or unsubstituted alkylsulfonyloxy group, or a substituted or unsubstituted arylsulfonyloxy group; M¹ is a hydrogen atom or a counter ion; and R¹, R² and R³ are each independently a hydrogen atom, a polar group, a substituted or unsubstituted saturated hydrocarbon group, a substituted or unsubstituted unsaturated hydrocarbon group, or a substituted or unsubstituted aromatic hydrocarbon group, and at least two of R¹, R² and R³ may be bonded to each other to form a ring.
[13] The reaction precursor production method according to [12], wherein the organic compound is represented by the following Formula (b): 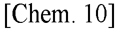 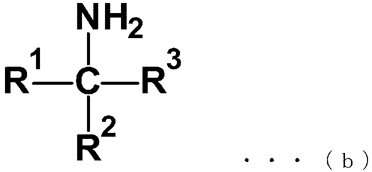 in Formula (b), R¹, R² and R³ are each independently a hydrogen atom, a polar group, a substituted or unsubstituted saturated hydrocarbon group, a substituted or unsubstituted unsaturated hydrocarbon group, or a substituted or unsubstituted aromatic hydrocarbon group, and at least two of R¹, R² and R³ may be bonded to each other to form a ring.
[14] The reaction precursor production method according to [12] or [13],
   wherein the nucleophile is used to substitute the amino group, which has reacted with the amino group eliminating agent, with a chemical species, and
   the chemical species includes at least one chemical species selected from the group consisting of a hydrogen atom, a hydroxy group, a halogen atom, a substituted or unsubstituted alkoxy group, a substituted or unsubstituted aryloxy group, an azide group, a substituted or unsubstituted amino group, a substituted or unsubstituted aromatic heterocyclic group, a substituted or unsubstituted alkylthio group, a substituted or unsubstituted arylthio group, a cyano group, a substituted or unsubstituted phosphino group, a substituted or unsubstituted amide group, a substituted or unsubstituted sulfonamide group, and a substituted or unsubstituted hydrocarbon group.
[15] The reaction precursor production method according to any one of [12] to [14], wherein the reaction product is represented by the following Formula (d): 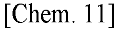 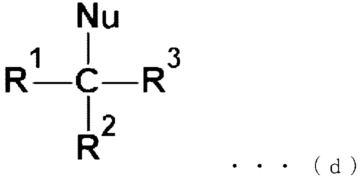 in Formula (d), R¹, R² and R³ are each independently a hydrogen atom, a polar group, a substituted or unsubstituted saturated hydrocarbon group, a substituted or unsubstituted unsaturated hydrocarbon group, or a substituted or unsubstituted aromatic hydrocarbon group, and at least two of R¹, R² and R³ may be bonded to each other to form a ring; and Nu is a hydrogen atom, a hydroxy group, a halogen atom, a substituted or unsubstituted alkoxy group, a substituted or unsubstituted aryloxy group, an azide group, a substituted or unsubstituted amino group, a substituted or unsubstituted aromatic heterocyclic group, a substituted or unsubstituted alkylthio group, a substituted or unsubstituted arylthio group, a cyano group, a substituted or unsubstituted phosphino group, a substituted or unsubstituted amide group, a substituted or unsubstituted sulfonamide group, or a substituted or unsubstituted hydrocarbon group.
[16] An optically active reaction product production method, including reacting an optically active organic compound having an amino group with an amino group eliminating agent containing a compound (a) represented by the following Formula (a) and additionally reacting the optically active organic compound with a nucleophile:

   Y¹-X¹-Rf-X²-Y² ··· (a)

   in Formula (a), Rf is a substituted or unsubstituted divalent hydrocarbon group containing at least one fluorine atom; X¹ and X² are each independently a carbonyl group or a sulfonyl group; and Y¹ and Y² are each independently a halogen atom, a substituted or unsubstituted alkylcarbonyloxy group, a substituted or unsubstituted arylcarbonyloxy group, a substituted or unsubstituted alkylsulfonyloxy group, or a substituted or unsubstituted arylsulfonyloxy group.
[17] The optically active reaction product production method according to [16], wherein the optically active organic compound is represented by the following Formula (b*): 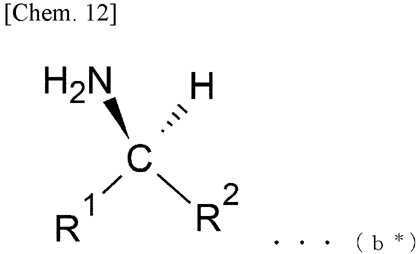 in Formula (b*), R¹ and R² are not the same and each independently a polar group, a substituted or unsubstituted saturated hydrocarbon group, a substituted or unsubstituted unsaturated hydrocarbon group, or a substituted or unsubstituted aromatic hydrocarbon group, and R¹ and R² may be bonded to each other to form a ring.
[18] The optically active reaction product production method according to [16] or [17],
   wherein the nucleophile is used to substitute the amino group, which has reacted with the amino group eliminating agent, with a chemical species, and
   the chemical species includes at least one chemical species selected from the group consisting of a hydrogen atom, a hydroxy group, a halogen atom, a substituted or unsubstituted alkoxy group, a substituted or unsubstituted aryloxy group, an azide group, a substituted or unsubstituted amino group, a substituted or unsubstituted aromatic heterocyclic group, a substituted or unsubstituted alkylthio group, a substituted or unsubstituted arylthio group, a cyano group, a substituted or unsubstituted phosphino group, a substituted or unsubstituted amide group, a substituted or unsubstituted sulfonamide group, and a substituted or unsubstituted hydrocarbon group.
[19] The optically active reaction product production method according to any one of [16] to [18], wherein the optically active reaction product is represented by the following Formula (d*): 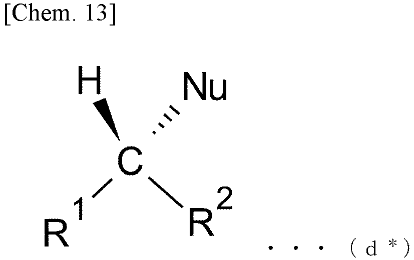 in Formula (d*), R¹ and R² are not the same and each independently a polar group, a substituted or unsubstituted saturated hydrocarbon group, a substituted or unsubstituted unsaturated hydrocarbon group, or a substituted or unsubstituted aromatic hydrocarbon group, and R¹ and R² may be bonded to each other to form a ring; and Nu is a hydrogen atom, a hydroxy group, a halogen atom, a substituted or unsubstituted alkoxy group, a substituted or unsubstituted aryloxy group, an azide group, a substituted or unsubstituted amino group, a substituted or unsubstituted aromatic heterocyclic group, a substituted or unsubstituted alkylthio group, a substituted or unsubstituted arylthio group, a cyano group, a substituted or unsubstituted phosphino group, a substituted or unsubstituted amide group, a substituted or unsubstituted sulfonamide group, or a substituted or unsubstituted hydrocarbon group.

### ADVANTAGEOUS EFFECTS OF INVENTION

According to the present invention, it is possible to provide an amino group eliminating agent, a reaction product production method, a reaction precursor, a reaction precursor production method, and an optically active reaction product production method, which are used to efficiently produce a reaction product by reacting an organic compound having an amino group with an amino group eliminating agent and additionally reacting the organic compound with a nucleophile.

### DESCRIPTION OF EMBODIMENTS

In the scope of this specification and claims, the term "aliphatic" is a relative concept with respect to the term "aromatic," and defines a group, compound or the like which has no aromaticity.

Unless otherwise specified, the term "alkyl group" includes linear, branched and cyclic monovalent saturated hydrocarbon groups. The same applies to alkyl groups in alkoxy groups.

Unless otherwise specified, the term "alkylene group" includes linear, branched and cyclic divalent saturated hydrocarbon groups.

Unless otherwise specified, the term "alkenylene group" includes linear, branched and cyclic divalent unsaturated hydrocarbon groups.

Unless otherwise specified, the term "aryl group" is defined as a monovalent group in which one hydrogen atom has been removed from an aromatic hydrocarbon ring. The same applies to an aryl group in a phenoxy group.

Unless otherwise specified, the term "heteroaryl group" is defined as a monovalent group in which one hydrogen atom has been removed from an aromatic heterocycle. The same applies to a heteroaryl group in a heteroaryloxy group.

The term "halogen atom" includes a fluorine atom, a chlorine atom, a bromine atom, an iodine atom, and the like.

The term "polar group" includes a carboxy group, a hydroxy group, an amino group, a sulfo group (-SO₃H) and the like.

The description "substituted or unsubstituted" includes both a case in which a hydrogen atom (-H) is substituted with a monovalent substituent and a case in which a methylene group (-CH₂-) or a methine group (>CH- or =CH-) is replaced with a divalent substituent.

The term "monovalent substituent" includes an alkyl group, an alkoxy group, a halogen atom, a halogenated alkyl group, a hydroxy group, a carboxy group, an amino group, a nitro group and the like.

The alkyl group as a substituent is preferably an alkyl group having 1 to 5 carbon atoms, and most preferably a methyl group, an ethyl group, a propyl group, an n-butyl group, or a tert-butyl group.

The alkoxy group as a substituent is preferably an alkoxy group having 1 to 5 carbon atoms, more preferably a methoxy group, an ethoxy group, an n-propoxy group, an iso-propoxy group, an n-butoxy group, or a tert-butoxy group, and most preferably a methoxy group or an ethoxy group.

The halogen atom as a substituent is preferably a fluorine atom or an iodine atom.

Examples of halogenated alkyl groups as a substituent include groups in which some or all of the hydrogen atoms in an alkyl group having 1 to 5 carbon atoms, for example, a methyl group, an ethyl group, a propyl group, an n-butyl group, or a tert-butyl group, are substituted with the halogen atom.

The term "divalent substituent" includes -O-, -C(=O)-O-, -O-C(=O)-, -C(=O)-, -O-C(=O)-O-, -C(=O)-NH-, -C(=O)-NH-C(=O)-, -N=, -NH-, -NH-C(=NH)- (H may be substituted with a substituent such as a hydrocarbon group, an acyl group, or an alkoxyalkyl group), -S-, -S(=O)₂-, -S(=O)₂-O-, a group represented by General Formula -Y²¹-O-Y²²-, - Y²¹-O-, -Y²¹-C(=O)-O-, -C(=O)-O-Y²¹-, -[Y²¹-C(=O)-O]_{m"}-Y²²-, -Y²¹-O-C(=O)-Y²²- or - Y²¹-S(=O)₂-O-Y²²- [in the formula, Y²¹ and Y²² are each independently a substituted or unsubstituted divalent hydrocarbon group, O is an oxygen atom, and m" is an integer of 0 to 3], and the like.

### <Amino group eliminating agent>

A first aspect of the present invention is an amino group eliminating agent containing a compound (a) represented by the following Formula (a), wherein the amino group eliminating agent is used to produce a reaction product obtained by reacting an organic compound having an amino group with the amino group eliminating agent and additionally reacting the organic compound with a nucleophile:

Y¹-X¹-Rf-X²-Y² ··· (a)

in Formula (a), Rf is a substituted or unsubstituted divalent hydrocarbon group containing at least one fluorine atom; X¹ and X² are each independently a carbonyl group or a sulfonyl group; and Y¹ and Y² are each independently a halogen atom, a substituted or unsubstituted alkylcarbonyloxy group, a substituted or unsubstituted arylcarbonyloxy group, a substituted or unsubstituted alkylsulfonyloxy group, or a substituted or unsubstituted arylsulfonyloxy group.

Rf is a substituted or unsubstituted divalent hydrocarbon group containing at least one fluorine atom.

### ·Substituted or unsubstituted divalent hydrocarbon group:

The substituted or unsubstituted divalent hydrocarbon group for Rf may be an aliphatic hydrocarbon group or an aromatic hydrocarbon group. In the substituted or unsubstituted divalent hydrocarbon group for Rf, at least one hydrogen atom is substituted with a fluorine atom.

### ··Aliphatic hydrocarbon group

The aliphatic hydrocarbon group is a hydrocarbon group having no aromaticity. The aliphatic hydrocarbon group may be saturated or unsaturated (for example, an alkenylene group and an alkynylene group).

Examples of aliphatic hydrocarbon groups include a chain aliphatic hydrocarbon group and an aliphatic hydrocarbon group containing a ring in the structure. The chain aliphatic hydrocarbon group may be a linear aliphatic hydrocarbon group or a branched aliphatic hydrocarbon group.

### ···Linear or branched aliphatic hydrocarbon group

The linear aliphatic hydrocarbon group preferably has 1 to 10 carbon atoms, more preferably has 1 to 6 carbon atoms, still more preferably has 1 to 4 carbon atoms, and most preferably has 1 to 3 carbon atoms.

The linear aliphatic hydrocarbon group is preferably a linear alkylene group, and specific examples thereof include a methylene group [-CH₂-], an ethylene group [-(CH₂)₂-], a trimethylene group [-(CH₂)₃-], a tetramethylene group [-(CH₂)₄-], and a pentamethylene group [-(CH₂)₅-].

The branched aliphatic hydrocarbon group preferably has 2 to 10 carbon atoms, more preferably has 3 to 6 carbon atoms, still more preferably has 3 or 4 carbon atoms, and most preferably has 3 carbon atoms.

The branched aliphatic hydrocarbon group is preferably a branched alkylene group, and specific examples thereof include alkylalkylene groups, for example, alkylmethylene groups such as -CH(CH₃)-, -CH(CH₂CH₃)-, -C(CH₃)₂-, -C(CH₃)(CH₂CH₃)-, - C(CH₃)(CH₂CH₂CH₃)-, and -C(CH₂CH₃)₂-; alkylethylene groups such as -CH(CH₃)CH₂-, - CH(CH₃)CH(CH₃)-, -C(CH₃)₂CH₂-, -CH(CH₂CH₃)CH₂-, and -C(CH₂CH₃)₂-CH₂-; alkyltrimethylene groups such as -CH(CH₃)CH₂CH₂- and -CH₂CH(CH₃)CH₂-; and alkyltetramethylene groups such as -CH(CH₃)CH₂CH₂CH₂- and -CH₂CH(CH₃)CH₂CH₂-. The alkyl group in the alkylalkylene group is preferably a linear alkyl group having 1 to 5 carbon atoms.

The linear or branched aliphatic hydrocarbon group may or may not have a substituent.

### ···Aliphatic hydrocarbon group containing ring in structure

Examples of aliphatic hydrocarbon groups containing a ring in the structure include a cyclic aliphatic hydrocarbon group (a group in which two hydrogen atoms have been removed from an aliphatic hydrocarbon ring) which may contain a substituent containing a heteroatom in the ring structure, a group in which the cyclic aliphatic hydrocarbon group is bonded to the end of a linear or branched aliphatic hydrocarbon group, and a group in which the cyclic aliphatic hydrocarbon group is inserted into a linear or branched aliphatic hydrocarbon group. Linear or branched aliphatic hydrocarbon groups are the same as those described above.

The cyclic aliphatic hydrocarbon group preferably has 3 to 20 carbon atoms and more preferably has 3 to 12 carbon atoms.

The cyclic aliphatic hydrocarbon group may be a polycyclic group or a monocyclic group. The monocyclic alicyclic hydrocarbon group is preferably a group in which two hydrogen atoms have been removed from a monocycloalkane. The monocycloalkane preferably has 3 to 6 carbon atoms, and specific examples thereof include cyclopropane, cyclopentane, and cyclohexane. The polycyclic alicyclic hydrocarbon group is preferably a group in which two hydrogen atoms have been removed from a polycycloalkane, the polycycloalkane preferably has 7 to 12 carbon atoms, and specific examples thereof include adamantane, norbornane, isobornane, tricyclodecane, and tetracyclododecane.

The cyclic aliphatic hydrocarbon group may or may not have a substituent. Examples of substituents include an alkyl group, an alkoxy group, a halogen atom, a halogenated alkyl group, a hydroxy group, and a carbonyl group.

In the cyclic aliphatic hydrocarbon group, some of the carbon atoms constituting the ring structure may be substituted with a heteroatom-containing substituent. The heteroatom-containing substituent is preferably -O-, -C(=O)-O-, -S-, -S(=O)₂-, or -S(=O)₂-O-

### ··Aromatic hydrocarbon group

The aromatic hydrocarbon group is a hydrocarbon group having at least one aromatic ring.

The aromatic ring is not particularly limited as long as it is a cyclic conjugated system having 4n+2 π electrons, and may be monocyclic or polycyclic. The aromatic ring preferably has 5 to 30 carbon atoms, more preferably has 5 to 20 carbon atoms, still more preferably has 6 to 15 carbon atoms, and particularly preferably has 6 to 12 carbon atoms. Here, the number of carbon atoms does not include the number of carbon atoms in the substituent.

Specific examples of aromatic rings include aromatic hydrocarbon rings such as benzene, naphthalene, anthracene, and phenanthrene; and aromatic heterocycles in which some carbon atoms constituting the aromatic hydrocarbon ring are substituted with heteroatoms. Examples of heteroatoms in aromatic heterocycles include an oxygen atom, a sulfur atom, and a nitrogen atom. Specific examples of aromatic heterocycles include a pyridine ring and a thiophene ring.

Specific examples of aromatic hydrocarbon groups include a group in which two hydrogen atoms have been removed from the aromatic hydrocarbon ring or aromatic heterocycle (an arylene group or a heteroarylene group); a group in which two hydrogen atoms have been removed from an aromatic compound containing two or more aromatic rings (for example biphenyl, fluorene, etc.); and a group in which one hydrogen atom of a group (an aryl group or a heteroaryl group) in which one hydrogen atom has been removed from the aromatic hydrocarbon ring or aromatic heterocycle is substituted with an alkylene group (for example, a group in which one hydrogen atom has been additionally removed from the aryl group in the arylalkyl group such as a benzyl group, a phenethyl group, a 1-naphthyl methyl group, a 2-naphthyl methyl group, a 1-naphthyl ethyl group, and a 2-naphthyl ethyl group). The alkylene group bonded to the aryl group or heteroaryl group preferably has 1 to 4 carbon atoms, more preferably has 1 or 2 carbon atoms, and particularly preferably has 1 carbon atom.

In the aromatic hydrocarbon group, the hydrogen atom in the aromatic hydrocarbon group may be substituted with a substituent. For example, the hydrogen atom in the aromatic hydrocarbon group, which is bonded to an aromatic ring, may be substituted with a substituent. Examples of substituents include an alkyl group, an alkoxy group, a halogen atom, a halogenated alkyl group, and a hydroxy group.

The divalent hydrocarbon group for Rf is preferably a substituted or unsubstituted linear, branched or cyclic aliphatic hydrocarbon group; a substituted or unsubstituted aromatic hydrocarbon group; or a combination of two or more thereof; and is more preferably a substituted or unsubstituted linear, branched or cyclic aliphatic hydrocarbon group; a substituted or unsubstituted aromatic hydrocarbon group; or a combination of two or more thereof.

The divalent hydrocarbon group for Rf preferably has 2 to 10 carbon atoms, more preferably has 2 to 8 carbon atoms, still more preferably has 2 to 6 carbon atoms, and particularly preferably has 2 to 4 carbon atoms.

Rf preferably has 1 to 10 fluorine atoms, more preferably has 2 to 8 fluorine atoms, still more preferably has 3 to 10 fluorine atoms, and particularly preferably has 3 to 8 fluorine atoms.

Specifically, a difluoromethylene group [-CF₂-], a perfluoroethylene group [-(CF₂)₂-], a perfluorotrimethylene group [-(CF₂)₃-], a perfluorotetramethylene group [-(CF₂)₄-], a perfluoropentamethylene group [-(CF₂)₅-], a perfluorophenyl group and the like are preferable.

X¹ and X² are each independently a carbonyl group or a sulfonyl group. In particular, it is preferable that at least one of X¹ and X² be a sulfonyl group, and it is more preferable that both of X¹ and X² be a sulfonyl group.

Y¹ and Y² are each independently a halogen atom, a substituted or unsubstituted alkylcarbonyloxy group, a substituted or unsubstituted arylcarbonyloxy group, a substituted or unsubstituted alkylsulfonyloxy group, or a substituted or unsubstituted arylsulfonyloxy group.

### ·Halogen atom

Examples of halogen atoms for Y¹ and Y² include a fluorine atom, a chlorine atom, a bromine atom, and an iodine atom. In particular, a fluorine atom is preferable.

### · Substituted or unsubstituted alkylcarbonyloxy group, substituted or unsubstituted arylcarbonyloxy group, substituted or unsubstituted alkylsulfonyloxy group, and substituted or unsubstituted arylsulfonyloxy group

In the substituted or unsubstituted alkylcarbonyloxy group, substituted or unsubstituted arylcarbonyloxy group, substituted or unsubstituted alkylsulfonyloxy group, and substituted or unsubstituted arylsulfonyloxy group for Y¹ and Y², alkyl groups and aryl groups are the same as those described above for the alkyl group and aryl group.

The amino group eliminating agent may contain, in addition to the compound (a), optional components such as a solvent, a compound containing a transition metal, a compound containing a ligand, and an organic catalyst.

The organic compound is an organic compound having at least one amino group. The number of amino groups in the organic compound may be one or more, is preferably 1 to 5, and more preferably 1 to 3.

The organic compound is preferably represented by the following Formula (b).

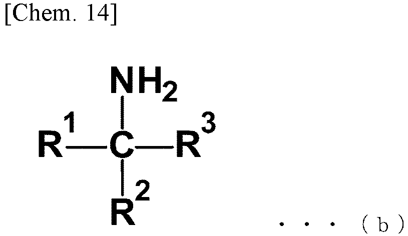

In Formula (b), R¹, R² and R³ are each independently a hydrogen atom, a polar group, a substituted or unsubstituted saturated hydrocarbon group, a substituted or unsubstituted unsaturated hydrocarbon group, or a substituted or unsubstituted aromatic hydrocarbon group, and at least two of R¹, R² and R³ may be bonded to each other to form a ring.

### ·Polar group

In Formula (b), examples of polar groups include a carboxy group, a hydroxy group, an amino group, and a sulfo group (-SO₃H).

### ·Saturated hydrocarbon group

In Formula (b), examples of saturated hydrocarbon groups include a linear or branched saturated hydrocarbon group having 1 to 10 carbon atoms and a cyclic saturated hydrocarbon group having 3 to 10 carbon atoms. Specific examples of linear or branched saturated hydrocarbon groups having 1 to 10 carbon atoms include a methyl group, an ethyl group, an n-propyl group, an isopropyl group, an n-butyl group, an isobutyl group, a sec-butyl group, a tert-butyl group, a pentyl group, a hexyl group, a heptyl group, an octyl group, a 2-ethylhexyl group, a nonyl group, and a decyl group. Specific examples of cyclic saturated hydrocarbon groups having 3 to 10 carbon atoms include a cyclopropyl group, a cyclobutyl group, a cyclopentyl group, a cyclohexyl group, a cycloheptyl group, a cyclooctyl group, a cyclononyl group, and a cyclodecyl group.

Examples of rings formed by bonding at least two of R¹, R² and R³ to each other include a cyclic saturated hydrocarbon group having 3 to 10 carbon atoms and a cyclic unsaturated hydrocarbon group having 3 to 10 carbon atoms. Specific examples of cyclic saturated hydrocarbon groups having 3 to 10 carbon atoms include a cyclopropyl group, a cyclobutyl group, a cyclopentyl group, a cyclohexyl group, a cycloheptyl group, a cyclooctyl group, a cyclononyl group, and a cyclodecyl group. Specific examples of cyclic unsaturated hydrocarbon groups having 3 to 10 carbon atoms include a cyclopropenyl group, a cyclobutenyl group, a cyclopentenyl group, a cyclohexenyl group, a cycloheptenyl group, a cyclooctenyl group, a cyclononenyl group, and a cyclodecenyl group.

### ·Unsaturated hydrocarbon group

**In** Formula (b), examples of unsaturated hydrocarbon groups include a linear or branched unsaturated hydrocarbon group having 2 to 10 carbon atoms and a cyclic unsaturated hydrocarbon group having 3 to 10 carbon atoms. Specific examples of linear or branched unsaturated hydrocarbon groups having 2 to 10 carbon atoms include alkenyl groups such as a vinyl group, a propenyl group, a butenyl group, a pentenyl group, and a hexenyl group. Specific examples of cyclic unsaturated hydrocarbon groups having 3 to 10 carbon atoms include cycloalkenyl groups such as a cyclopropenyl group, a cyclobutenyl group, a cyclopentenyl group, and a cyclohexenyl group.

### ·Aromatic hydrocarbon group

In Formula (b), examples of aromatic hydrocarbon groups include an aromatic hydrocarbon group having 6 to 10 carbon atoms, and specific examples thereof include a phenyl group, a methylphenyl group, an ethylphenyl group, a hydroxyphenyl group, a chlorophenyl group, a nitrophenyl group, and a naphthyl group.

R¹, R² and R³ are each independently preferably a hydrogen atom, a carboxy group, a substituted or unsubstituted linear or branched saturated hydrocarbon group having 1 to 10 carbon atoms, a substituted or unsubstituted cyclic saturated hydrocarbon group having 3 to 10 carbon atoms, a substituted or unsubstituted linear or branched unsaturated hydrocarbon group having 2 to 10 carbon atoms, a substituted or unsubstituted cyclic unsaturated hydrocarbon group having 3 to 10 carbon atoms or a substituted or unsubstituted aromatic hydrocarbon group having 6 to 10 carbon atoms ; and more preferably a hydrogen atom, a carboxy group, a substituted or unsubstituted linear or branched saturated hydrocarbon group having 1 to 10 carbon atoms, a substituted or unsubstituted cyclic saturated hydrocarbon group having 3 to 10 carbon atoms or a substituted or unsubstituted aromatic hydrocarbon group having 6 to 10 carbon atoms.

The ring formed by bonding at least two of R¹, R² and R³ to each other is preferably a substituted or unsubstituted cyclic saturated hydrocarbon group having 3 to 10 carbon atoms, a substituted or unsubstituted cyclic unsaturated hydrocarbon group having 3 to 10 carbon atoms, or a substituted or unsubstituted aromatic hydrocarbon group having 6 to 10 carbon atoms, and more preferably a substituted or unsubstituted cyclic saturated hydrocarbon group having 3 to 10 carbon atoms or a substituted or unsubstituted aromatic hydrocarbon group having 6 to 10 carbon atoms.

In R¹, R² and R³, the monovalent substituent that substitutes a hydrogen atom (-H) is preferably an alkyl group, an alkoxy group, a halogen atom, a hydroxy group, a carboxy group, or a nitro group, and more preferably an alkyl group having 1 to 5 carbon atoms, an alkoxy group having 1 to 5 carbon atoms, a halogen atom, or a nitro group.

In R¹, R² and R³, the divalent substituent that replaces a methylene group (-CH₂-) or a methine group (>CH-, or =CH-) is preferably -O-, -C(=O)-O-, -O-C(=O)-, -C(=O)-, -O-C(=O)-O-, -C(=O)-NH-, -C(=O)-NH-C(=O)-, -N=, -NH-, -NH-C(=NH)-(H may be substituted with a substituent such as a hydrocarbon group, an acyl group, or an alkoxyalkyl group), -S-, -S(=O)₂-, -S(=O)₂-O-, a group represented by General Formula -Y²¹-O-Y²²-, - Y²¹-O-, -Y²¹-C(=O)-O-, -C(=O)-O-Y²¹-, -[Y²¹-C(=O)-O]_{m"}-Y²²-, or -Y²¹-O-C(=O)-Y²²- [in the formula, Y²¹ and Y²² are each independently a substituted or unsubstituted divalent hydrocarbon group having 2 to 10 carbon atoms, O is an oxygen atom, and m" is an integer of 0 to 3].

The nucleophile is used to substitute the amino group, which has reacted with the amino group eliminating agent, with a chemical species.

The chemical species includes at least one selected from the group consisting of a hydrogen atom, a hydroxy group, a halogen atom, a substituted or unsubstituted alkoxy group, a substituted or unsubstituted aryloxy group, an azide group, a substituted or unsubstituted amino group, a substituted or unsubstituted aromatic heterocyclic group, a substituted or unsubstituted alkylthio group, a substituted or unsubstituted arylthio group, a cyano group, a substituted or unsubstituted phosphino group, a substituted or unsubstituted amide group, a substituted or unsubstituted sulfonamide group, and a substituted or unsubstituted hydrocarbon group.

Examples of nucleophiles having a hydrogen atom include sodium hydride, sodium borohydride, and lithium aluminum hydride.

Examples of nucleophiles having a hydroxy group include water, sodium hydroxide, and potassium hydroxide.

Examples of nucleophiles having a halogen atom include fluorinating agents such as hydrogen fluoride, sodium fluoride, potassium fluoride, cesium fluoride, silver fluoride, triethylamine trihydrofluoride, hydrogen fluoridepyridine, tetramethylammonium fluoride, tetrabutylammonium fluoride (TBAF), and tetrabutylammonium difluorotriphenylsilicate (TBAT); sodium bromide; tetrabutylammonium chloride; and sodium iodide. Among these, tetrabutylammonium difluorotriphenylsilicate (TBAT) is preferable.

Examples of nucleophiles having a substituted or unsubstituted alkoxy group include a substituted or unsubstituted alcohol having 1 to 10 carbon atoms and a substituted or unsubstituted metal alkoxide having 1 to 10 carbon atoms. Examples of unsubstituted alcohols having 1 to 10 carbon atoms include methanol, ethanol, and propanol. As the substituted alcohol having 1 to 10 carbon atoms, an alcohol having 1 to 10 carbon atoms substituted with at least one fluorine atom is preferable, and an alcohol having 1 to 10 carbon atoms substituted with 1 to 11 fluorine atoms is more preferable. Specific examples of substituted alcohols having 1 to 10 carbon atoms include trifluoromethanol, trifluoroethanol, pentafluoropropanol, tetrafluoropropanol, perfluoroethanol, perfluoropropanol, and 1H,1H,2H,2H-tridecafluoro-1-n-octanol. Examples of unsubstituted metal alkoxides having 1 to 10 carbon atoms include sodium methoxide, sodium ethoxide, and sodium propoxide.

Examples of nucleophiles having a substituted or unsubstituted aryloxy group include phenol, and sodium phenoxide.

Examples of nucleophiles having an azide group include trimethylsilyl azide and sodium azide.

Examples of nucleophiles having a substituted or unsubstituted amino group include a monoalkylamine having one alkyl group having 1 to 10 carbon atoms, a dialkyl amine having two alkyl groups having 1 to 10 carbon atoms, and a trialkylamine having three alkyl groups having 1 to 10 carbon atoms. Specific examples of monoalkylamines having one alkyl group having 1 to 10 carbon atoms include methylamine, ethylamine, and propylamine. Specific examples of dialkyl amines having two alkyl groups having 1 to 10 carbon atoms include dimethylamine, methylethylamine, diethylamine, and dipropylamine. Specific examples of trialkylamines having three alkyl groups having 1 to 10 carbon atoms include trimethylamine, N,N-dimethylethylamine, N,N-diethylmethylamine, triethylamine, and tripropylamine.

Examples of nucleophiles having a substituted or unsubstituted aromatic heterocyclic group include pyrrole, imidazole, 1-methylimidazole, and pyridine.

Examples of nucleophiles having a substituted or unsubstituted alkylthio group include methanethiol, ethanethiol, and propanethiol.

Examples of nucleophiles having a substituted or unsubstituted arylthio group include thiophenol.

Examples of nucleophiles having a cyano group include trimethylsilyl cyanide.

Examples of nucleophiles having a substituted or unsubstituted phosphino group include trialkylphosphine such as trimethylphosphine; and triarylphosphine such as triphenylphosphine.

Examples of nucleophiles having a substituted or unsubstituted amide group include alkyl amides such as acetamide and trifluoroacetamide; arylamidos such as benzamide; and compounds having an imide such as phthalimide.

Examples of nucleophiles having a substituted or unsubstituted sulfonamide group include alkyl sulfonamides such as trifluoromethanesulfonamide and potassium trifluoromethanesulfonamide; aryl sulfonamides such as benzenesulfonamide; alkyl sulfonimides such as potassium bis(trifluoromethanesulfonyl)imide; and arylsulfonimides such as diphenyl sulfonamide.

Examples of nucleophiles having a substituted or unsubstituted hydrocarbon group include Grignard reagents such as an alkyl Grignard reagent and an aryl Grignard reagent; malonic esters such as dimethyl malonate and diethyl malonate; and acetoacetic esters such as methyl acetoacetate and ethyl acetoacetate.

The reaction product is preferably represented by the following Formula (d).

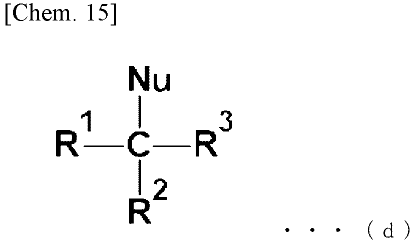

**In** Formula (d), R¹, R² and R³ are each independently a hydrogen atom, a polar group, a substituted or unsubstituted saturated hydrocarbon group, a substituted or unsubstituted unsaturated hydrocarbon group, or a substituted or unsubstituted aromatic hydrocarbon group, and at least two of R¹, R² and R³ may be bonded to each other to form a ring; and Nu is a hydrogen atom, a hydroxy group, a halogen atom, a substituted or unsubstituted alkoxy group, a substituted or unsubstituted aryloxy group, an azide group, a substituted or unsubstituted amino group, a substituted or unsubstituted aromatic heterocyclic group, a substituted or unsubstituted alkylthio group, a substituted or unsubstituted arylthio group, a cyano group, a substituted or unsubstituted phosphino group, a substituted or unsubstituted amide group, a substituted or unsubstituted sulfonamide group, or a substituted or unsubstituted hydrocarbon group.

In Formula (d), R¹, R² and R³ are the same as those described above for Formula (b).

In Formula (d), Nu is the same as that described for the chemical species in the nucleophile.

According to the amino group eliminating agent in the first aspect of the present invention, when a compound having two reactive sites in the same molecule is used as the amino group eliminating agent in order to directly substitute the amino group constituting a primary alkylamine compound with a fluorine group or the like, which could not be converted using conventional nucleophiles, fluorinated alkyl compounds and the like can be efficiently produced. This makes it possible to synthesize reaction products such as a fluorine compound, which have been difficult to synthesize in the related art, and simplify the synthesis step. In addition, when primary alkylamines, which are abundant in natural products, are used as synthetic raw materials, fluorinated alkyl compounds and the like can be efficiently produced at a low cost.

### <Reaction product production method>

A second aspect of the present invention is a reaction product production method including reacting an organic compound having an amino group with an amino group eliminating agent containing a compound (a) represented by the following Formula (a) and additionally reacting the organic compound with a nucleophile.

Y¹-X¹-Rf-X²-Y² ··· (a)

In Formula (a), Rf is a substituted or unsubstituted divalent hydrocarbon group containing at least one fluorine atom; X¹ and X² are each independently a carbonyl group or a sulfonyl group; and Y¹ and Y² are each independently a halogen atom, a substituted or unsubstituted alkylcarbonyloxy group, a substituted or unsubstituted arylcarbonyloxy group, a substituted or unsubstituted alkylsulfonyloxy group, or a substituted or unsubstituted arylsulfonyloxy group.

In Formula (d), Y¹, X¹, Rf, X², and Y² are the same as those described above for Formula (a) in the first aspect of the present invention.

Organic compounds having an amino group are the same as those described in the first aspect of the present invention.

Nucleophiles are the same as those described in the first aspect of the present invention.

The reaction product production method in the second aspect of the present invention includes reacting an organic compound having an amino group with an amino group eliminating agent containing a compound (a) represented by the following Formula (a) (hereinafter also referred to as a first reaction step), and additionally reacting the organic compound with a nucleophile (hereinafter also referred to as a second reaction step).

A multi-step reaction in which the product obtained after the first reaction step is isolated and the second reaction step is additionally performed may be used or a one-pot synthesis in which the first reaction step and the second reaction step proceed continuously may be used. The one-pot synthesis is preferable because the reaction product can be efficiently produced.

In the first reaction step and the second reaction step, a basic substance, a solvent and the like can be used.

Specific examples of basic substances include trimethylamine, triethylamine, diisopropylamine, tripropylamine, pyridine, 4-dimethylaminopyridine (DMAP), diazabicycloundecene (DBU), 1,5,7-triazabicyclo[4.4.0]dec-5-ene (TBD), diazabicyclononene (DBN), 7-methyl-1,5,7-triazabicyclo[4.4.0]dec-5-ene pyridine (MTBD), tert-butylimino-tris(dimethylamino)phosphorane, tert-butylimino-tri(pyrrolidino)phosphorene (BTPP), and 2-tert-butyl-1,1,3,3-tetramethylguanidine (BTMG). Among these, diazabicycloundecene (DBU) is particularly preferable.

The amount of the basic substance used with respect to the organic compound having an amino group is preferably in a range of 0.5 mol or more and 10 mol or less and more preferably in a range of 1.5 mol or more and 3.0 mol or less relative to 1 mol of the reactive amino group (NH₂-). When the amount of the basic substance used is 0.5 mol or more relative to 1 mol of the reactive amino group (NH₂-), the amount of the reaction product produced is less likely to be less than the reaction equivalent. In addition, when the amount of the basic substance used is 10 mol or less relative to 1 mol of the reactive amino group (NH₂-), the reaction product is less likely to be decomposed by an excessive amount of a base.

The amount of the nucleophile used with respect to the organic compound having an amino group is preferably in a range of 1 mol or more and 10 mol or less and in a range of 1.3 mol or more and 3.0 mol or less relative to 1.0 mol of the reactive amino group (NH₂-). When the amount of the nucleophile used is 1.0 mol or more relative to 1 mol of the reactive amino group (NH₂-), the amount of the nucleophile used can be minimized, and it is easier to reduce the production cost. In addition, when the amount of the nucleophile used is 10 mol or less relative to 1 mol of the reactive amino group (NH₂-), it is easier to shorten the reaction time, the amount of the nucleophile used is reduced, and it is easier to reduce the production cost.

The solvent is preferably an aprotic solvent. Specific examples of aprotic solvents include acetonitrile, propionitrile, dimethylformamide, dimethylacetamide, methylformamide, N-methylpyrrolidone, diethyl ether, diisopropyl ether, tert-butyl methyl ether, cyclopentyl methyl ether, tetrahydrofuran, 1,2-dimethoxyethane, 1,3-dioxane, 1, 4-dioxane, diglyme, triglyme, dimethyl sulfoxide, sulfolane, pentane, hexane, heptane, octane, cyclohexane, benzene, toluene, xylene, and ethylbenzene. Among these, the solvent is particularly preferably toluene. In addition, a mixture of a plurality of solvents can be used.

The reaction temperatures in the first reaction step and the second reaction step are both preferably in a range of 0°C or higher and a boiling point of a solvent or lower, more preferably in a range of 100 to 200°C, and still more preferably in a range of 130 to 150°C. The reaction proceeds easily when the reaction temperature is 0°C or higher. In addition, it is preferable to perform the first reaction step and the second reaction step under conditions in which the solvent is refluxed.

The reaction times in the first reaction step and the second reaction step are preferably in a range of 1 hour or longer and 3 hours or shorter. However, even if the reaction time is about 30 minutes, since 50% or more of the entire reaction has proceeded, the reaction temperature may be shorter than 1 hour depending on temperature conditions.

When the reaction is slow, the reaction can also be performed in an autoclave or under microwave radiation. The reaction temperature is preferably in a range of 100 to 200°C and more preferably in a range of 130 to 150°C. The reaction time is preferably in a range of 10 minutes or longer and 3 hours or shorter and more preferably in a range of 30 minutes or longer and 1 hour or shorter.

According to the reaction product production method in the second aspect of the present invention, when a compound having two reactive sites in the same molecule is used as a deaminating agent in order to directly substitute the amino group constituting a primary alkylamine compound with a fluorine group or the like, which could not be converted using conventional nucleophiles, fluorinated alkyl compounds and the like can be efficiently produced. This makes it possible to synthesize reaction products such as a fluorine compound, which have been difficult to synthesize in the related art, and simplify the synthesis step. In addition, when primary alkylamines, which are abundant in natural products, are used as synthetic raw materials, fluorinated alkyl compounds and the like can be efficiently produced at a low cost.

### <Reaction precursor>

A third aspect of the present invention is a reaction precursor containing a compound (e) represented by the following Formula (e) or a compound (f) represented by the following Formula (f), which is used to obtain a reaction product by reacting with a nucleophile.

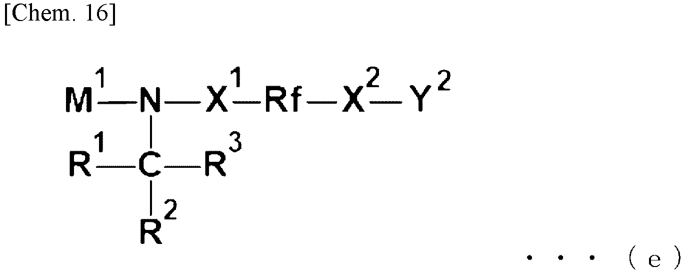

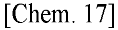

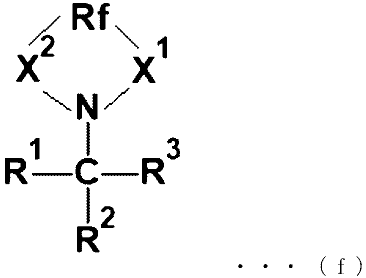

In Formulae (e) and (f), Rf is a substituted or unsubstituted divalent hydrocarbon group containing at least one fluorine atom; X¹ and X² are each independently a carbonyl group or a sulfonyl group; Y² is a halogen atom, a substituted or unsubstituted alkylcarbonyloxy group, a substituted or unsubstituted arylcarbonyloxy group, a substituted or unsubstituted alkylsulfonyloxy group, or a substituted or unsubstituted arylsulfonyloxy group; M¹ is a hydrogen atom or a counter ion; and R¹, R² and R³ are each independently a hydrogen atom, a polar group, a substituted or unsubstituted saturated hydrocarbon group, a substituted or unsubstituted unsaturated hydrocarbon group, or a substituted or unsubstituted aromatic hydrocarbon group, and at least two of R¹, R² and R³ may be bonded to each other to form a ring.

In Formulae (e) and (f), X¹, Rf, X², and Y² are the same as those described for Formula (a) in the first aspect of the present invention.

In Formulae (e) and (f), R¹, R² and R³ are the same as those described above for Formula (b) in the first aspect of the present invention.

Nucleophiles are the same as those described in the first aspect of the present invention.

Reaction products are the same as those described in the first aspect of the present invention.

In Formulae (e) and (f), the tertiary amino group bonded to a carbonyl group or a sulfonyl group is substituted with the chemical species Nu contained in a nucleophile according to the reaction with the nucleophile, and thereby a reaction product is obtained.

The reaction precursor may contain, in addition to the compound (e) or (f), optional components such as a solvent, a basic substance, a nucleophile, a compound containing a transition metal, a compound containing a ligand, and an organic catalyst.

According to the reaction precursor in the third aspect of the present invention, by reacting it with various nucleophiles, a desired reaction product can be obtained in just a single step. Therefore, it is possible to provide a synthetic intermediate that is beneficial in the fields of pharmaceuticals, agricultural chemicals, electronic materials, functional materials and the like.

### <Reaction precursor production method>

A fourth aspect of the present invention is a method of producing a reaction precursor containing a compound (e) represented by the following Formula (e) or a compound (f) represented by the following Formula (f), which is used to obtain a reaction product by reacting with a nucleophile, including reacting an organic compound having an amino group with an amino group eliminating agent containing a compound (a) represented by the following Formula (a):

Y¹-X¹-Rf-X²-Y² ··· (a)

In Formula (a), Rf is a substituted or unsubstituted divalent hydrocarbon group containing at least one fluorine atom; X¹ and X² are each independently a carbonyl group or a sulfonyl group; and Y¹ and Y² are each independently a halogen atom, a substituted or unsubstituted alkylcarbonyloxy group, a substituted or unsubstituted arylcarbonyloxy group, a substituted or unsubstituted alkylsulfonyloxy group, or a substituted or unsubstituted arylsulfonyloxy group.

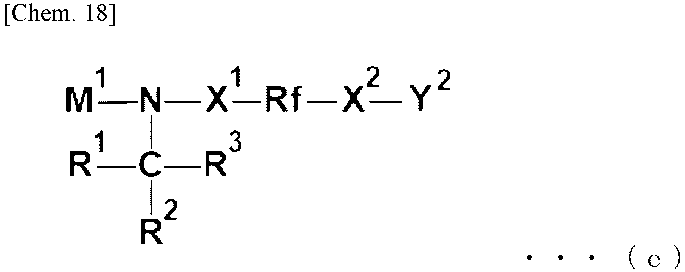

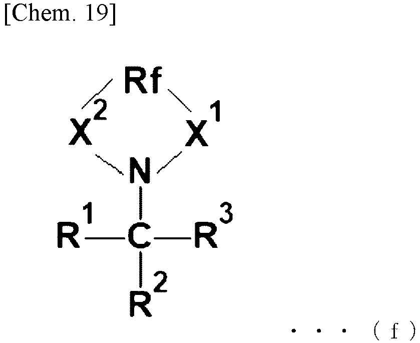

In Formulae (e) and (f), Rf is a substituted or unsubstituted divalent hydrocarbon group containing at least one fluorine atom; X¹ and X² are each independently a carbonyl group or a sulfonyl group; Y² is a halogen atom, a substituted or unsubstituted alkylcarbonyloxy group, a substituted or unsubstituted arylcarbonyloxy group, a substituted or unsubstituted alkylsulfonyloxy group, or a substituted or unsubstituted arylsulfonyloxy group; M¹ is a hydrogen atom or a counter ion; and R¹, R² and R³ are each independently a hydrogen atom, a polar group, a substituted or unsubstituted saturated hydrocarbon group, a substituted or unsubstituted unsaturated hydrocarbon group, or a substituted or unsubstituted aromatic hydrocarbon group, and at least two of R¹, R² and R³ may be bonded to each other to form a ring.

In Formula (a), Y¹, X¹, Rf, X², and Y² are the same as those described above for Formula (a) in the first aspect of the present invention.

In Formulae (e) and (f), X¹, Rf, X², and Y² are the same as those described above for Formula (a) in the first aspect of the present invention.

In Formulae (e) and (f), R¹, R² and R³ are the same as those described above for Formula (b) in the first aspect of the present invention.

Nucleophiles are the same as those described in the first aspect of the present invention.

Reaction products are the same as those described in the first aspect of the present invention.

The reaction precursor production method in the fourth aspect of the present invention includes reacting an organic compound having an amino group with an amino group eliminating agent containing a compound (a) represented by the following Formula (a) (hereinafter also referred to as a first reaction step).

Here, the first reaction step may include reacting an organic compound having an amino group with an amino group eliminating agent containing a compound (a) represented by the following Formula (a) (hereinafter also referred to as a reaction step 1a). According to the reaction step 1a, a compound (e) represented by Formula (e) can be synthesized. The first reaction step may further include obtaining a compound (f) represented by Formula (f) from a compound (e) represented by Formula (e) (hereinafter also referred to as a reaction step 1b). According to the reaction step 1b, a compound (f) represented by Formula (f) can be synthesized.

A one-pot synthesis in which the reaction step 1a and the reaction step 1b proceed continuously may be used. The one-pot synthesis is preferable because the reaction product can be efficiently produced.

When a compound (e) or a compound (f) is used to produce a reaction product, a multi-step reaction in which the reaction step 1a is performed to synthesize a compound (e), which is then isolated, the reaction step 1b is performed to synthesize a compound (f), and the second reaction step is additionally performed to synthesize a reaction product may be used or a one-pot synthesis in which the reaction step 1a and the second reaction step proceed continuously may be used. The one-pot synthesis is preferable because the reaction product can be efficiently produced.

A multi-step reaction in which the one-pot synthesis in which the reaction step 1a and the reaction step 1b proceed continuously is performed, the obtained product (compound (f)) is then isolated and the second reaction step is additionally performed may be used, or a one-pot synthesis in which the reaction step 1b and the second reaction step proceed continuously may be used. The one-pot synthesis is preferable because the reaction product can be efficiently produced.

In the reaction step 1a and the reaction step 1b, a basic substance, a solvent and the like can be used.

Basic substances and solvents are the same as those described in the second aspect of the present invention.

The amount of the basic substance used with respect to the compound (e) is preferably in a range of 0.5 mol or more and 10 mol or less and more preferably in a range of 1.5 mol or more and 3.0 mol or less relative to 1 mol of the reactive amino group (NH₂-). When the amount of the basic substance used is 0.5 mol or more relative to 1 mol of the reactive amino group (NH₂-), the amount of the reaction product produced is less likely to be less than the reaction equivalent. In addition, when the amount of the basic substance used is 10 mol or less relative to 1 mol of the reactive amino group (NH₂-), the reaction product is less likely to be decomposed by an excessive amount of a base.

The amount of the basic substance used with respect to the compound (f) is preferably in a range of 0.5 mol or more and 10 mol or less and more preferably in a range of 1.5 mol or more and 3.0 mol or less relative to 1 mol of the reactive amino group (NH₂-). When the amount of the basic substance used is 0.5 mol or more relative to 1 mol of the reactive amino group (NH₂-), the amount of the reaction product produced is less likely to be less than the reaction equivalent. **In** addition, when the amount of the basic substance used is 10 mol or less relative to 1 mol of the reactive amino group (NH₂-), the reaction product is less likely to be decomposed by an excessive amount of a base.

The amount of the nucleophile used with respect to the compound (e) is preferably in a range of 1 mol or more and 10 mol or less and in a range of 1.3 mol or more and 3.0 mol or less relative to 1.0 mol of the reactive amino group (NH₂-). When the amount of the nucleophile used is 1.0 mol or more relative to 1 mol of the reactive amino group (NH₂-), the amount of the nucleophile used can be minimized, and it is easier to reduce the production cost. In addition, when the amount of the nucleophile used is 10 mol or less relative to 1 mol of the reactive amino group (NH₂-), it is easier to shorten the reaction time, the amount of the nucleophile used is reduced, and it is easier to reduce the production cost.

The amount of the nucleophile used with respect to the compound (f) is preferably in a range of 1 mol or more and 10 mol or less and in a range of 1.3 mol or more and 3.0 mol or less relative to 1.0 mol of the reactive amino group (NH₂-). When the amount of the nucleophile used is 1.0 mol or more relative to 1 mol of the reactive amino group (NH₂-), the amount of the nucleophile used can be minimized, and it is easier to reduce the production cost. In addition, when the amount of the nucleophile used is 10 mol or less relative to 1 mol of the reactive amino group (NH₂-), it is easier to shorten the reaction time, the amount of the nucleophile used is reduced, and it is easier to reduce the production cost.

The reaction temperatures in the reaction step 1a and the reaction step 1b are both preferably in a range of 0°C or higher and a boiling point of a solvent or lower. The reaction proceeds easily when the reaction temperature is 0°C or higher. In addition, it is preferable to perform the reaction step 1a and the reaction step 1b under conditions in which the solvent is refluxed. The reaction temperature in the reaction step 1b is preferably higher than the reaction temperature in the reaction step 1a. [Reaction temperature in the reaction step 1b]-[reaction temperature in the reaction step 1a] is preferably in a range of higher than 0°C and a boiling point of a solvent or lower and more preferably in a range of 30°C or higher and 100°C or shorter. When the difference between the reaction temperatures is within the above range, decomposition of the reaction intermediate and production of by-products are easily minimized and the yield of the reaction product is easily improved.

The reaction times in the reaction step 1a and the reaction step 1b are preferably in a range of 1 hour or longer and 3 hours or shorter. The reaction time in the reaction step 1b is preferably longer than the reaction time in the reaction step 1a. [Reaction time in the reaction step 1b]-[reaction time in the reaction step 1a] is preferably in a range of longer than 0 hours and 3 hours or shorter and more preferably in a range of 30 minutes or longer and 1 hour or shorter. When the difference between the reaction times is within the above range, decomposition of the reaction intermediate and production of by-products are easily minimized and the yield of the reaction product is easily improved.

When the reaction is slow, the reaction can also be performed in an autoclave or under microwave radiation. The reaction temperature is preferably in a range of 100 to 200°C and more preferably in a range of 130 to 150°C. The reaction time is preferably in a range of 10 minutes or longer and 3 hours or shorter and more preferably in a range of 30 minutes or longer and 1 hour or shorter.

According to the reaction precursor production method in the fourth aspect of the present invention, it is possible to provide a reaction precursor that can be reacted with various nucleophiles and allows a desired reaction product to be obtained in just a single step. Therefore, it is possible to provide a synthetic intermediate that is beneficial in the fields of pharmaceuticals, agricultural chemicals, electronic materials, functional materials and the like.

<Optically active reaction product production method>

A fifth aspect of the present invention is an optically active reaction product production method including reacting an optically active organic compound having an amino group with an amino group eliminating agent containing a compound (a) represented by the following Formula (a) and additionally reacting the optically active organic compound with a nucleophile.

Y¹-X¹-Rf-X²-Y² ··· (a)

**In** Formula (a), Rf is a substituted or unsubstituted divalent hydrocarbon group containing at least one fluorine atom; X¹ and X² are each independently a carbonyl group or a sulfonyl group; and Y¹ and Y² are each independently a halogen atom, a substituted or unsubstituted alkylcarbonyloxy group, a substituted or unsubstituted arylcarbonyloxy group, a substituted or unsubstituted alkylsulfonyloxy group, or a substituted or unsubstituted arylsulfonyloxy group.

In Formula (a), Y¹, X¹, Rf, X², and Y² are the same as those described above for Formula (a) in the first aspect of the present invention.

The optically active organic compound is preferably represented by the following Formula (b*).

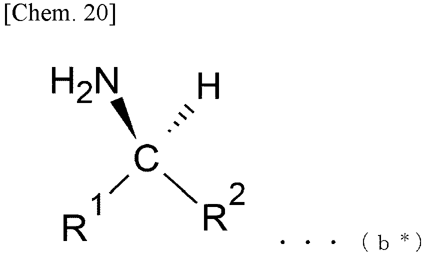

In Formula (b*), R¹ and R² are not the same and each independently a polar group, a substituted or unsubstituted saturated hydrocarbon group, a substituted or unsubstituted unsaturated hydrocarbon group, or a substituted or unsubstituted aromatic hydrocarbon group, and R¹ and R² may be bonded to each other to form a ring.

In Formula (b*), R¹ and R² are the same as those described above for Formula (b) in the first aspect of the present invention.

Nucleophiles are the same as those described in the first aspect of the present invention.

The optically active reaction product is preferably represented by the following Formula (d*).

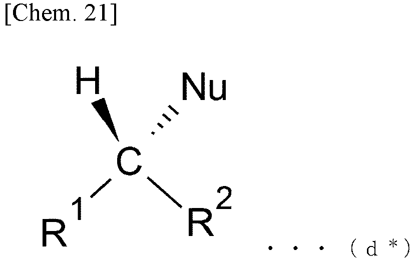

In Formula (d*), R¹ and R² are not the same and each independently a polar group, a substituted or unsubstituted saturated hydrocarbon group, a substituted or unsubstituted unsaturated hydrocarbon group, or a substituted or unsubstituted aromatic hydrocarbon group, and R¹ and R² may be bonded to each other to form a ring.

In Formula (d*), R¹ and R² are the same as those described above for Formula (b) in the first aspect of the present invention.

According to the optically active reaction product production method in the fifth aspect of the present invention, when a compound having two reactive sites in the same molecule is used as a deaminating agent in order to directly substitute the amino group constituting an optically active primary alkylamine compound, which could not be converted using conventional nucleophiles, with a fluorine group or the like, optically active fluorinated alkyl compounds and the like can be efficiently produced. This makes it possible to synthesize reaction products such as an optically active fluorine compound, which have been difficult to synthesize in the related art, and simplify the synthesis step. In addition, for optically active reaction products, which have been difficult to synthesize, by using optically active primary alkylamines, which are abundant in natural products such as amino acids, as synthetic raw materials, optically active fluorinated alkyl compounds can be efficiently produced at a low cost.

While the embodiments of the present invention have been described above, the technical scope of the present invention is not limited to the embodiments, and various modifications can be made without departing from the spirit of the present invention.

### EXAMPLES

The present invention will be described below in more detail with reference to examples, but the present invention is not limited to these examples.

Unless otherwise specified, in examples and comparative examples, reaction products were synthesized under the same reaction conditions as in Example 1.

In the examples and comparative examples, the NMR instrument used for analysis was AVANCE 400 (commercially available from Bruker) (¹H-NMR: 400 MHz, ¹⁹F-NMR: 376 MHz). For ¹H-NMR, tetramethylsilane was used as an internal standard (0 ppm), and for ¹⁹F-NMR, trichlorofluoromethane was used as an internal standard (0 ppm).

### [Example 1]

An alkyl iodide compound represented by the following Formula (d-1) was synthesized using a primary alkylamine represented by the following Formula (b-1).

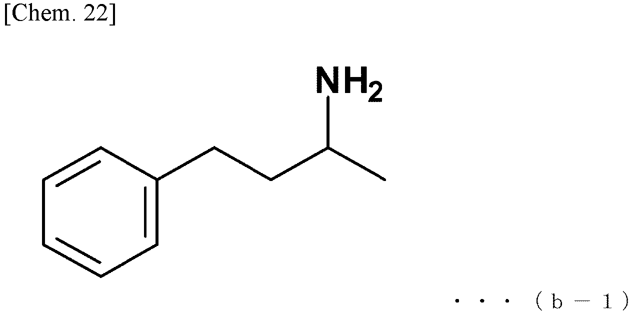

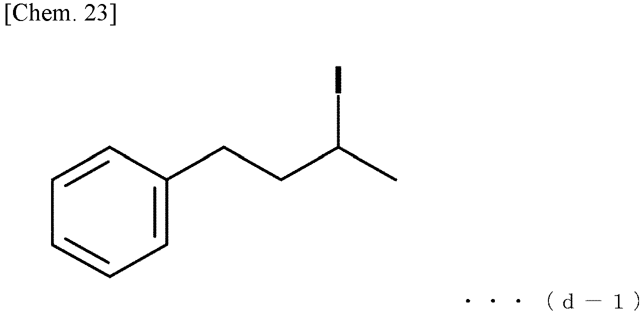

Under a nitrogen atmosphere, 3-amino-1-phenylbutane (74.6 mg, 0.50 mmol), 1,1,2,2,3,3-hexafluoropropane-1,3-disulfonyl fluoride (316.2 mg, 1.0 mmol), and sodium iodide (374.7 mg, 2.5 mmol) were added to toluene (10 mL) and heated to 110°C. Diazabicycloundecene (DBU) (152.2 mg, 1.0 mmol) was slowly added thereto, and the mixture was stirred at 110°C for 1 hour and then quenched with a 2 M hydrochloric acid aqueous solution (10 mL). Chloroform (10 mL) was added to the obtained reaction solution, liquid separation was performed, the organic layer was then washed once with a 2 M hydrochloric acid aqueous solution (10 mL), and the obtained organic layer was washed twice with a saturated sodium carbonate aqueous solution (10 mL). The washed organic layer was dried over magnesium sulfate, and the solvent was then distilled off under a reduced pressure. The obtained crude product was purified through silica gel column chromatography (hexane/ethyl acetate=100/1) and the solvent was distilled off under a reduced pressure to obtain a compound represented by Formula (d1-1) in a yield of 41%.

By NMR analysis, it was confirmed that a compound represented by Formula (d1-1) was obtained.
¹H-NMR (400 MHz, CDCl₃)δ(ppm)=7.31 to 7.26(m, 2H), 7.21 to 7.18(m, 3H), 4.14 to 4.09(m, 1H), 2.88 to 2.66(m, 2H), 2.20 to 2.11(m, 1H), 1.95(d, 3H), 1.92 to 1.84(m, 1H).

### [Example 2]

An alkyl bromide compound represented by the following Formula (d-2) was synthesized using a primary alkylamine represented by the following Formula (b-2).

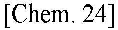

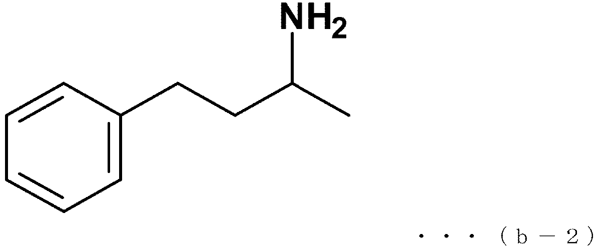

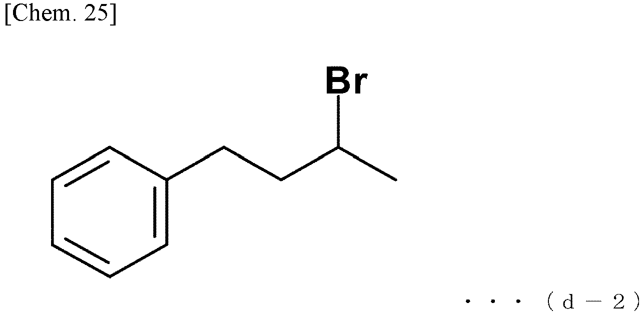

Under a nitrogen atmosphere, 3-amino-1-phenylbutane (74.6 mg, 0.50 mmol), 1,1,2,2,3,3-hexafluoropropane-1,3-disulfonyl fluoride (316.2 mg, 1.0 mmol), and sodium bromide (257.2 mg, 2.5 mmol) were added to toluene (10 mL) and heated to 110°C. Diazabicycloundecene (DBU) (152.2 mg, 1.0 mmol) was slowly added thereto, and the mixture was stirred at 110°C for 1 hour and then quenched with a 2 M hydrochloric acid aqueous solution (10 mL). Chloroform (10 mL) was added to the obtained reaction solution, liquid separation was performed, the organic layer was then washed once with a 2 M hydrochloric acid aqueous solution (10 mL), and the obtained organic layer was washed twice with a saturated sodium carbonate aqueous solution (10 mL). The washed organic layer was dried over magnesium sulfate, and the solvent was then distilled off under a reduced pressure. The obtained crude product was purified through silica gel column chromatography (hexane/ethyl acetate=100/1) and the solvent was distilled off under a reduced pressure to obtain a compound represented by Formula (d-2) in a yield of 45%.

By NMR analysis, it was confirmed that a compound represented by Formula (d-2) was obtained.
¹H-NMR (400 MHz, CDCl₃)δ(ppm)=7.31 to 7.27(m, 2H), 7.21 to 7.18(m, 3H), 4.12 to 4.04(m, 1H), 2.90 to 2.69(m, 2H), 2.19 to 1.96(m, 2H), 1.73(d, 3H).

### [Example 3]

An alkyl chloride compound represented by the following Formula (d-3) was synthesized using a primary alkylamine represented by the following Formula (b-3).

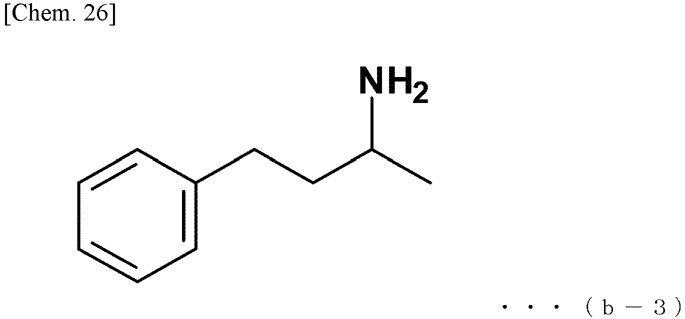

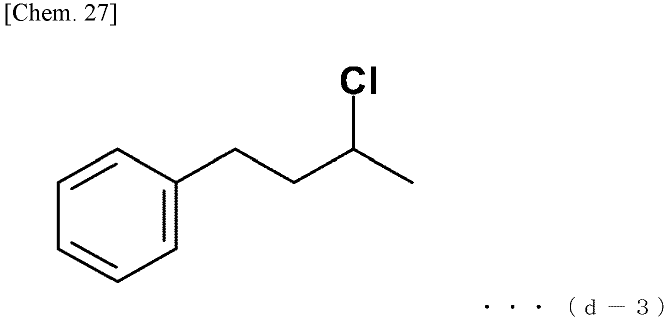

Under a nitrogen atmosphere, 3-amino-1-phenylbutane (74.6 mg, 0.50 mmol), 1,1,2,2,3,3-hexafluoropropane-1,3-disulfonyl fluoride (316.2 mg, 1.0 mmol), and tetrabutylammonium chloride (694.7 mg, 2.5 mmol) were added to toluene (10 mL) and heated to 110°C. Diazabicycloundecene (DBU) (152.2 mg, 1.0 mmol) was slowly added thereto, and the mixture was stirred at 110°C for 1 hour and then quenched with a 2 M hydrochloric acid aqueous solution (10 mL). Chloroform (10 mL) was added to the obtained reaction solution, liquid separation was performed, the organic layer was then washed once with a 2 M hydrochloric acid aqueous solution (10 mL), and the obtained organic layer was washed twice with a saturated sodium carbonate aqueous solution (10 mL). The washed organic layer was dried over magnesium sulfate, and the solvent was then distilled off under a reduced pressure. The obtained crude product was purified through silica gel column chromatography (hexane/ethyl acetate=100/1) and the solvent was distilled off under a reduced pressure to obtain a compound represented by Formula (d-3) in a yield of 75%.

By NMR analysis, it was confirmed that a compound represented by Formula (d-3) was obtained.
¹H-NMR (400 MHz, CDCl₃)δ(ppm)=7.31 to 7.27(m, 2H), 7.21 to 7.18(m, 3H), 4.03 to 3.95(m, 1H), 2.89 to 2.71(m, 2H), 2.08 to 1.95(m, 2H), 1.53(d, 3H).

### [Example 4]

A fluorinated alkyl compound represented by the following Formula (d-4) was synthesized using a primary alkylamine compound represented by the following Formula (b-4).

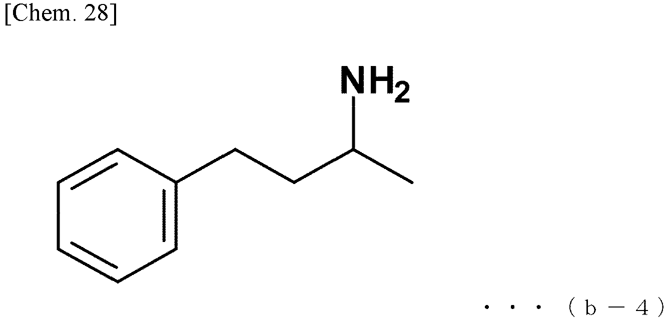

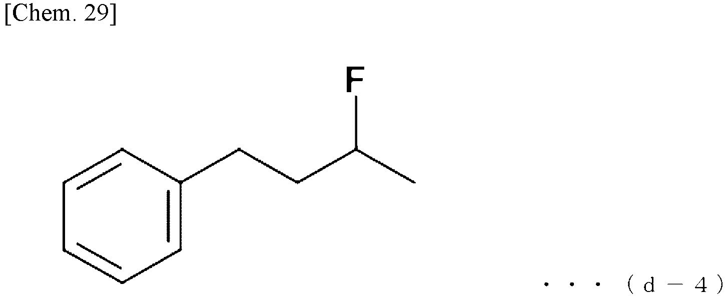

Under a nitrogen atmosphere, 3-amino-1-phenylbutane (74.6 mg, 0.50 mmol), 1,1,2,2,3,3-hexafluoropropane-1,3-disulfonyl fluoride (316.2 mg, 1.0 mmol), and tetrabutylammonium difluorotriphenylsilicate (TBAT) (135.0 mg, 0.25 mmol) were added to toluene (10 mL) and heated to 110°C. Diazabicycloundecene (DBU) (152.2 mg, 1.0 mmol) was slowly added thereto, the mixture was stirred at 110°C for 1 hour and then quenched with a 2 M hydrochloric acid aqueous solution (10 mL) to obtain a reaction solution.

Chloroform (10 mL) was added to the obtained reaction solution, liquid separation was performed, the organic layer was then washed once with a 2 M hydrochloric acid aqueous solution (10 mL), and the obtained organic layer was washed twice with a saturated sodium carbonate aqueous solution (10 mL). The washed organic layer was dried over magnesium sulfate, and the solvent was then distilled off under a reduced pressure. The obtained crude product was purified through silica gel column chromatography (hexane/ethyl acetate=100/1) and the solvent was distilled off under a reduced pressure to obtain a fluorinated alkyl compound represented by Formula (d-4) in a yield of 53%.

By NMR analysis, it was confirmed that a fluorinated alkyl compound represented by Formula (d-4) was obtained.
¹H-NMR (400 MHz, CDCl₃)δ(ppm)=7.31 to 7.18(m, 5H), 4.75 to 4.56(m, 1H), 2.84 to 2.65(m, 2H), 2.05 to 1.70(m, 2H), 1.55 to 1.37(m, 3H).
¹⁹F-NMR (376 MHz, CDCl₃)δ(ppm)=-174.04 to -174.36(m, 1F).

### [Example 5]

A primary alkylamine represented by the following Formula (b-5) was used and its amino group was substituted with an -OCH₂CF₃ group to synthesize an alkyl ether compound represented by the following Formula (d-5).

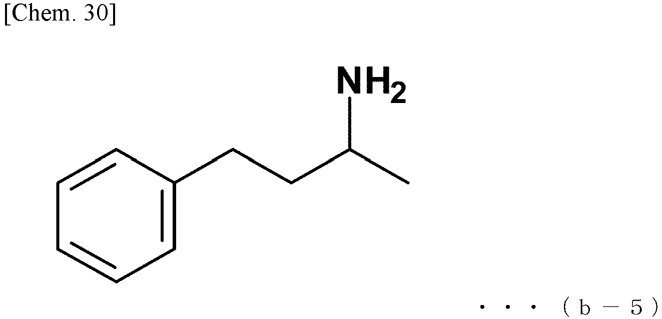

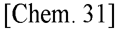

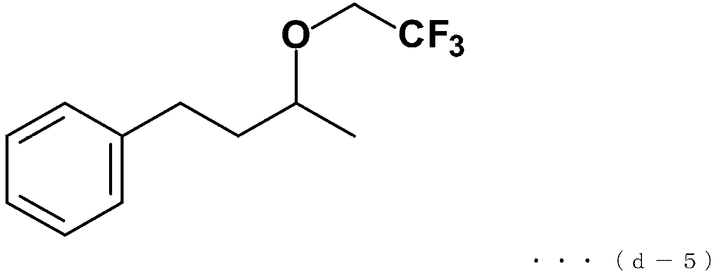

Under a nitrogen atmosphere, 3-amino-1-phenylbutane (74.6 mg, 0.50 mmol), 1,1,2,2,3,3-hexafluoropropane-1,3-disulfonyl fluoride (316.2 mg, 1.0 mmol), and 2, 2, 2-trifluoroethanol (250.1 mg, 2.5 mmol) were added to toluene (10 mL) and heated to 110°C. Diazabicycloundecene (DBU) (152.2 mg, 1.0 mmol) was slowly added thereto, and the mixture was stirred at 110°C for 1 hour and then quenched with a 2 M hydrochloric acid aqueous solution (10 mL). Chloroform (10 mL) was added to the obtained reaction solution, liquid separation was performed, the organic layer was then washed once with a 2 M hydrochloric acid aqueous solution (10 mL), and the obtained organic layer was washed twice with a saturated sodium carbonate aqueous solution (10 mL). The washed organic layer was dried over magnesium sulfate, and the solvent was then distilled off under a reduced pressure. The obtained crude product was purified through silica gel column chromatography (hexane/ethyl acetate=10/1) and the solvent was distilled off under a reduced pressure to obtain a compound represented by Formula (d-5) in a yield of 67%.

By NMR analysis, it was confirmed that a compound represented by Formula (d-5) was obtained.
¹H-NMR (400 MHz, CDCl₃)δ(ppm)=7.29 to 7.26(m, 2H), 7.19 to 7.16(m, 3H), 3.25 to 3.06(m, 2H), 2.81 to 2.71(m, 1H), 2.70 to 2.63(m, 2H), 1.79 to 1.60(m, 2H), 1.11(d, 3H).
¹⁹F-NMR (376 MHz, CDCl₃)δ(ppm)=-72.15 to -72.20(m, 3F).

### [Example 6]

A primary alkylamine represented by the following Formula (b-6) was used and its amino group was substituted with an -OCH₂CF₂CF₃ group to synthesize an alkyl ether compound represented by the following Formula (b-6).

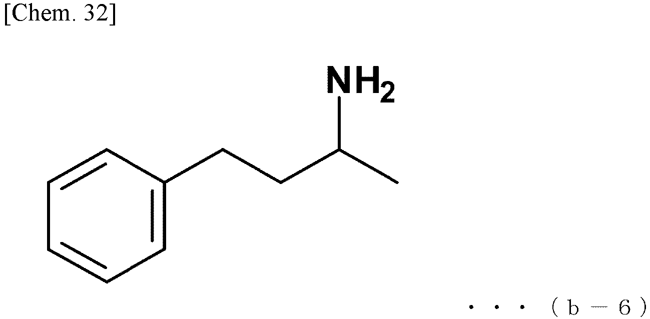

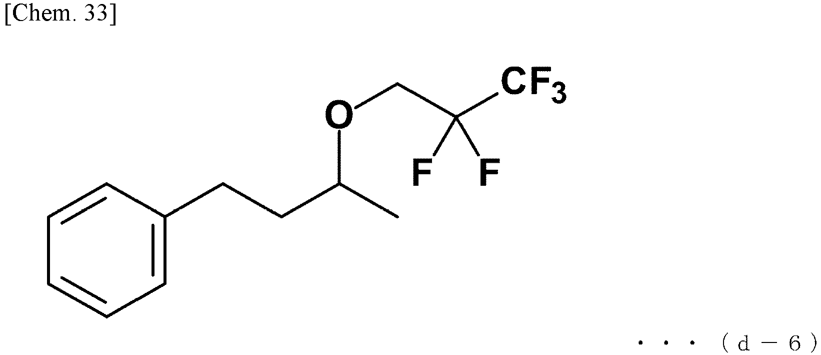

Under a nitrogen atmosphere, 3-amino-1-phenylbutane (74.6 mg, 0.50 mmol), 1,1,2,2,3,3-hexafluoropropane-1,3-disulfonyl fluoride (316.2 mg, 1.0 mmol), and 2,2,3,3,3-pentafluoro-1-propanol (187.6 mg, 1.25 mmol) were added to toluene (10 mL) and heated to 110°C. Diazabicycloundecene (DBU) (152.2 mg, 1.0 mmol) was slowly added thereto, and the mixture was stirred for 1 hour and then quenched with a 2 M hydrochloric acid aqueous solution (10 mL). Chloroform (10 mL) was added to the obtained reaction solution, liquid separation was performed, the organic layer was then washed once with a 2 M hydrochloric acid aqueous solution (10 mL), and the obtained organic layer was washed twice with a saturated sodium carbonate aqueous solution (10 mL). The washed organic layer was dried over magnesium sulfate, and the solvent was then distilled off under a reduced pressure. The obtained crude product was purified through silica gel column chromatography (hexane/ethyl acetate=10/1) and the solvent was distilled off under a reduced pressure to obtain a compound represented by Formula (d-6) in a yield of 44%.

By NMR analysis, it was confirmed that a compound represented by Formula (d-6) was obtained.
¹H-NMR (400 MHz, CDCl₃)δ(ppm)=7.30 to 7.26(m, 2H), 7.20 to 7.17(m, 3H), 3.30 to 3.08(m, 2H), 2.78 to 2.71(m, 1H), 1.78 to 1.60(m, 2H), 1.79 to 1.60(m, 2H), 1.12(d, 3H).
¹⁹F-NMR (376 MHz, CDCl₃)δ(ppm)=-83.80 to -83.83(m, 3F), -121.81 to -121.89(m, 2F).

### [Example 7]

A primary alkylamine represented by the following Formula (b-7) was used and its amino group was substituted with an -OCH₂CF₂CF₂H group to synthesize an alkyl ether compound represented by the following Formula (d-7).

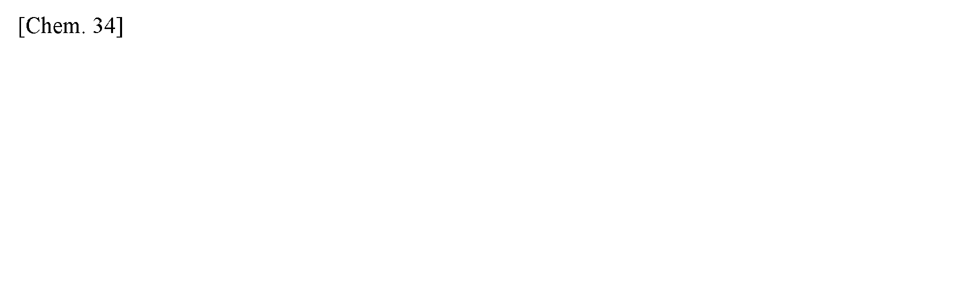

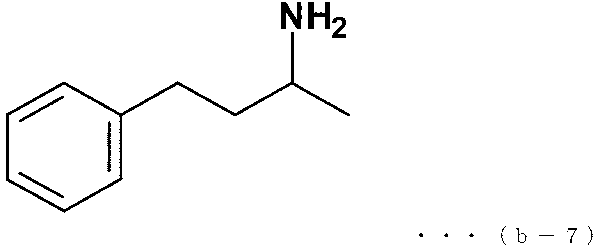

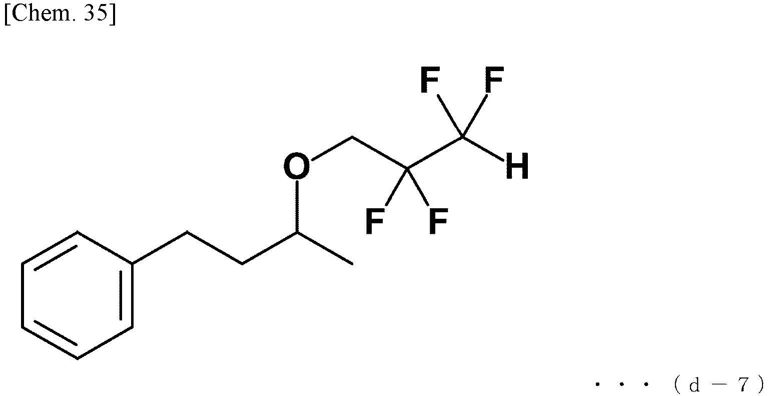

Under a nitrogen atmosphere, 3-amino-1-phenylbutane (74.6 mg, 0.50 mmol), 1,1,2,2,3,3-hexafluoropropane-1,3-disulfonyl fluoride (316.2 mg, 1.0 mmol), and 2,2,3,3-tetrafluoro-1-propanol (165.1 mg, 1.25 mmol) were added to toluene (10 mL) and heated to 110°C. Diazabicycloundecene (DBU) (152.2 mg, 1.0 mmol) was slowly added thereto, and the mixture was stirred at 110°C for 1 hour and then quenched with a 2 M hydrochloric acid aqueous solution (10 mL). Chloroform (10 mL) was added to the obtained reaction solution, liquid separation was performed, the organic layer was then washed once with a 2 M hydrochloric acid aqueous solution (10 mL), and the obtained organic layer was washed twice with a saturated sodium carbonate aqueous solution (10 mL). The washed organic layer was dried over magnesium sulfate, and the solvent was then distilled off under a reduced pressure. The obtained crude product was purified through silica gel column chromatography (hexane/ethyl acetate=10/1) and the solvent was distilled off under a reduced pressure to obtain a compound represented by Formula (d-7) in a yield of 37%.

By NMR analysis, it was confirmed that a compound represented by Formula (d-7) was obtained.
¹H-NMR (400 MHz, CDCl₃)δ(ppm)=7.30 to 7.26(m, 2H), 7.23 to 7.15(m, 3H), 6.17 to 5.87(m, 1H), 3.24 to 3.01(m, 2H), 2.78 to 2.58(m, 3H), 1.79 to 1.60(m, 2H), 1.12(d, 3H).
¹⁹F-NMR (376 MHz, CDCl₃)δ(ppm)=-122.72 to -123.01(m, 2F), -139.57 to -139.88(m, 2F).

### [Example 8]

A primary alkylamine represented by the following Formula (b-8) was used and its amino group was substituted with an -OCH₂CH₂CF₂CF₂CF₂CF₂CF₂CF₃ group to synthesize an alkyl ether compound represented by the following Formula (d-8).

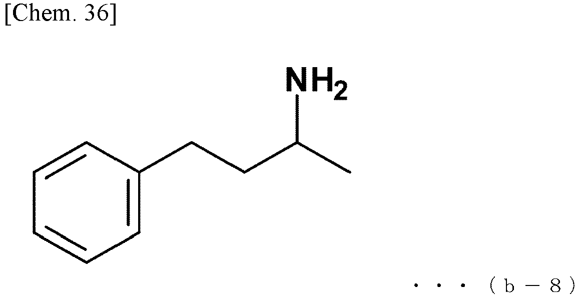

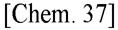

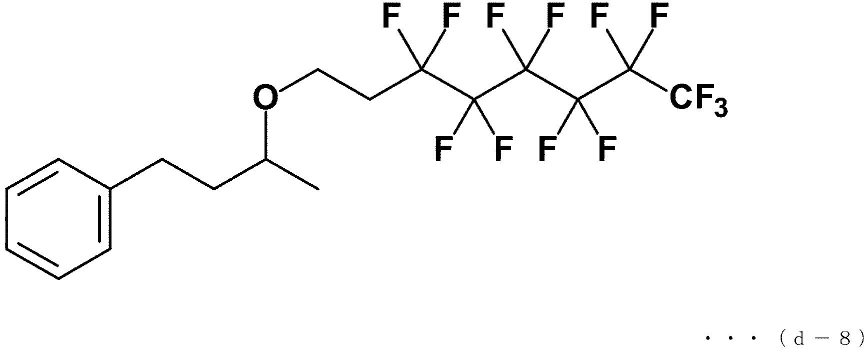

Under a nitrogen atmosphere, 3-amino-1-phenylbutane (74.6 mg, 0.50 mmol), 1,1,2,2,3,3-hexafluoropropane-1,3-disulfonyl fluoride (316.2 mg, 1.0 mmol), and 1H,1H,2H,2H-tridecafluoro-1-n-octanol (455, 1 mg, 1.25 mmol) were added to toluene (10 mL) and heated to 110°C. Diazabicycloundecene (DBU) (152.2 mg, 1.0 mmol) was slowly added thereto, and the mixture was stirred at 110°C for 1 hour and then quenched with a 2 M hydrochloric acid aqueous solution (10 mL). Chloroform (10 mL) was added to the obtained reaction solution, liquid separation was performed, the organic layer was then washed once with a 2 M hydrochloric acid aqueous solution (10 mL), and the obtained organic layer was washed twice with a saturated sodium carbonate aqueous solution (10 mL). The washed organic layer was dried over magnesium sulfate, and the solvent was then distilled off under a reduced pressure. The obtained crude product was purified through silica gel column chromatography (hexane/ethyl acetate=10/1) and the solvent was distilled off under a reduced pressure to obtain a compound represented by Formula (d-8) in a yield of 18%.

By NMR analysis, it was confirmed that a compound represented by Formula (d-8) was obtained.
¹H-NMR (400 MHz, CDCl₃)δ(ppm)=7.30 to 7.26(m, 2H), 7.20 to 7.17(m, 3H), 3.01 to 2.85(m, 2H), 2.74 to 2.59(m, 3H), 2.33 to 2.18(m, 2H), 1.81 to 1.60(m, 2H), 1.12(d, 3H).
¹⁹F-NMR (376 MHz, CDCl₃)δ(ppm)=-80.74 to -80.79(m, 3F), -113.55 to -113.64(m, 2F), - 121.89(m, 2F), -122.86(m, 2F), -123.66(m, 2F), -126.07 to -126.17(m, 2F).

### [Example 9]

An alkylazide compound represented by the following Formula (d-9) was synthesized using a primary alkylamine represented by the following Formula (b-9).

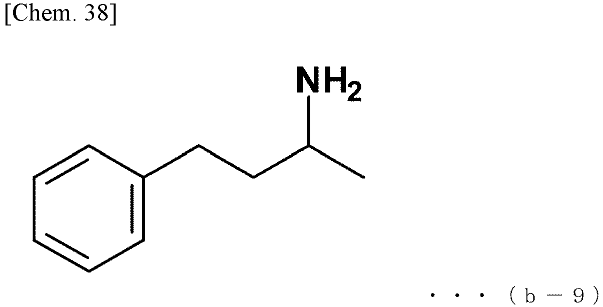

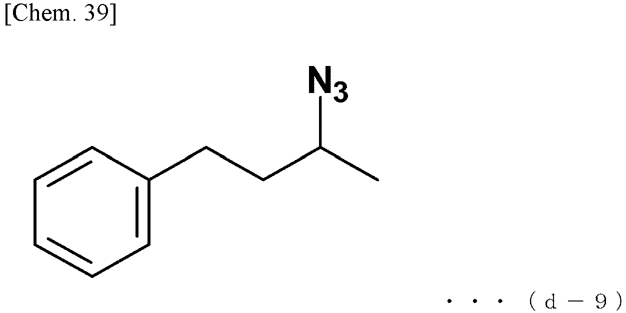

Under a nitrogen atmosphere, 3-amino-1-phenylbutane (74.6 mg, 0.50 mmol), 1,1,2,2,3,3-hexafluoropropane-1,3-disulfonyl fluoride (316.2 mg, 1.0 mmol), and trimethylsilyl azide (144.0 mg, 1.25 mmol) were added to toluene (10 mL) and heated to 110°C. Diazabicycloundecene (DBU) (152.2 mg, 1.0 mmol) was slowly added thereto, and the mixture was stirred at 110°C for 1 hour and then quenched with a 2 M hydrochloric acid aqueous solution (10 mL). Chloroform (10 mL) was added to the obtained reaction solution, liquid separation was performed, the organic layer was then washed once with a 2 M hydrochloric acid aqueous solution (10 mL), and the obtained organic layer was washed twice with a saturated sodium carbonate aqueous solution (10 mL). The washed organic layer was dried over magnesium sulfate, and the solvent was then distilled off under a reduced pressure. The obtained crude product was purified through silica gel column chromatography (hexane/ethyl acetate=10/1) and the solvent was distilled off under a reduced pressure to obtain a compound represented by Formula (d-9) in a yield of 64%.

By NMR analysis, it was confirmed that a compound represented by Formula (d-9) was obtained.
¹H-NMR (400 MHz, CDCl₃)δ(ppm)=7.31 to 7.27(m, 2H), 7.23 to 7.17(m, 3H), 3.45 to 3.40(m, 1H), 2.78 to 2.62(m, 2H), 1.85 to 1.75(m, 2H), 1.28(d, 3H).

### [Example 10]

The reaction was performed in the same manner as in Example 1 except that a primary alkylamine represented by the following Formula (b-10) was used in place of a primary alkylamine represented by Formula (b-1) and reacted with various nucleophiles to obtain a reaction product represented by the following Formula (d-10).

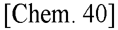

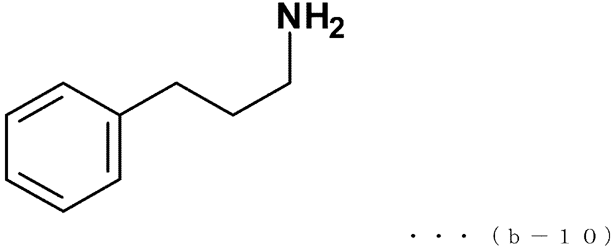

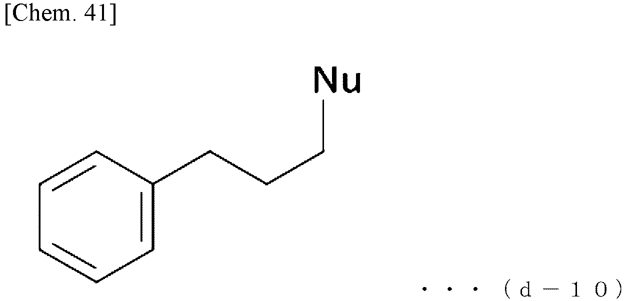

### [Example 11]

A fluorinated alkyl compound represented by the following Formula (d-11) was synthesized using a primary alkylamine represented by Formula (b-10) and tetrabutylammonium difluorotriphenylsilicate (TBAT) as a nucleophile. The yield was 35%.

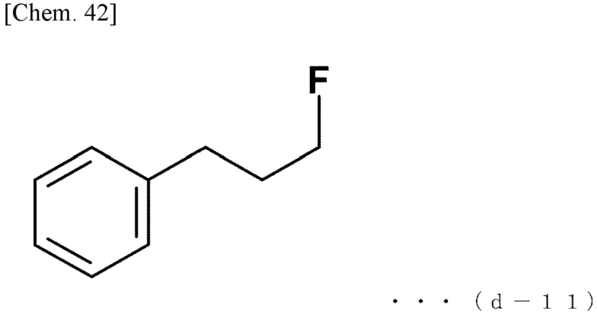

¹⁹F-NMR (376 MHz, CDCl₃)δ(ppm)=-219.94 to -220.32(m, 1F).

### [Example 12]

The reaction was performed in the same manner as in Example 1 except that a primary alkylamine represented by the following Formula (b-12) was used in place of a primary alkylamine represented by Formula (b-1) and reacted with various nucleophiles to obtain a reaction product represented by the following Formula (d-12).

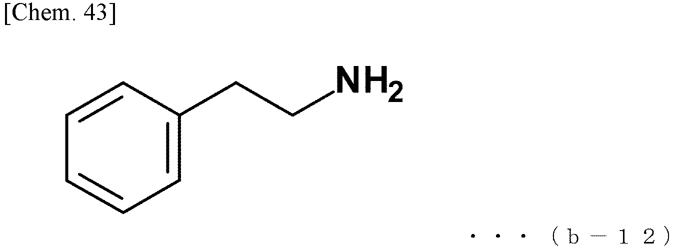

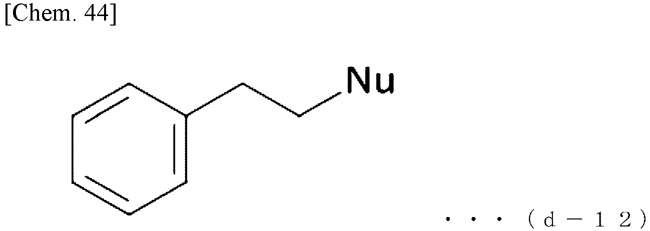

### [Example 13]

A fluorinated alkyl compound represented by the following Formula (d-13) was synthesized using a primary alkylamine represented by Formula (b-12) and tetrabutylammonium difluorotriphenylsilicate (TBAT) as a nucleophile. The yield was 31%.

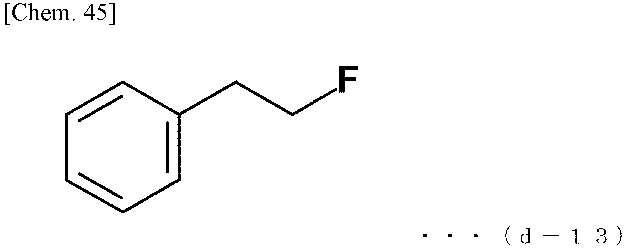

¹⁹F-NMR (376 MHz, CDCl₃)δ(ppm)=-215.02-215.39(m, 1F).

### [Example 14]

The reaction was performed in the same manner as in Example 1 except that a primary alkylamine represented by the following Formula (b-14) was used in place of a primary alkylamine represented by Formula (b-1) and reacted with various nucleophiles to obtain a reaction product represented by the following Formula (d-14).

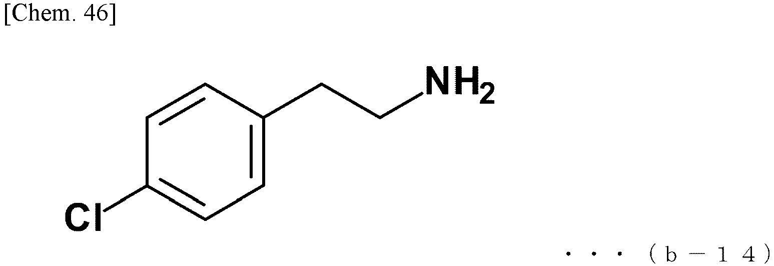

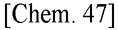

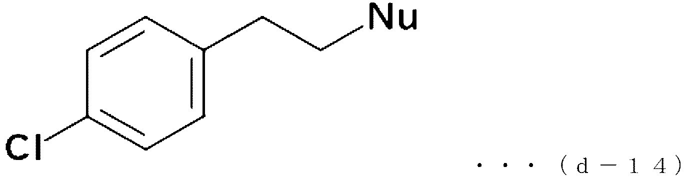

### [Example 15]

A fluorinated alkyl compound represented by the following Formula (d-15) was synthesized using a primary alkylamine represented by Formula (b-14) and tetrabutylammonium difluorotriphenylsilicate (TBAT) as a nucleophile. The yield was 44%.

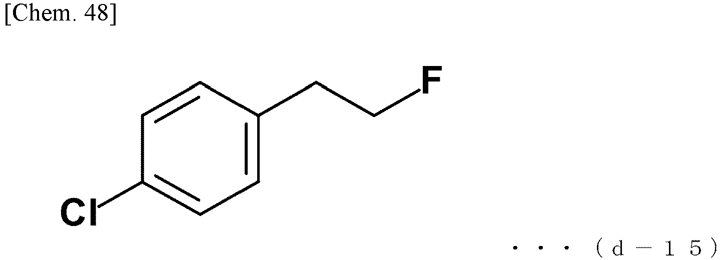

¹⁹F-NMR (376 MHz, CDCl₃)δ(ppm)=-215.87 to -216.24(m, 1F).

### [Example 16]

The reaction was performed in the same manner as in Example 1 except that a primary alkylamine represented by the following Formula (b-16) was used in place of a primary alkylamine represented by Formula (b-1) and reacted with various nucleophiles to obtain a reaction product represented by the following Formula (d-16).

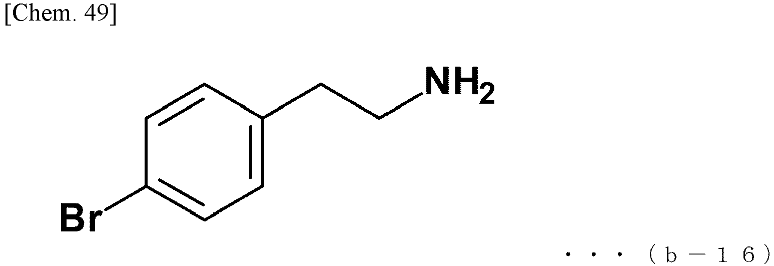

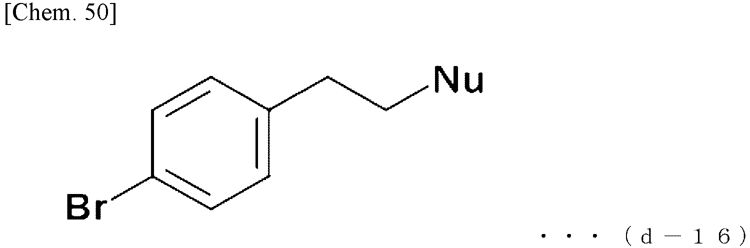

### [Example 17]

A fluorinated alkyl compound represented by the following Formula (d-17) was synthesized using a primary alkylamine represented by Formula (b-16) and tetrabutylammonium difluorotriphenylsilicate (TBAT) as a nucleophile. The yield was 32%.

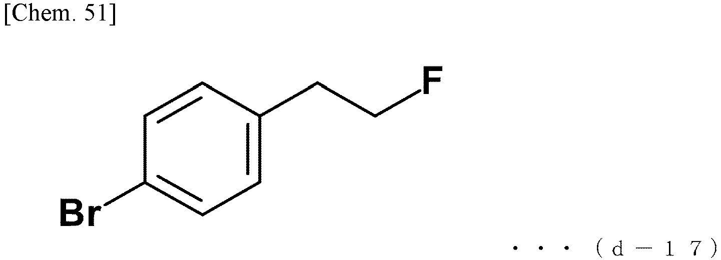

¹⁹F-NMR (376 MHz, CDCl₃)δ(ppm)=-215.88 to -216.25(m, 1F).

### [Example 18]

The reaction was performed in the same manner as in Example 1 except that a primary alkylamine represented by the following Formula (b-18) was used in place of a primary alkylamine represented by Formula (b-1) and reacted with various nucleophiles to obtain a reaction product represented by the following Formula (d-18).

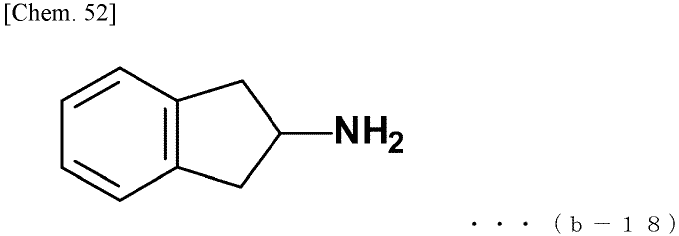

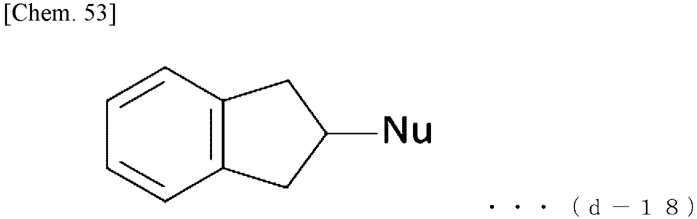

### [Example 19]

A fluorinated alkyl compound represented by the following Formula (d-19) was synthesized using a primary alkylamine represented by Formula (b-18) and tetrabutylammonium difluorotriphenylsilicate (TBAT) as a nucleophile. The yield was 28%.

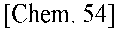

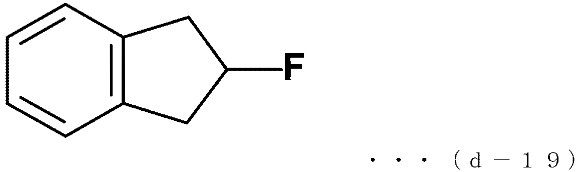

¹⁹F-NMR (376 MHz, CDCl₃)δ(ppm)=-173.62 to -174.09(m, 1F).

### [Example 20]

The reaction was performed in the same manner as in Example 1 except that a primary alkylamine represented by the following Formula (b-20) was used in place of a primary alkylamine represented by Formula (b-1), and reacted with various nucleophiles to obtain a reaction product represented by the following Formula (d-20).

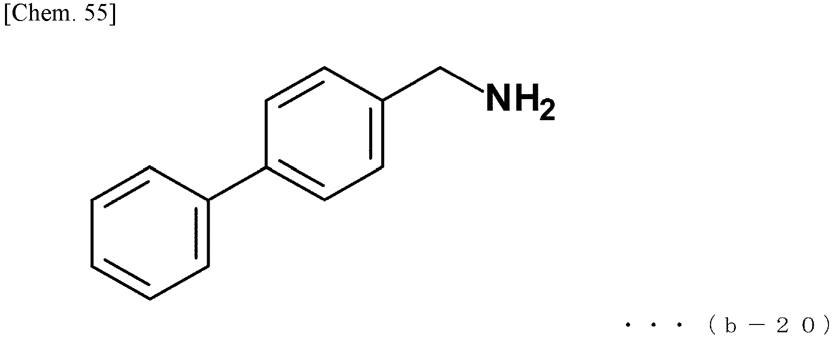

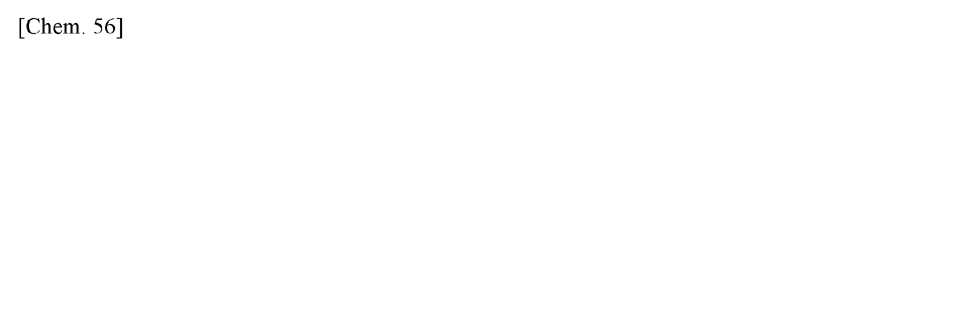

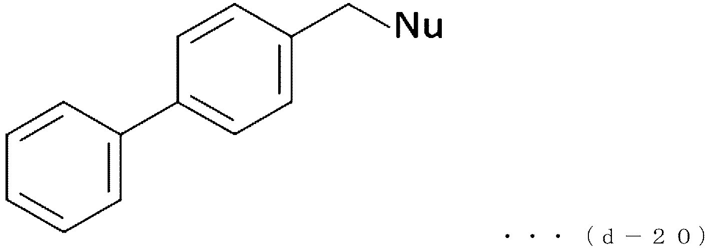

### [Example 21]

A fluorinated alkyl compound represented by the following Formula (d-21) was synthesized using a primary alkylamine represented by Formula (b-20) and tetrabutylammonium difluorotriphenylsilicate (TBAT) as a nucleophile. The yield was 14%.

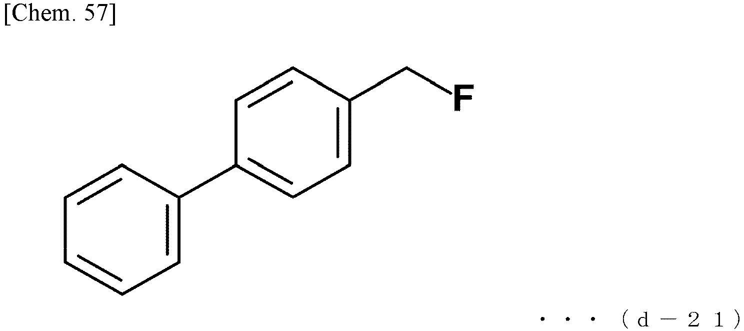

¹⁹F-NMR (376 MHz, CDCl₃)δ(ppm)=-206.29(t, 1F).

### [Example 22]

The reaction was performed in the same manner as in Example 1 except that a primary alkylamine represented by the following Formula (b-22) was used in place of a primary alkylamine represented by Formula (b-1) and reacted with various nucleophiles to obtain a reaction product represented by the following Formula (d-22).

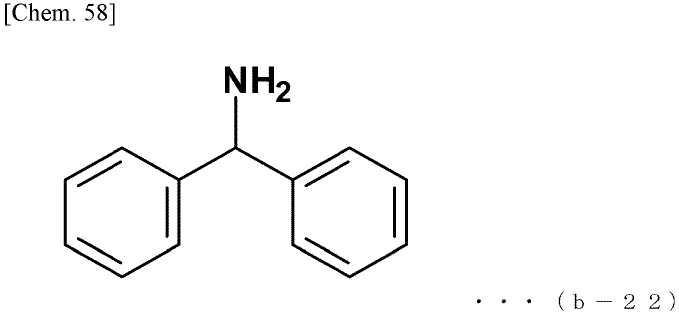

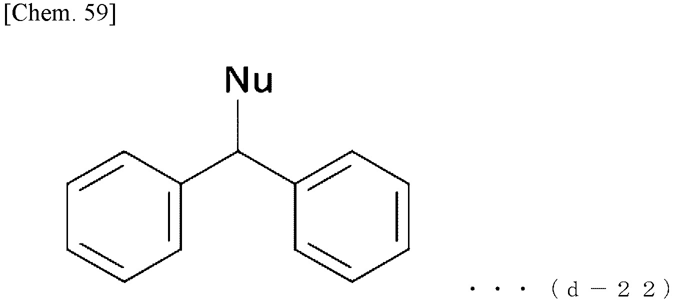

### [Example 23]

An alkyl iodide compound represented by the following Formula (d-23) was synthesized using a primary alkylamine represented by Formula (b-22) and tetrabutylammonium iodide as a nucleophile. The yield was 19%.

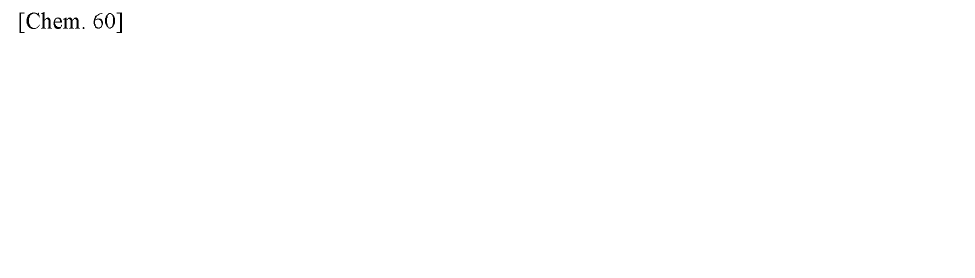

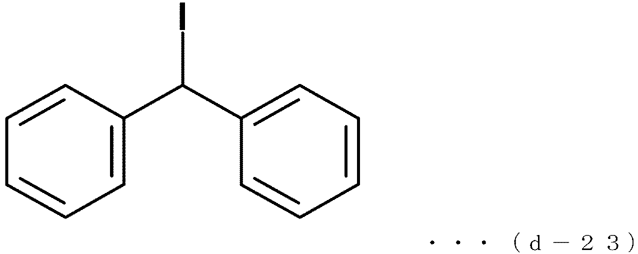

¹H-NMR (400 MHz, CDCl₃)δ(ppm)=7.39 to 7.29(m, 10H), 6.41(s, 1H).

### [Example 24]

An alkyl bromide compound represented by the following Formula (d-24) was synthesized using a primary alkylamine represented by Formula (b-22) and tetrabutylammonium bromide as a nucleophile. The yield was 18%.

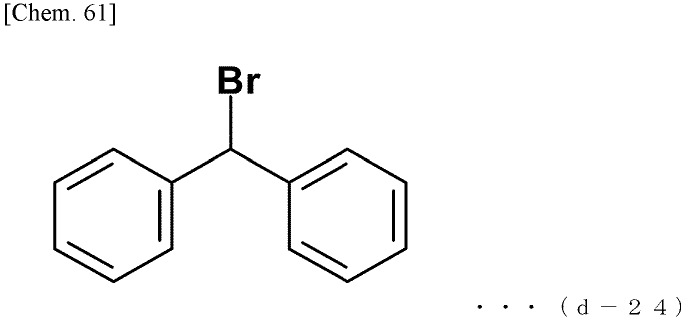

¹H-NMR (400 MHz, CDCl₃)δ(ppm)=7.44 to 7.20(m, 10H), 6.27(s, 1H).

### [Example 25]

An alkyl chloride compound represented by the following Formula (d-25) was synthesized using a primary alkylamine represented by Formula (b-22) and tetrabutylammonium chloride as a nucleophile. The yield was 19%.

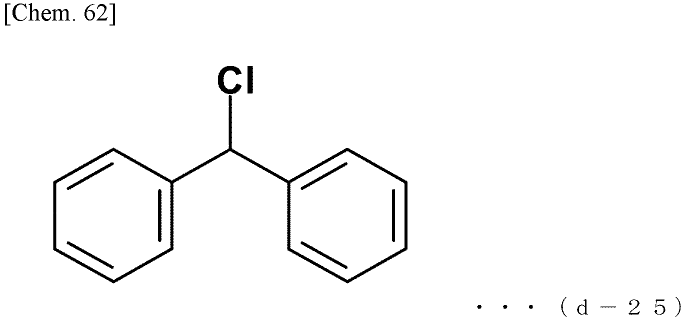

¹H-NMR (400 MHz, CDCl₃)δ(ppm)=7.36 to 7.23(m, 10H), 6.13(s, 1H).

### [Example 26]

An alkyl ether compound represented by the following Formula (d-26) was synthesized using a primary alkylamine represented by Formula (b-22) and 2, 2, 2-trifluoroethanol as a nucleophile. The yield was 65%.

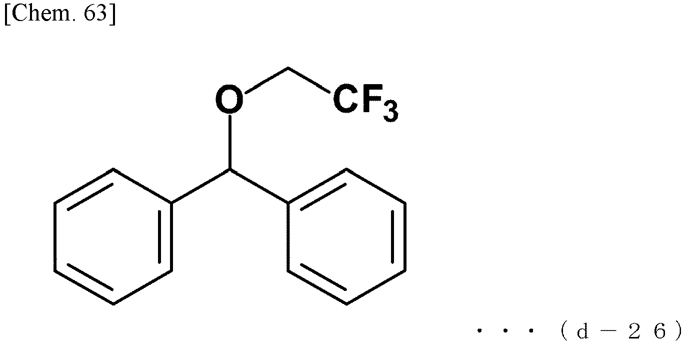

¹H-NMR (400 MHz, CDCl₃)δ(ppm)=7.47 to 7.20(m, 10H), 4.97(s, 1H), 3.14(q, 2H).
¹⁹F-NMR (376 MHz, CDCl₃)δ(ppm)=-71.21(t, 3F).

### [Example 27]

An alkyl ether compound represented by the following Formula (d-27) was synthesized using a primary alkylamine represented by Formula (b-22) and 2,2,3,3,3-pentafluoro-1-propanol as a nucleophile. The yield was 39%.

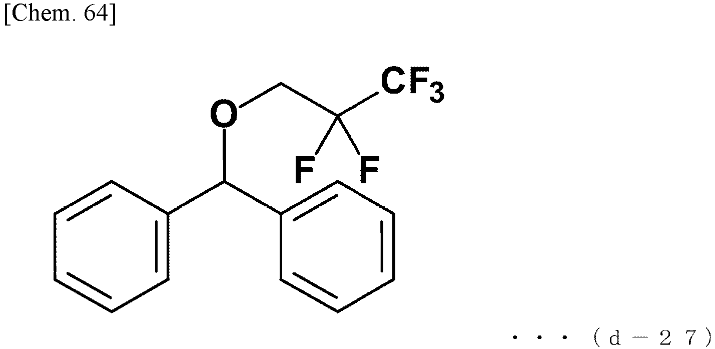

¹H-NMR (400 MHz, CDCl₃)δ(ppm)=7.45 to 7.19(m, 10H), 4.94(s, 1H), 3.22 to 3.14(m, 2H).
¹⁹F-NMR (376 MHz, CDCl₃)δ(ppm)=-83.84(m, 3F), -121.14 to -121.22(m, 2F).

### [Example 28]

An alkyl ether compound represented by the following Formula (d-28) was synthesized using a primary alkylamine represented by Formula (b-22) and 2,2,3,3-tetrafluoro-1-propanol as a nucleophile. The yield was 49%.

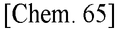

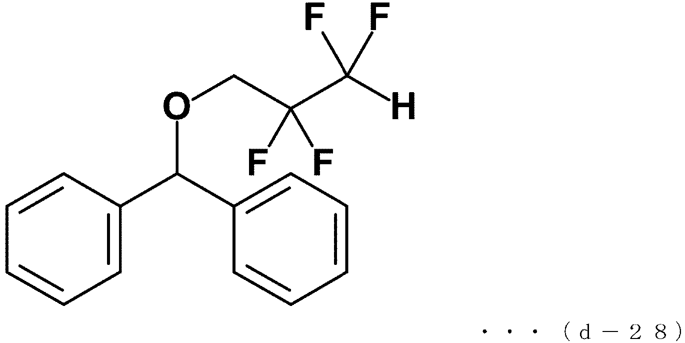

¹H-NMR (400 MHz, CDCl₃)δ(ppm)=7.37 to 7.21(m, 10H), 6.19 to 5.90(m, 1H), 4.88(s, 1H), 3.14 to 3.07(m, 2H).
¹⁹F-NMR (376 MHz, CDCl₃)δ(ppm)=-122.09 to -122.20(m, 2F), -138.71 to -138.87(m, 2F).

### [Example 29]

An alkyl ether compound represented by the following Formula (d-29) was synthesized using a primary alkylamine represented by Formula (b-22) and 1H,1H,2H,2H-tridecafluoro-1-n-octanol as a nucleophile. The yield was 15%.

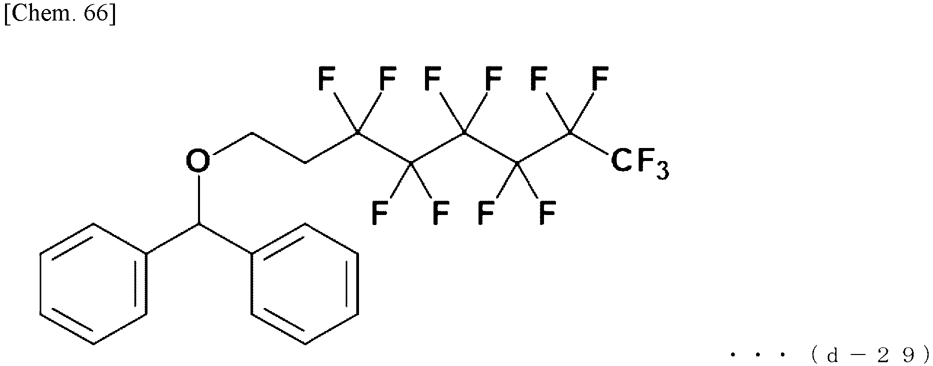

¹H-NMR (400 MHz, CDCl₃)δ(ppm)=7.40 to 7.19(m, 10H), 4.84(s, 1H), 2.93 to 2.89(m, 2H), 2.38 to 2.25(m, 2H).
¹⁹F-NMR (376 MHz, CDCl₃)δ(ppm)=-80.74 to -80.80(m, 3F), -113.46 to -113.55(m, 2F), - 121.88(m, 2F), -122.87(m, 2F), -123.60 to -123.63(m, 2F), -126.07 to -126.16(m, 2F).

### [Example 30]

An alkylazide compound represented by the following Formula (d-30) was synthesized using a primary alkylamine represented by Formula (b-22) and trimethylsilyl azide as a nucleophile. The yield was 85%.

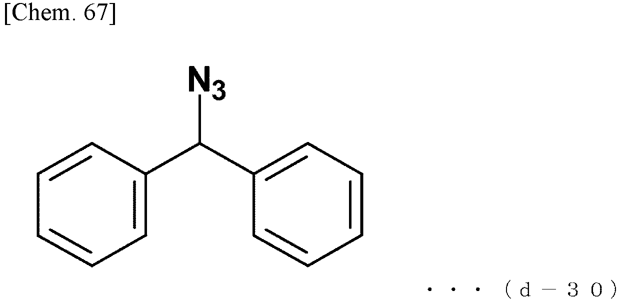

¹H-NMR (400 MHz, CDCl₃)δ(ppm)=7.41 to 7.31(m, 10H), 5.74(s, 1H).

### [Example 31]

The reaction was performed in the same manner as in Example 1 except that a primary alkylamine represented by the following Formula (b-31) was used in place of a primary alkylamine represented by Formula (b-1) and reacted with various nucleophiles to obtain a reaction product represented by the following Formula (d-31).

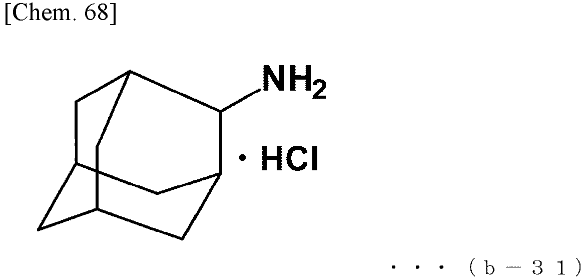

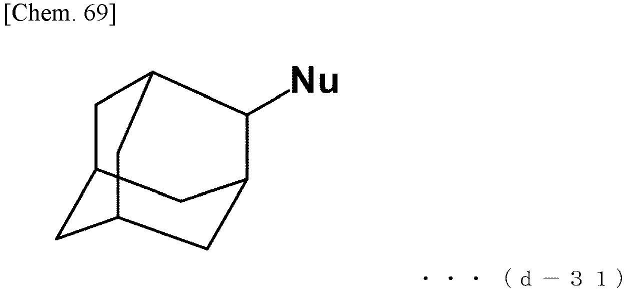

### [Example 32]

A fluorinated alkyl compound represented by the following Formula (d-32) was synthesized using a primary alkylamine represented by Formula (b-31) and tetrabutylammonium difluorotriphenylsilicate (TBAT) as a nucleophile. The yield was 12%.

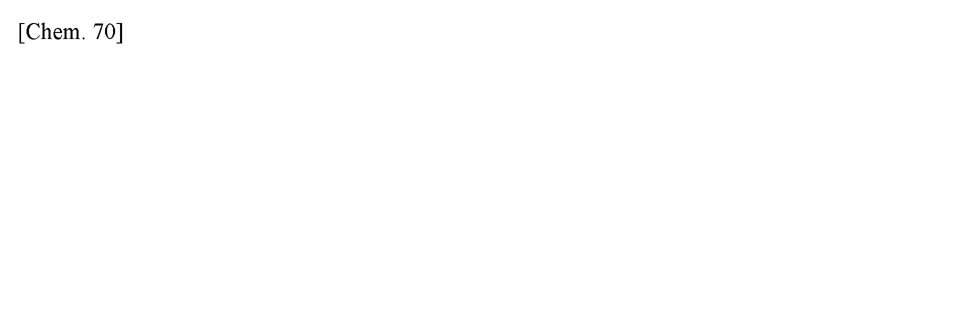

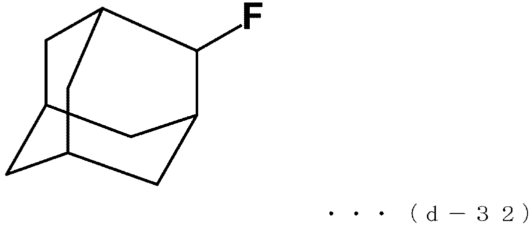

¹⁹F-NMR (376 MHz, CDCl₃)δ(ppm)=-173.81 to -173.97(m, 1F).

### [Example 33]

The reaction was performed in the same manner as in Example 1 except that a primary alkylamine represented by the following Formula (b-33) was used in place of a primary alkylamine represented by Formula (b-1) and reacted with various nucleophiles to obtain a reaction product represented by the following Formula (d-33).

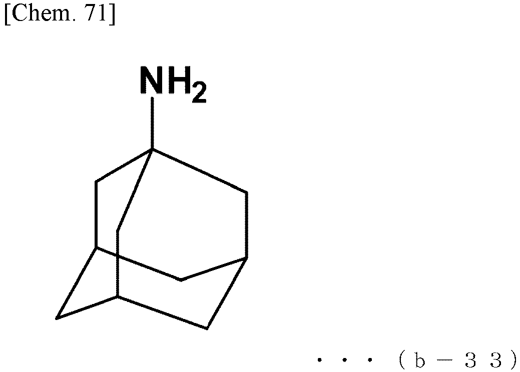

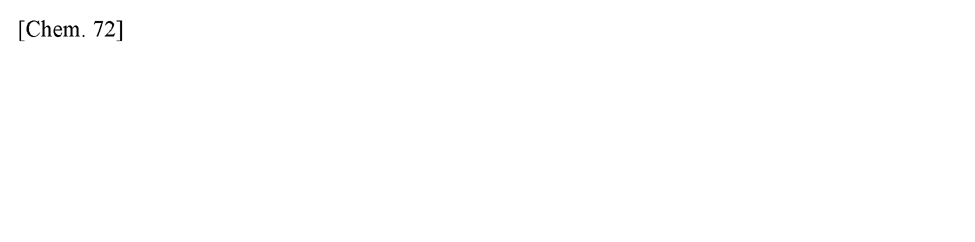

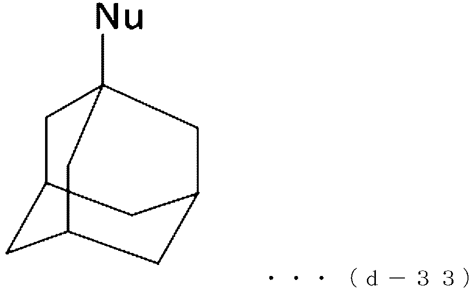

### [Example 34]

A fluorinated alkyl compound represented by the following Formula (d-34) was synthesized using a primary alkylamine represented by Formula (b-33) and tetrabutylammonium difluorotriphenylsilicate (TBAT) as a nucleophile. The yield was 20%.

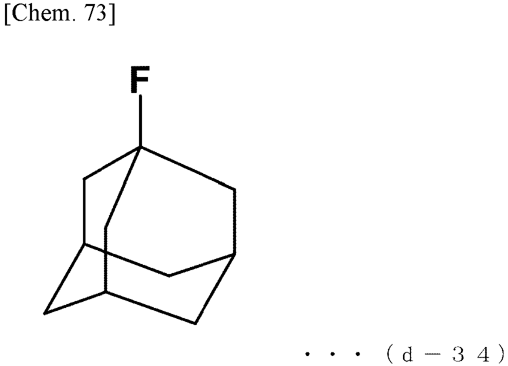

¹⁹F-NMR (376 MHz, CDCl₃)δ(ppm)=-128.50 to -128.52(m, 1F).

### [Example 35]

The reaction was performed in the same manner as in Example 1 except that a primary alkylamine represented by the following Formula (b-35) was used in place of a primary alkylamine represented by Formula (b-1) and reacted with various nucleophiles to obtain a reaction product represented by the following Formula (d-35).

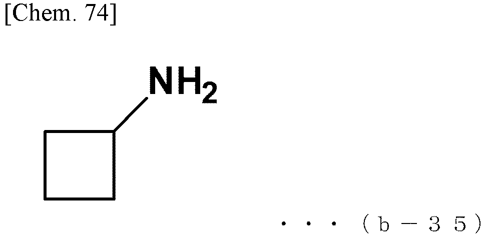

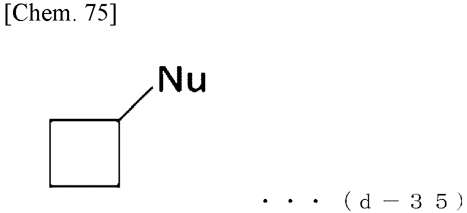

### [Example 36]

A fluorinated alkyl compound represented by the following Formula (d-36) was synthesized using a primary alkylamine represented by Formula (b-35) and tetrabutylammonium difluorotriphenylsilicate (TBAT) as a nucleophile. The yield was 12%.

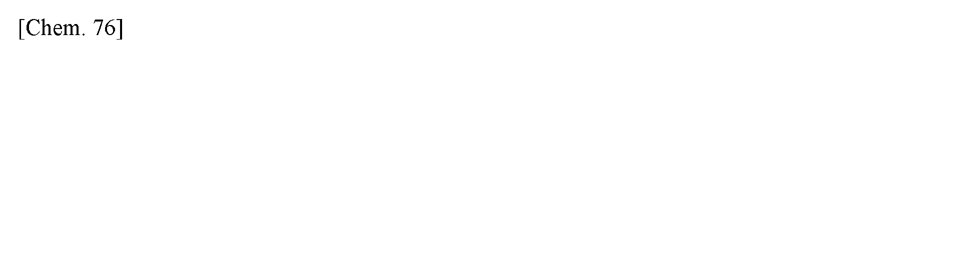

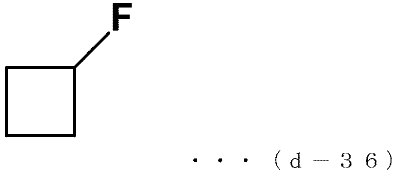

¹⁹F-NMR (376 MHz, CDCl₃)δ(ppm)=-159.39 to -159.71(m, 1F).

### [Example 37]

The reaction was performed in the same manner as in Example 1 except that a primary alkylamine represented by the following Formula (b-37) was used in place of a primary alkylamine represented by Formula (b-1) and reacted with various nucleophiles to obtain a reaction product represented by the following Formula (d-37).

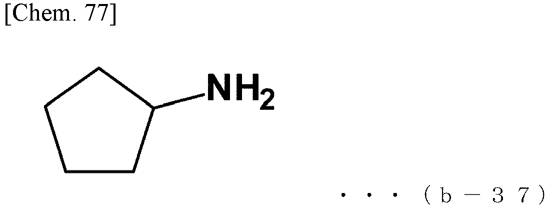

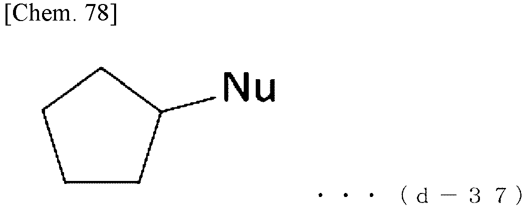

### [Example 38]

A fluorinated alkyl compound represented by the following Formula (d-38) was synthesized using a primary alkylamine represented by Formula (b-37) and tetrabutylammonium difluorotriphenylsilicate (TBAT) as a nucleophile. The yield was 41%.

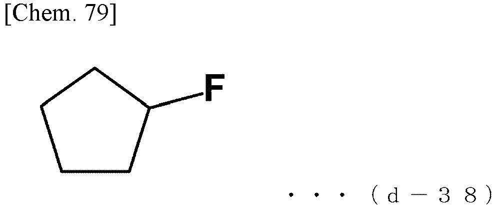

¹⁹F-NMR (376 MHz, CDCl₃)δ(ppm)=-169.42 to -169.88(m, 1F).

### [Example 39]

The reaction was performed in the same manner as in Example 1 except that a primary alkylamine represented by the following Formula (b-39) was used in place of a primary alkylamine represented by Formula (b-1) and reacted with various nucleophiles to obtain a reaction product represented by the following Formula (d-39).

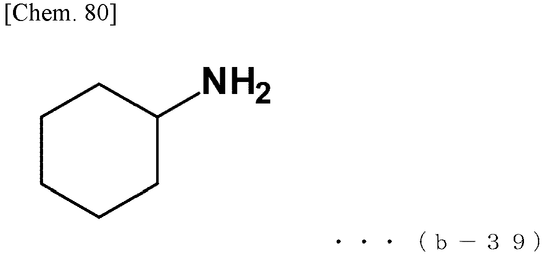

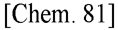

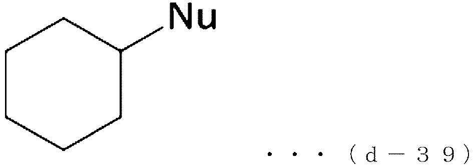

### [Example 40]

A fluorinated alkyl compound represented by the following Formula (d-40) was synthesized using a primary alkylamine represented by Formula (b-39) and tetrabutylammonium difluorotriphenylsilicate (TBAT) as a nucleophile. The yield was 2%.

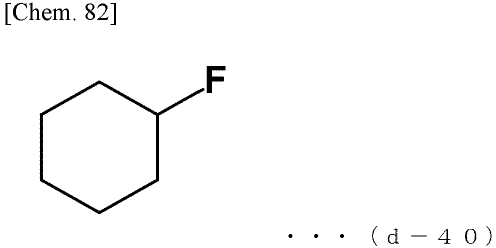

¹⁹F-NMR (376 MHz, CDCl₃)δ(ppm)=-171.97 to -172.30(m, 1F).

### [Example 41]

The reaction was performed in the same manner as in Example 1 except that a primary alkylamine represented by the following Formula (b-41) was used in place of a primary alkylamine represented by Formula (b-1) and reacted with various nucleophiles to obtain a reaction product represented by the following Formula (d-41).

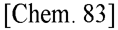

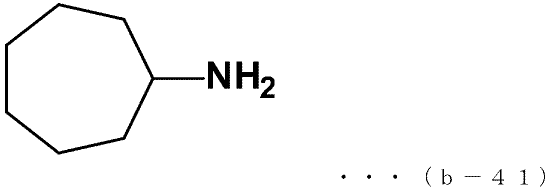

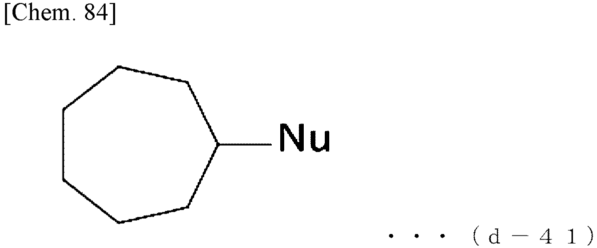

### [Example 42]

A fluorinated alkyl compound represented by the following Formula (d-42) was synthesized using a primary alkylamine represented by Formula (b-41) and tetrabutylammonium difluorotriphenylsilicate (TBAT) as a nucleophile. The yield was 51%.

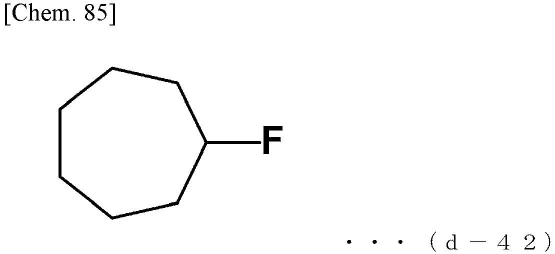

¹⁹F-NMR (376 MHz, CDCl₃)δ(ppm)=-163.25 to -163.57(m, 1F).

### [Example 43]

The reaction was performed in the same manner as in Example 1 except that a primary alkylamine represented by the following Formula (b-43) was used in place of a primary alkylamine represented by Formula (b-1) and reacted with various nucleophiles to obtain a reaction product represented by the following Formula (d-43).

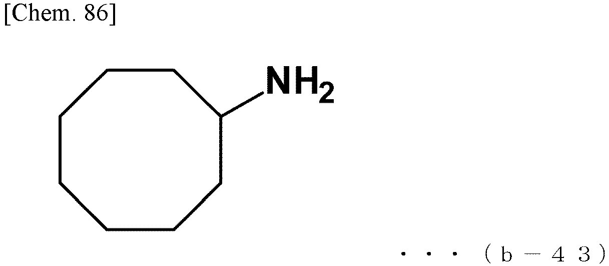

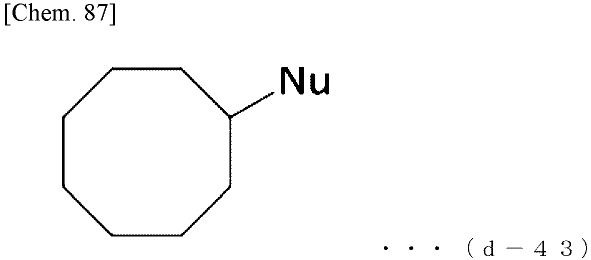

### [Example 44]

A fluorinated alkyl compound represented by the following Formula (d-44) was synthesized using a primary alkylamine represented by Formula (b-43) and tetrabutylammonium difluorotriphenylsilicate (TBAT) as a nucleophile. The yield was 21%.

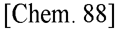

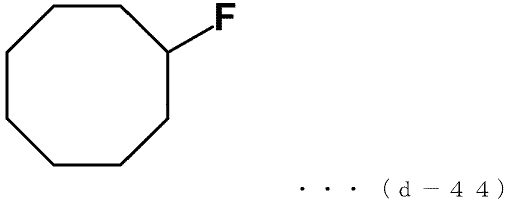

¹⁹F-NMR (376 MHz, CDCl₃)δ(ppm)=-158.27 to -158.91(m, 1F).

### [Example 45]

The reaction was performed in the same manner as in Example 1 except that a primary alkylamine represented by the following Formula (b-45) was used in place of a primary alkylamine represented by Formula (b-1) and reacted with various nucleophiles to obtain a reaction product represented by the following Formula (d-45).

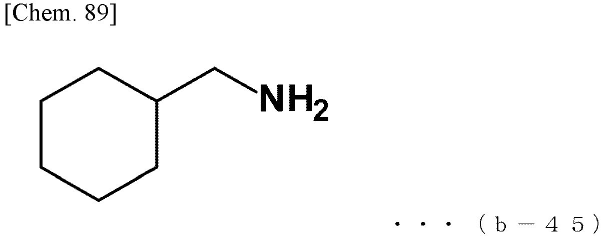

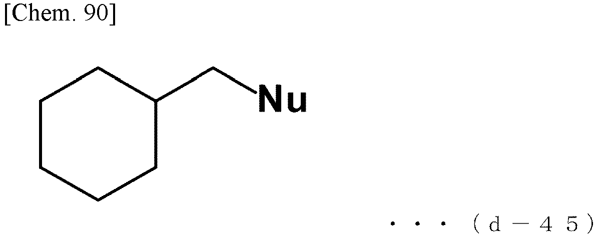

### [Example 46]

A fluorinated alkyl compound represented by the following Formula (d-46) was synthesized using a primary alkylamine represented by Formula (b-45) and tetrabutylammonium difluorotriphenylsilicate (TBAT) as a nucleophile. The yield was 23%.

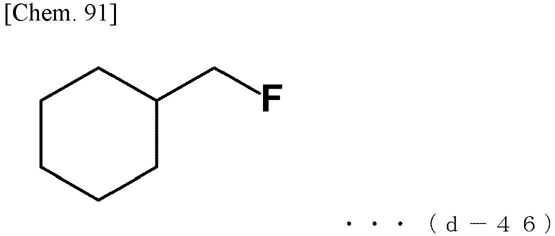

¹⁹F-NMR (376 MHz, CDCl₃)δ(ppm)=-221.92 to -222.22(m, 1F).

### [Example 47]

The reaction was performed in the same manner as in Example 1 except that a primary alkylamine represented by the following Formula (b-47) was used in place of a primary alkylamine represented by Formula (b-1) and reacted with various nucleophiles to obtain a reaction product represented by the following Formula (d-47).

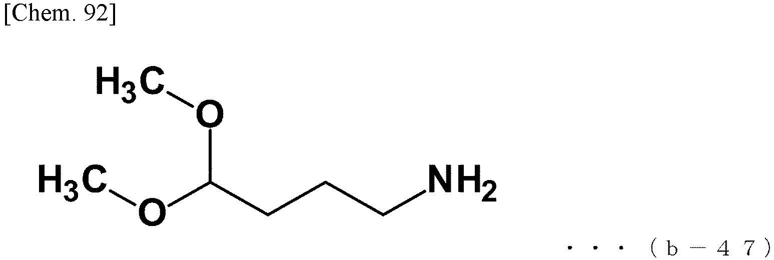

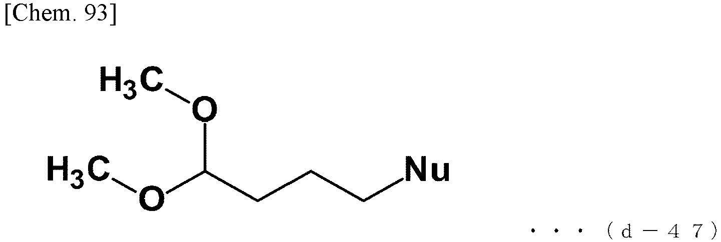

### [Example 48]

A fluorinated alkyl compound represented by the following Formula (d-48) was synthesized using a primary alkylamine represented by Formula (b-47) and tetrabutylammonium difluorotriphenylsilicate (TBAT) as a nucleophile. The yield was 32%.

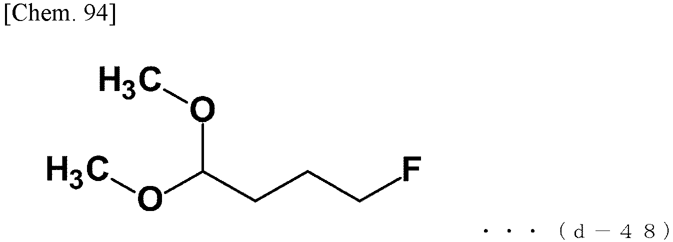

¹⁹F-NMR (376 MHz, CDCl₃)δ(ppm)=-218.18 to -218.57(m, 1F).

### [Example 49]

The reaction was performed in the same manner as in Example 1 except that a primary alkylamine represented by the following Formula (b-49) was used in place of a primary alkylamine represented by Formula (b-1) and reacted with various nucleophiles to obtain a reaction product represented by the following Formula (d-49).

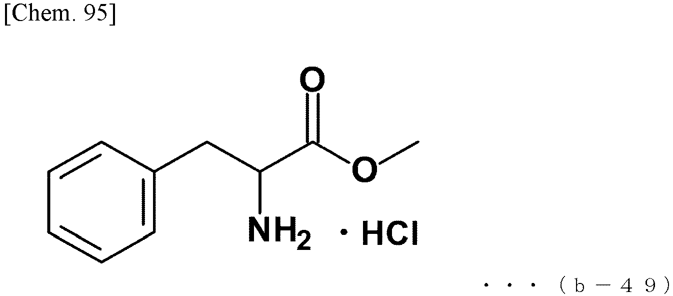

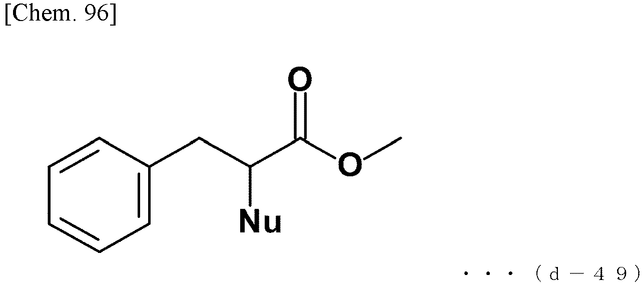

### [Example 50]

A fluorinated alkyl compound represented by the following Formula (d-50) was synthesized using a primary alkylamine represented by Formula (b-49) and tetrabutylammonium difluorotriphenylsilicate (TBAT) as a nucleophile. The yield was 3%.

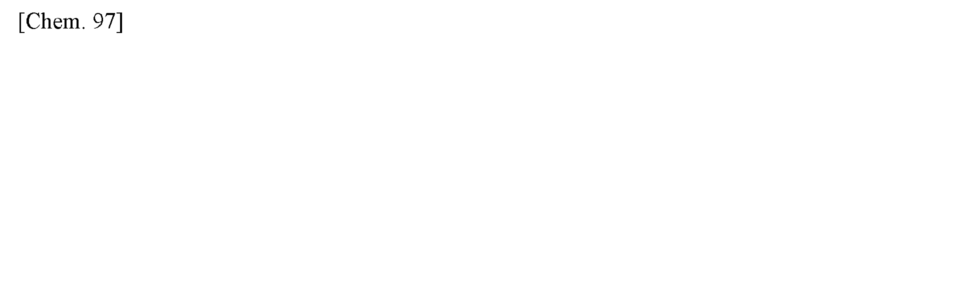

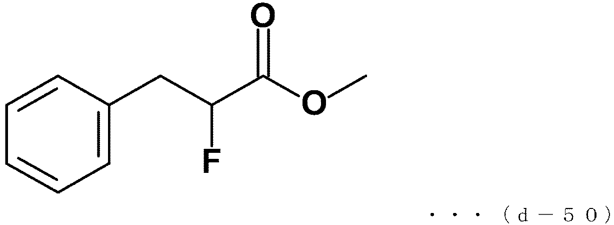

¹⁹F-NMR (376 MHz, CDCl₃)δ(ppm)=-189.97 to -190.24(m, 1F).

### [Example 51]

The reaction was performed in the same manner as in Example 1 except that a primary alkylamine represented by the following Formula (b-51) was used in place of a primary alkylamine represented by Formula (b-1) and reacted with various nucleophiles to obtain a reaction product represented by the following Formula (d-51).

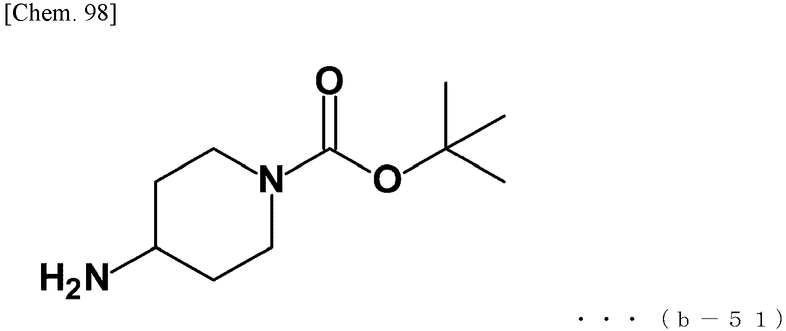

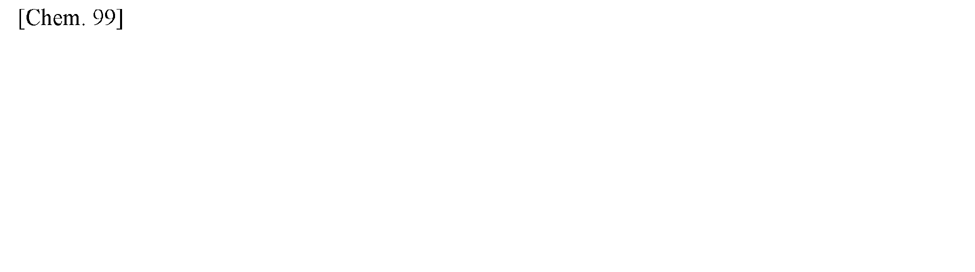

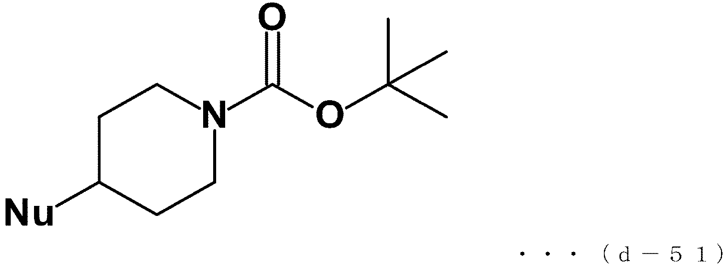

### [Example 52]

A fluorinated alkyl compound represented by the following Formula (d-52) was synthesized using a primary alkylamine represented by Formula (b-51) and tetrabutylammonium difluorotriphenylsilicate (TBAT) as a nucleophile. The yield was 33%.

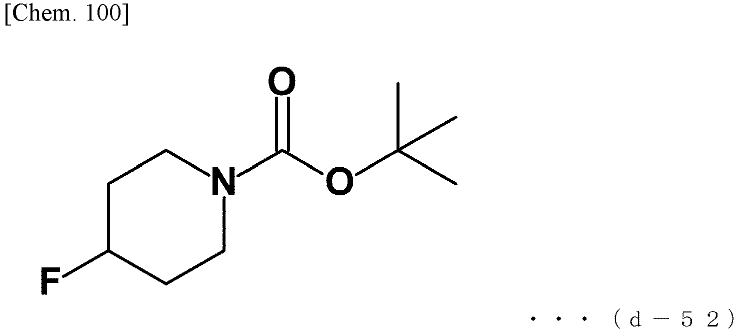

¹⁹F-NMR (376 MHz, CDCl₃)δ(ppm)=-181.99 to -182.33(m, 1F).

### [Example 53]

The reaction was performed in the same manner as in Example 1 except that a primary alkylamine represented by the following Formula (b-53) was used in place of a primary alkylamine represented by Formula (b-1) and reacted with various nucleophiles to obtain a reaction product represented by the following Formula (d-53).

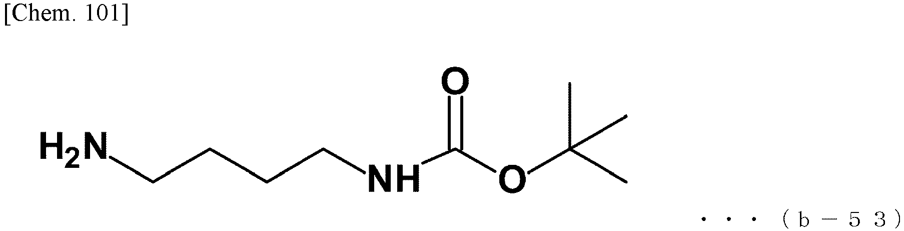

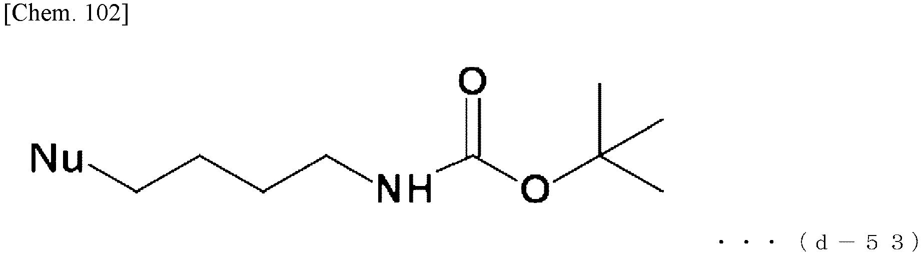

### [Example 54]

A fluorinated alkyl compound represented by the following Formula (d-54) was synthesized using a primary alkylamine represented by Formula (b-53) and tetrabutylammonium difluorotriphenylsilicate (TBAT) as a nucleophile. The yield was 16%.

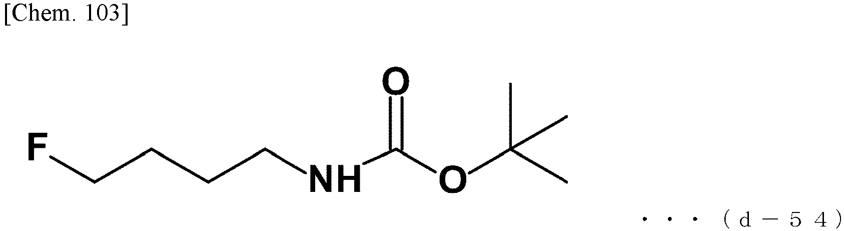

¹⁹F-NMR (376 MHz, CDCl₃)δ(ppm)=-218.16 to -218.55(m, 1F).

### [Example 55]

The reaction was performed in the same manner as in Example 1 except that a primary alkylamine represented by the following Formula (b-55) was used in place of a primary alkylamine represented by Formula (b-1) and reacted with various nucleophiles to obtain a reaction product represented by the following Formula (d-55).

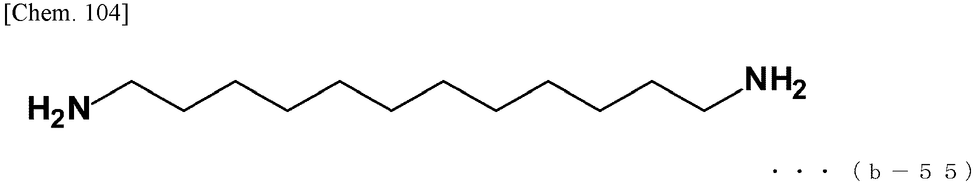

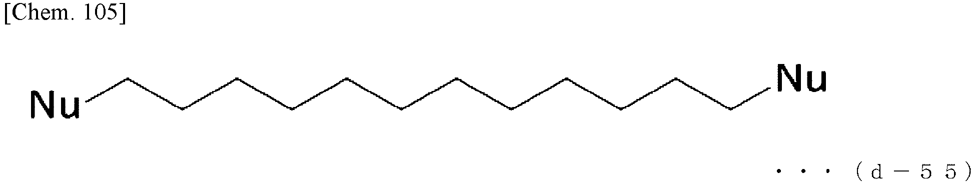

### [Example 56]

A fluorinated alkyl compound represented by the following Formula (d-56) was synthesized using a primary alkylamine represented by Formula (b-55) and tetrabutylammonium difluorotriphenylsilicate (TBAT) as a nucleophile. The yield was 21%.

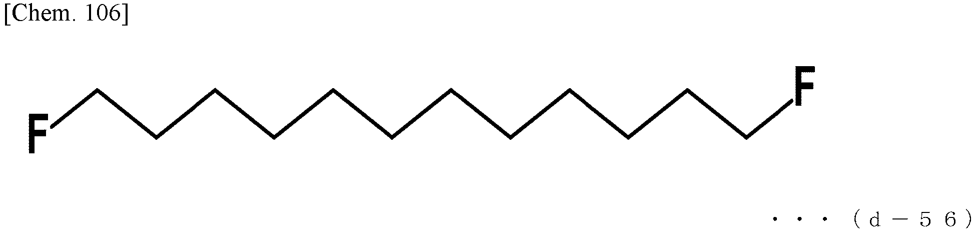

¹⁹F-NMR (376 MHz, CDCl₃)δ(ppm)=-217.88 to -218.27(m, 2F).

### [Example 57]

The reaction was performed in the same manner as in Example 1 except that a primary alkylamine represented by the following Formula (b-57) was used in place of a primary alkylamine represented by Formula (b-1) and reacted with various nucleophiles to obtain a reaction product represented by the following Formula (d-57).

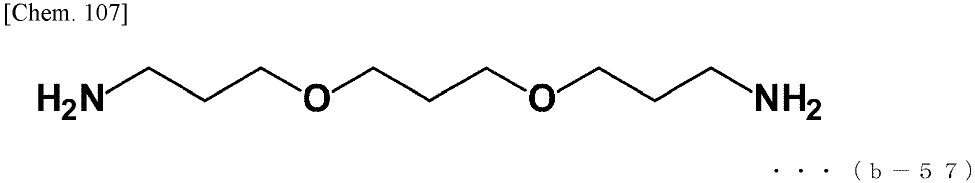

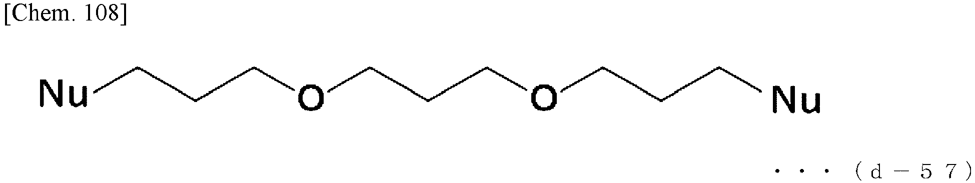

### [Example 58]

A fluorinated alkyl compound represented by the following Formula (d-58) was synthesized using a primary alkylamine represented by Formula (b-57) and tetrabutylammonium difluorotriphenylsilicate (TBAT) as a nucleophile. The yield was 36%.

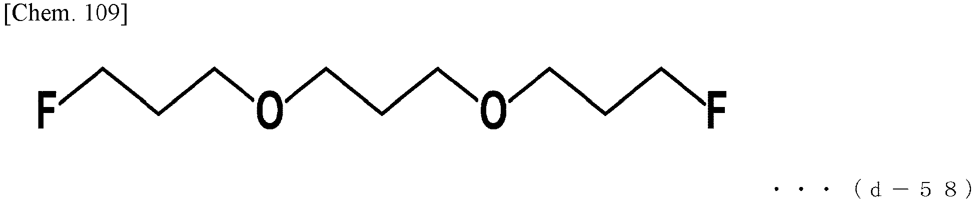

¹⁹F-NMR (376 MHz, CDCl₃)δ(ppm)=-221.68 to -222.07(m, 2F).

### [Example 59]

The reaction was performed in the same manner as in Example 1 except that a primary alkylamine represented by the following Formula (b*-59) was used in place of a primary alkylamine represented by Formula (b-1) and reacted with various nucleophiles to obtain a reaction product represented by the following Formula (d*-59).

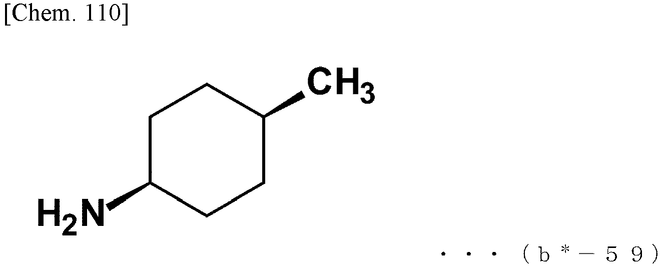

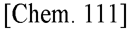

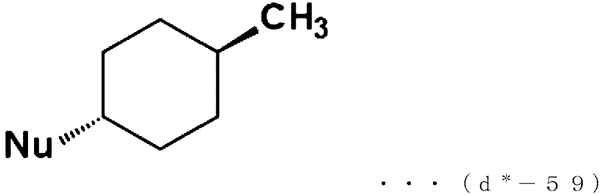

### [Example 60]

A fluorinated alkyl compound represented by the following Formula (d*-60) was synthesized using a primary alkylamine represented by Formula (b*-59) and tetrabutylammonium difluorotriphenylsilicate (TBAT) as a nucleophile. The yield was 7%.

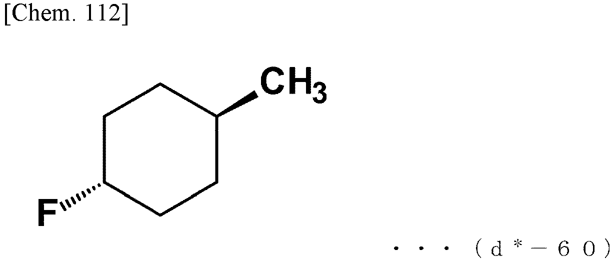

¹⁹F-NMR (376 MHz, CDCl₃)δ(ppm)=-169.48 to -169.80(m, 1F).

### [Example 61]

The reaction was performed in the same manner as in Example 1 except that a primary alkylamine represented by the following Formula (b*-61) was used in place of a primary alkylamine represented by Formula (b-1) and reacted with various nucleophiles to obtain a reaction product represented by the following Formula (d*-61).

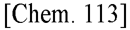

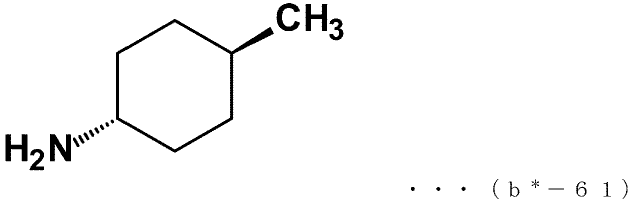

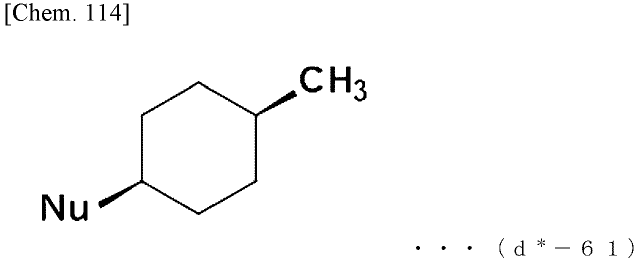

### [Example 62]

A fluorinated alkyl compound represented by the following Formula (d*-62) was synthesized using a primary alkylamine represented by Formula (b*-61) and tetrabutylammonium difluorotriphenylsilicate (TBAT) as a nucleophile. The yield was 20%.

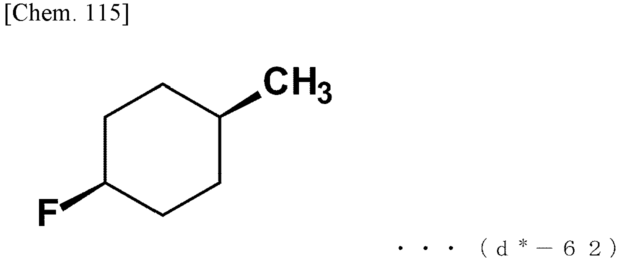

¹⁹F-NMR (376 MHz, CDCl₃)δ(ppm)=-183.14 to -183.74(m, 1F).

### [Example 63]

The reaction was performed in the same manner as in Example 1 except that 1,1,2,2-tetrafluoroethane-1,2-disulfonyl fluoride represented by the following Formula (a-63) was used in place of 1,1,2,2,3,3-hexafluoropropane-1,3-disulfonyl fluoride.

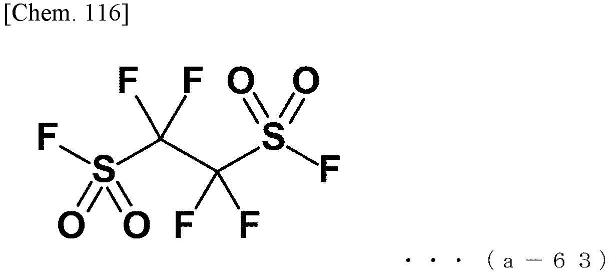

### [Example 64]

A fluorinated alkyl compound represented by the above (d-4) was synthesized using a primary alkylamine represented by Formula (b-1), tetrabutylammonium difluorotriphenylsilicate (TBAT) as a nucleophile, and 1,1,2,2-tetrafluoroethane-1,2-disulfonyl fluoride in place of 1,1,2,2,3,3-hexafluoropropane-1,3-disulfonyl fluoride. The yield was 31%.

### [Example 65]

The reaction was performed in the same manner as in Example 1 except that 2,2,3,3,4,4-hexafluoro-4-(fluorosulfonyl)butanoyl fluoride represented by the following Formula (a-111) was used in place of 1,1,2,2,3,3-hexafluoropropane-1,3-disulfonyl fluoride.

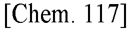

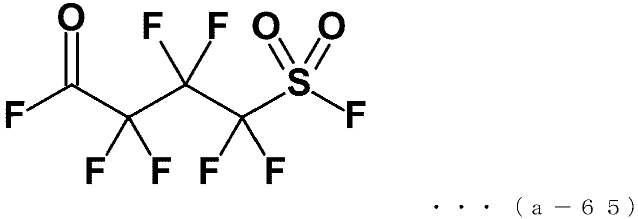

### [Example 66]

A fluorinated alkyl compound represented by the above (d-4) was synthesized using primary alkylamine represented by Formula (b-1), tetrabutylammonium difluorotriphenylsilicate (TBAT) as a nucleophile, and 2,2,3,3,4,4-hexafluoro-4-(fluorosulfonyl)butanoyl fluoride in place of 1,1,2,2,3,3-hexafluoropropane-1,3-disulfonyl fluoride. The yield was 3%.

### [Example 67]

A reaction precursor represented by the following Formula (e-67) obtained by reacting a primary alkylamine represented by Formula (b-1) with 1,1,2,2,3,3-hexafluoropropane-1,3-disulfonyl fluoride as an amino group eliminating agent was isolated and then reacted with TBAT to synthesize a fluorinated alkyl compound represented by Formula (d-4).

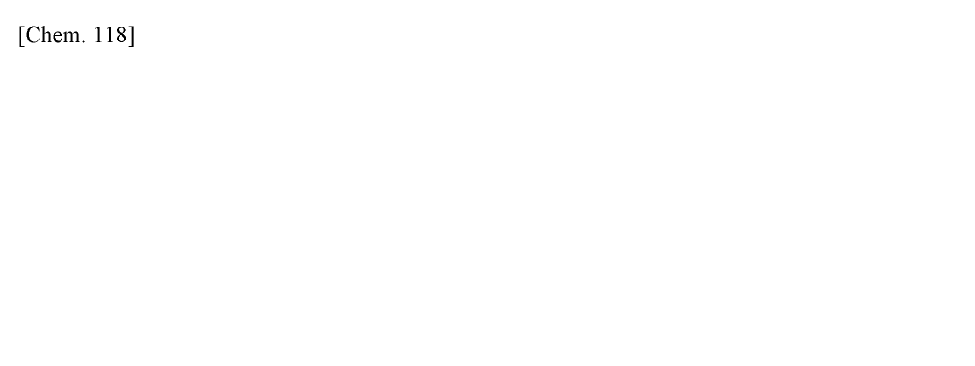

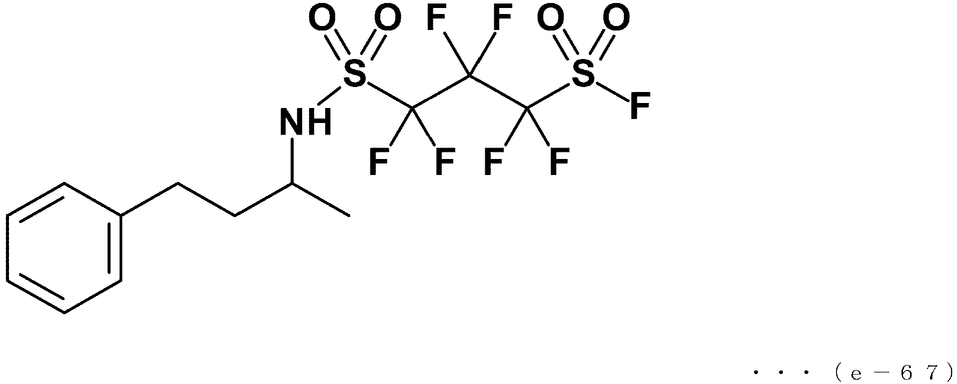

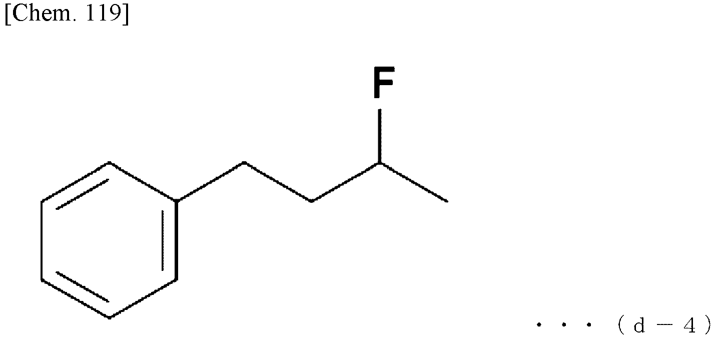

Under a nitrogen atmosphere, 3-amino-1-phenylbutane (149.2 mg, 1.0 mmol) and 1,1,2,2,3,3-hexafluoropropane-1,3-disulfonyl fluoride (379.4 mg, 1.2 mmol) were added to diisopropyl ether (20 mL). Triethylamine (151.8 mg, 1.5 mmol) was slowly added thereto, and the mixture was stirred at room temperature for27 hours and then quenched with a 2 M hydrochloric acid aqueous solution (20 mL). Chloroform (20 mL) was added to the obtained reaction solution, liquid separation was performed, the organic layer was then washed once with a 2 M hydrochloric acid aqueous solution (10 mL), and the obtained organic layer was washed twice with a saturated sodium carbonate aqueous solution (20 mL). The washed organic layer was dried over magnesium sulfate, and the solvent was then distilled off under a reduced pressure. The obtained crude product was purified through silica gel column chromatography (hexane to hexane/ethyl acetate=20/1), and the solvent was distilled off under a reduced pressure to obtain a compound represented by Formula (e-67) in a yield of 36%.

By NMR analysis, it was confirmed that a compound represented by Formula (e-67) was obtained.
¹H-NMR (400 MHz, CDCl₃)δ(ppm)=7.32 to 7.26(m, 2H), 7.23 to 7.17(m, 3H), 5.02 to 4.99(m, 1H), 3.79 to 3.74(m, 1H), 2.75 to 2.68(m, 2H), 1.92 to 1.86(m, 2H), 1.34(d, 3H). ¹⁹F-NMR (376 MHz, CDCl₃)δ(ppm)=-46.76 to -46.69(m, 1F), -107.22 to -107.35(m, 2F), - 112.40 to -112.63(m, 2F), -118.38 to -118.49(m, 2F).

Subsequently, under a nitrogen atmosphere, a compound represented by Formula (e-67) (160.3 mg, 0.36 mmol) and TBAT (97.2 mg, 0.18 mmol) were added to toluene (7.2 mL). DBU (54.8 mg, 0.18 mmol) was slowly added thereto, and the mixture was stirred at 110°C for 1 hour and then quenched with a 2 M hydrochloric acid aqueous solution (10 mL). Chloroform (10 mL) was added to the obtained reaction solution, liquid separation was performed, the organic layer was then washed once with a 2 M hydrochloric acid aqueous solution (10 mL), and the obtained organic layer was washed twice with a saturated sodium carbonate aqueous solution (10 mL). The washed organic layer was dried over magnesium sulfate, and the solvent was then distilled off under a reduced pressure. The obtained crude product was purified through silica gel column chromatography and the solvent was distilled off under a reduced pressure to obtain a compound represented by Formula (d-4) in a yield of 68%.

By NMR analysis, it was confirmed that a compound represented by Formula (d-4) was obtained.
¹H-NMR (400 MHz, CDCl₃)δ(ppm)=7.31 to 7.18(m, 5H), 4.75 to 4.56(m, 1H), 2.84 to 2.65(m, 2H), 2.05 to 1.70(m, 2H), 1.55 to 1.37(m, 3H).
¹⁹F-NMR (376 MHz, CDCl₃)δ(ppm)=-174.04 to -174.36(m, 1F).

### [Example 68]

A reaction precursor represented by the following Formula (f-68) was synthesized by reacting a primary alkylamine represented by the following Formula (b-68) with 1,1,2,2,3,3-hexafluoropropane-1,3-disulfonyl fluoride as an amino group eliminating agent.

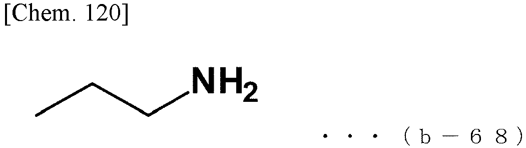

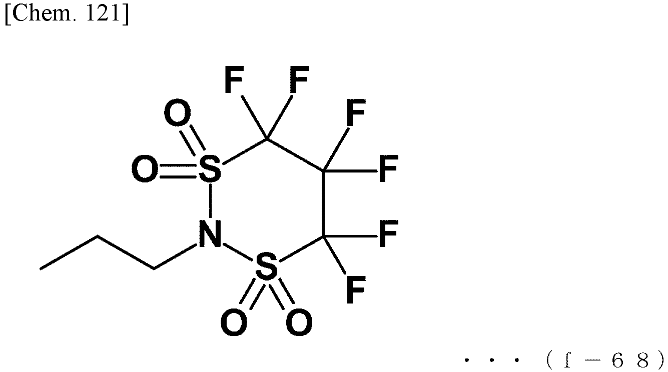

Under a nitrogen atmosphere, 1,1,2,2,3,3-hexafluoropropane-1,3-disulfonyl fluoride (10.0 g, 31.6 mmol) was added to acetonitrile (38 mL) and cooled to 0°C. A mixed solution of propylamine (5.61 g, 94.9 mmol) and acetonitrile (13 mL) was slowly added thereto, the mixture was stirred at room temperature for 1 hour and the reaction solution was then filtered.

Water (100 mL) and chloroform (50 mL) were added to the obtained reaction solution, liquid separation was performed, the organic layer was then washed twice with water (50 mL), and the obtained organic layer was washed once with a saturated sodium carbonate aqueous solution (50 mL). The washed organic layer was dried over magnesium sulfate, and the solvent was then distilled off under a reduced pressure. The obtained crude product was distilled off under a reduced pressure to obtain a compound represented by Formula (f-68) in a yield of 85%.

By NMR analysis, it was confirmed that a reaction precursor represented by Formula (f-68) was obtained.
¹H-NMR (400 MHz, CDCl₃)δ(ppm)=3.99(t, 2H), 1.87(qt, 2H), 1.03(t, 3H).

### [Example 69]

A reaction precursor represented by Formula (f-68) obtained by reacting a primary alkylamine represented by Formula (b-68) with 1,1,2,2,3,3-hexafluoropropane-1,3-disulfonyl fluoride as an amino group eliminating agent was isolated and then reacted with various nucleophiles to obtain a reaction product represented by the following Formula (d-69).

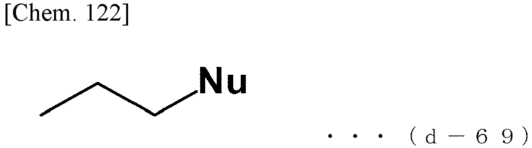

### [Example 70]

Using a reaction precursor obtained from a primary alkylamine represented by Formula (b-68) and an amino group eliminating agent, an alkyl iodide compound represented by the following Formula (d-70) was synthesized using tetrabutylammonium iodide as a nucleophile. The yield was 80%.

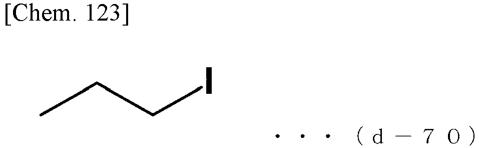

¹H-NMR (400 MHz, CDCl₃)δ(ppm)=3.16(t, 2H), 1.82(qt, 2H), 0.98(t, 3H).

### [Example 71]

Using a reaction precursor obtained from a primary alkylamine represented by Formula (b-68) and an amino group eliminating agent, an ether compound represented by the following Formula (d-71) was synthesized using phenol and sodium hydride as nucleophiles. The yield was 91%.

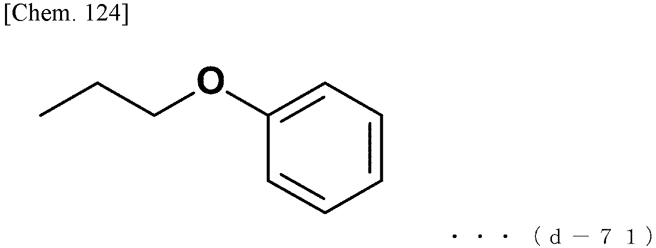

¹H-NMR (400 MHz, CDCl₃)δ(ppm)=7.47 to 7.43(m, 2H), 7.40 to 7.36(m, 1H), 7.31 to 7.26(m, 2H), 3.32(t, 2H), 1.64(qt, 2H), 0.98(t, 3H).

### [Example 72]

Using a reaction precursor obtained from a primary alkylamine represented by Formula (b-68) and an amino group eliminating agent, a sulfide compound represented by the following Formula (d-72) was synthesized using 4-methylbenzenethiol and sodium hydride as nucleophiles. The yield was 20%.

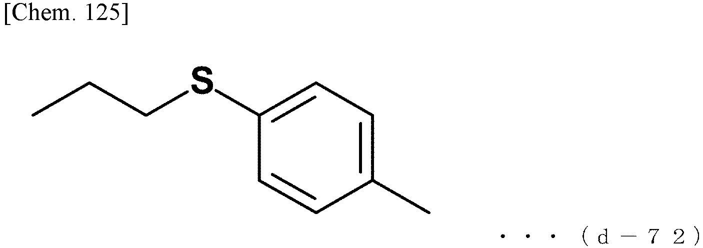

¹H-NMR (400 MHz, CDCl₃)δ(ppm)=7.25(d, 2H), 7.09(d, 2H), 2.85(t, 2H), 2.32(s, 3H), 1.69 to 1.60(m, 2H), 1.00(t, 3H).

### [Example 73]

Using a reaction precursor obtained from a primary alkylamine represented by Formula (b-68) and an amino group eliminating agent, an amine compound represented by the following Formula (d-73) was synthesized using aniline as a nucleophile. The yield was 22%.

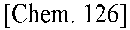

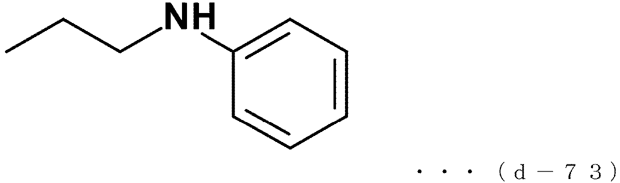

¹H-NMR (400 MHz, CDCl₃)δ(ppm)=7.35 to 7.31(m, 2H), 7.08 to 7.06(m, 1H), 6.99 to 6.96(m, 2H), 3.51(s, 1H), 3.24 to 3.21(m, 2H), 1.70 to 1.63(m, 2H), 0.98(t, 3H).

### [Example 74]

The reaction was performed in the same manner as in Example 70 except that, using a reaction precursor obtained from a primary alkylamine represented by Formula (b-68) and an amino group eliminating agent and using diethyl malonate and sodium hydride as nucleophiles, an alkyl compound represented by the following Formula (d-74) was synthesized. The yield was 92%.

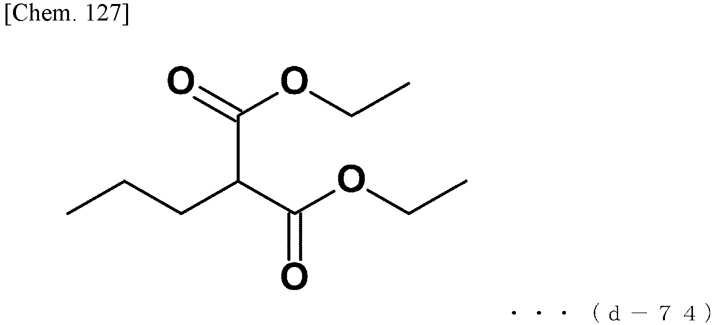

¹H-NMR (400 MHz, CDCl₃)δ(ppm)=4.19(q, 4H), 3.33(t, 1H), 1.91 to 1.85(m, 2H), 1.39 to 1.33(m, 2H), 1.27(t, 6H), 0.94(t, 3H).

### [Example 75]

The reaction was performed in the same manner as in Example 70 except that, using a reaction precursor obtained from a primary alkylamine represented by Formula (b-68) and an amino group eliminating agent and using potassium trifluoromethanesulfonamide and potassium fluoride as nucleophiles and acetonitrile as a solvent in place of toluene, an alkyl compound represented by the following Formula (d-75) was synthesized. The yield was 81%.

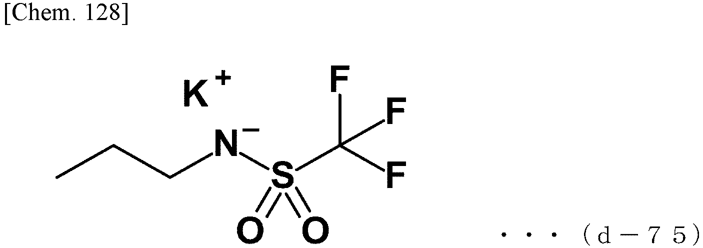

¹H-NMR (400 MHz, CDCl₃)δ(ppm)=3.02 to 2.98(m, 2H), 1.54 to 1.45(m, 2H), 0.90(t, 3H).
¹⁹F-NMR (376 MHz, CDCl₃)δ(ppm)=-74.36(s, 3F).

### [Example 76]

Using a reaction precursor obtained from a primary alkylamine represented by Formula (b-68) and an amino group eliminating agent, a quaternary ammonium compound represented by the following Formula (d-76) was synthesized using trimethylamine as a nucleophile. The yield was 97%.

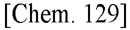

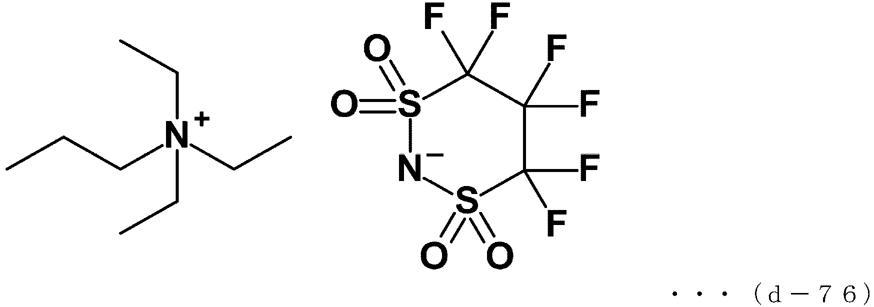

¹H-NMR (400 MHz, CDCl₃)δ(ppm)=3.31 to 3.26(m, 6H), 3.09 to 3.05(m, 2H), 1.74 to 1.68(m, 2H), 1.38 to 1.31(m, 9H), 1.05(t, 3H).
¹⁹F-NMR (376 MHz, CDCl₃)δ(ppm)=-119.20 to -119.65(m, 4F), -125.87 to -126.42(m, 2F).

### [Example 77]

Using a reaction precursor obtained from a primary alkylamine represented by Formula (b-68) and an amino group eliminating agent, an imidazolium salt represented by the following Formula (d-77) was synthesized using 1-methylimidazole as a nucleophile. The yield was 98%.

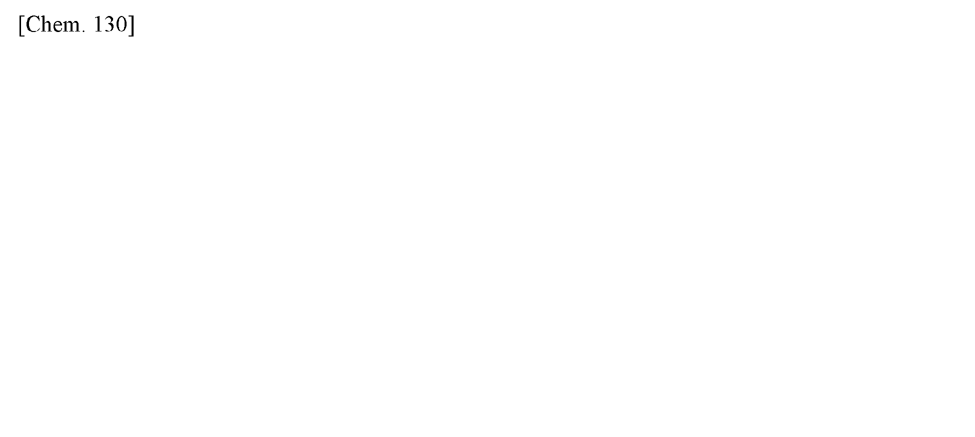

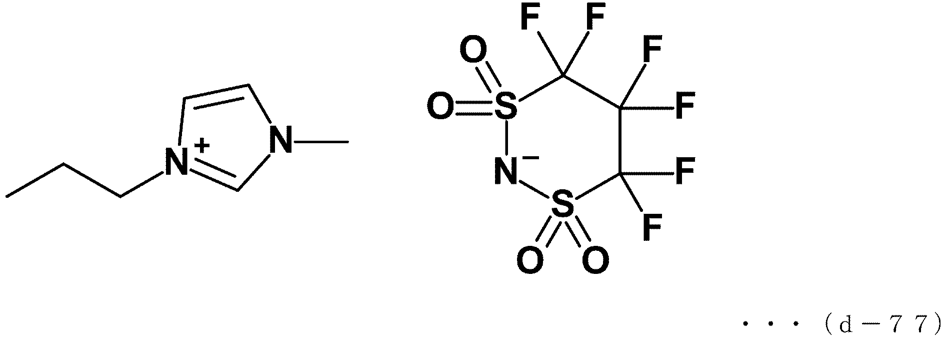

¹H-NMR (400 MHz, CDCl₃)δ(ppm)=8.73(s, 1H), 7.29 to 7.27(m, 2H), 4.15(t, 2H), 3.95(s, 3H), 1.91(qt, 2H), 0.98(t, 3H).
¹⁹F-NMR (376 MHz, CDCl₃)δ(ppm)=-119.26 to -119.66(m, 4F), -125.88 to -126.30(m, 2F).

### [Example 78]

Using a reaction precursor obtained from a primary alkylamine represented by Formula (b-68) and an amino group eliminating agent, a phosphonium salt represented by the following Formula (d-78) was synthesized using triphenylphosphine as a nucleophile. The yield was 52%.

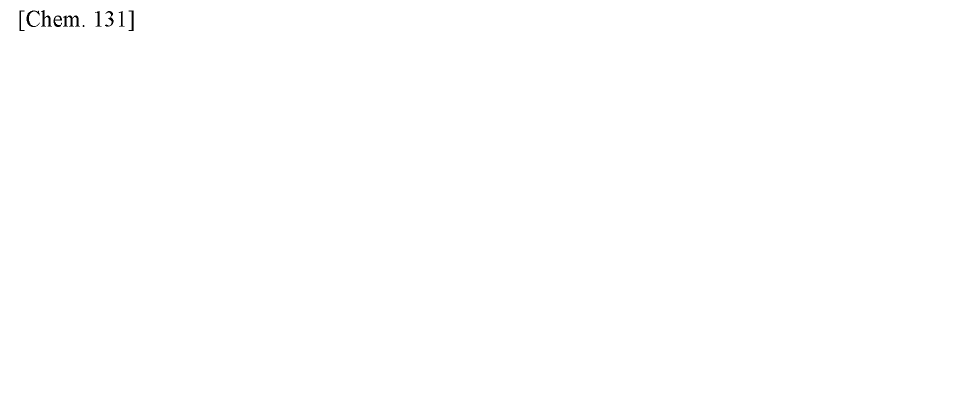

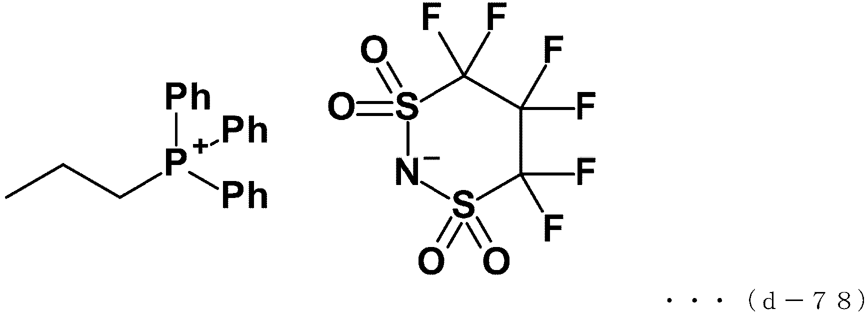

¹H-NMR (400 MHz, CDCl₃)δ(ppm)=7.84 to 7.80(m, 3H), 7.74 to 7.63(m, 12H), 3.19 to 3.12(m, 2H), 1.75 to 1.64(m, 2H), 1.20 to 1.16(m, 3H).
¹⁹F-NMR (376 MHz, CDCl₃)δ(ppm)=-119.27 to -119.50(m, 4F), -125.83 to -126.47(m, 2F).

### [Comparative Example 1]

The reaction was performed in the same manner as in Example 1 except that 1,1,2,2,3,3-hexafluoropropane-1,3-disulfonyl fluoride was not used.

### [Comparative Example 2]

The reaction was performed using a primary alkylamine represented by Formula (b-1), and tetrabutylammonium difluorotriphenylsilicate (TBAT) as a nucleophile without adding 1,1,2,2,3,3-hexafluoropropane-1,3-disulfonyl fluoride. A fluorinated alkyl compound represented by the above (d-4) was not produced.

### [Comparative Example 3]

The reaction was performed in the same manner as in Example 1 except that p-toluenesulfonyl chloride represented by the following Formula (a'-3) was used in place of 1,1,2,2,3,3-hexafluoropropane-1,3-disulfonyl fluoride.

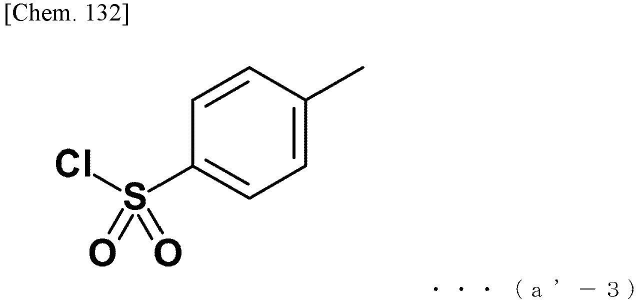

### [Comparative Example 4]

The reaction was performed using a primary alkylamine represented by Formula (b-1), tetrabutylammonium iodide as a nucleophile, and p-toluenesulfonyl chloride represented by Formula (a'-3) in place of 1,1,2,2,3,3-hexafluoropropane-1,3-disulfonyl fluoride. An alkyl iodide compound represented by the above (d-1) was not produced.

### [Comparative Example 5]

The reaction was performed in the same manner as in Example 1 except that nonafluoro-1-butanesulfonyl fluoride represented by the following Formula (a'-5) was used in place of 1,1,2,2,3,3-hexafluoropropane-1,3-disulfonyl fluoride.

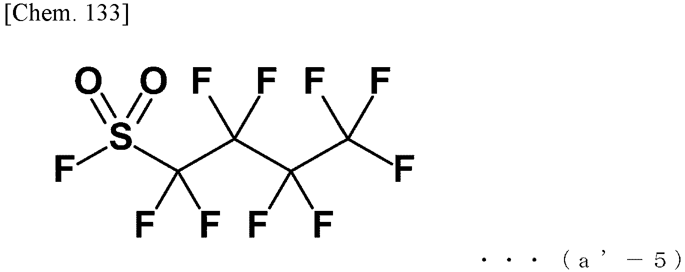

### [Comparative Example 6]

The reaction was performed using a primary alkylamine represented by Formula (b-1), tetrabutylammonium difluorotriphenylsilicate (TBAT) as a nucleophile, and nonafluoro-1-butanesulfonyl fluoride represented by Formula (a'-5) in place of 1,1,2,2,3,3-hexafluoropropane-1,3-disulfonyl fluoride. A fluorinated alkyl compound represented by the above (d-4) was not produced.

### [Comparative Example 7]

The reaction was performed in the same manner as in Example 1 except that trifluoromethanesulfonic anhydride represented by the following Formula (a'-7) was used in place of 1,1,2,2,3,3-hexafluoropropane-1,3-disulfonyl fluoride.

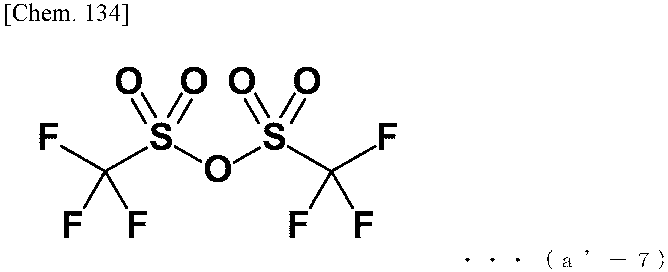

### [Comparative Example 8]

The reaction was performed using a primary alkylamine represented by Formula (b-1), tetrabutylammonium difluorotriphenylsilicate (TBAT) as a nucleophile, and trifluoromethanesulfonic anhydride represented by Formula (a'-7) in place of 1,1,2,2,3,3-hexafluoropropane-1,3-disulfonyl fluoride. A fluorinated alkyl compound represented by the above (d-4) was not produced.

### [Comparative Example 9]

The reaction was performed in the same manner as in Example 1 except that propane-1,3-disulfonyl fluoride represented by the following Formula (a'-9) was used in place of 1,1,2,2,3,3-hexafluoropropane-1,3-disulfonyl fluoride.

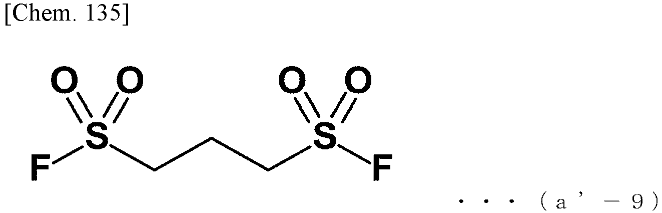

### [Comparative Example 10]

The reaction was performed using a primary alkylamine represented by Formula (b-1), tetrabutylammonium difluorotriphenylsilicate (TBAT) as a nucleophile, and propane-1,3-disulfonyl fluoride represented by Formula (a'-9) in place of 1,1,2,2,3,3-hexafluoropropane-1,3-disulfonyl fluoride. A fluorinated alkyl compound represented by the above (d-4) was not produced.

### [Comparative Example 11]

The reaction was performed in the same manner as in Example 1 except that propane-1,3-disulfonyl chloride represented by the following Formula (a'-10) was used in place of 1,1,2,2,3,3-hexafluoropropane-1,3-disulfonyl fluoride.

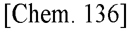

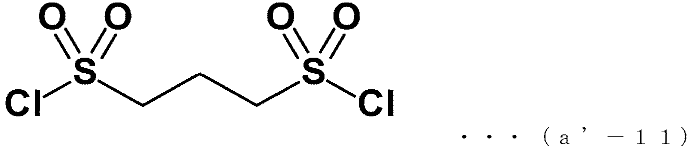

### [Comparative Example 12]

The reaction was performed using a primary alkylamine represented by Formula (b-1), tetrabutylammonium difluorotriphenylsilicate (TBAT) as a nucleophile, and propane-1,3-disulfonyl chloride represented by Formula (a'-11) in place of 1,1,2,2,3,3-hexafluoropropane-1,3-disulfonyl fluoride. A fluorinated alkyl compound represented by the above (d-4) was not produced.

### [Comparative Example 13]

The reaction was performed in the same manner as in Example 1 except that 1,2-benzenedisulfonyl fluoride represented by the following Formula (a'-13) was used in place of 1,1,2,2,3,3-hexafluoropropane-1,3-disulfonyl fluoride.

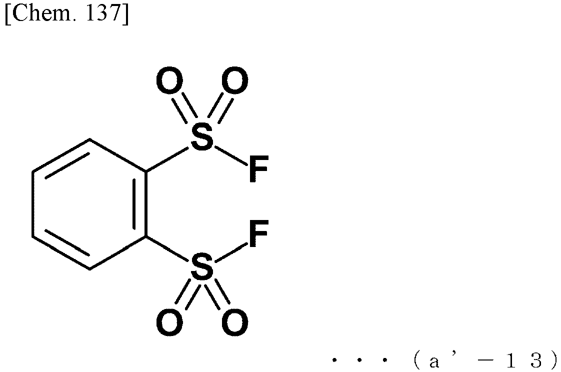

### [Comparative Example 14]

The reaction was performed using a primary alkylamine represented by Formula (b-1), tetrabutylammonium difluorotriphenylsilicate (TBAT) as a nucleophile, and 1,2-benzenedisulfonyl fluoride represented by Formula (a'-13) in place of 1,1,2,2,3,3-hexafluoropropane-1,3-disulfonyl fluoride. A fluorinated alkyl compound represented by the above (d-4) was not produced.

### [Comparative Example 15]

The reaction was performed in the same manner as in Example 1 except that 1,2-benzenedisulfonyl chloride represented by the following Formula (a'-15) was used in place of 1,1,2,2,3,3-hexafluoropropane-1,3-disulfonyl fluoride.

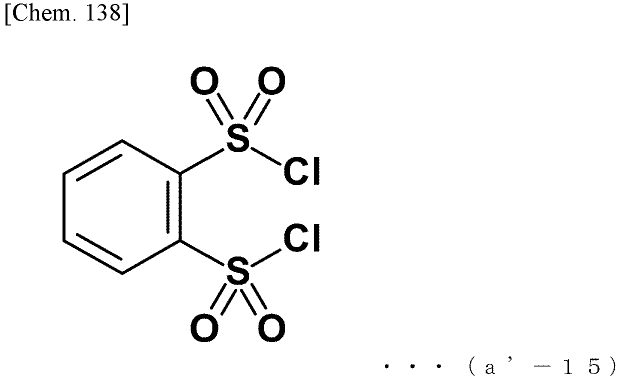

### [Comparative Example 16]

The reaction was performed using a primary alkylamine represented by Formula (b-1), tetrabutylammonium difluorotriphenylsilicate (TBAT) as a nucleophile, and 1,2-benzenedisulfonyl chloride represented by Formula (a'-15) in place of 1,1,2,2,3,3-hexafluoropropane-1,3-disulfonyl fluoride. A fluorinated alkyl compound represented by the above (d-4) was not produced.

### INDUSTRIAL APPLICABILITY

According to the present invention, it is possible to provide an amino group eliminating agent, a reaction product production method, a reaction precursor, a reaction precursor production method, and an optically active reaction product production method, which are used to efficiently produce a reaction product by reacting an organic compound having an amino group with an amino group eliminating agent and additionally reacting the organic compound with a nucleophile. Therefore, the present invention is industrially applicable.

## Claims

1. An amino group eliminating agent containing a compound (a) represented by the following Formula (a), which is used to produce a reaction product obtained by reacting an organic compound having an amino group with the amino group eliminating agent and additionally reacting the organic compound with a nucleophile:
Y¹-X¹-Rf-X²-Y² ··· (a)
in Formula (a), Rf is a substituted or unsubstituted divalent hydrocarbon group containing at least one fluorine atom; X¹ and X² are each independently a carbonyl group or a sulfonyl group; and Y¹ and Y² are each independently a halogen atom, a substituted or unsubstituted alkylcarbonyloxy group, a substituted or unsubstituted arylcarbonyloxy group, a substituted or unsubstituted alkylsulfonyloxy group, or a substituted or unsubstituted arylsulfonyloxy group.

2. The amino group eliminating agent according to claim 1, wherein the organic compound is represented by the following Formula (b): 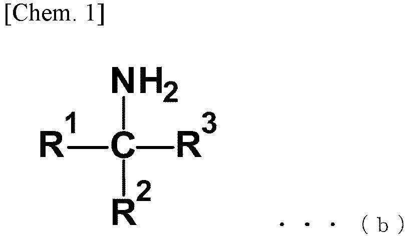 in Formula (b), R¹, R² and R³ are each independently a hydrogen atom, a polar group, a substituted or unsubstituted saturated hydrocarbon group, a substituted or unsubstituted unsaturated hydrocarbon group, or a substituted or unsubstituted aromatic hydrocarbon group, and at least two of R¹, R² and R³ may be bonded to each other to form a ring.

3. The amino group eliminating agent according to claim 1,
wherein the nucleophile is used to substitute the amino group, which has reacted with the amino group eliminating agent, with a chemical species, and
the chemical species includes at least one selected from the group consisting of a hydrogen atom, a hydroxy group, a halogen atom, a substituted or unsubstituted alkoxy group, a substituted or unsubstituted aryloxy group, an azide group, a substituted or unsubstituted amino group, a substituted or unsubstituted aromatic heterocyclic group, a substituted or unsubstituted alkylthio group, a substituted or unsubstituted arylthio group, a cyano group, a substituted or unsubstituted phosphino group, a substituted or unsubstituted amide group, a substituted or unsubstituted sulfonamide group, and a substituted or unsubstituted hydrocarbon group.

4. The amino group eliminating agent according to any one of claims 1 to 3, wherein the reaction product is represented by the following Formula (d): 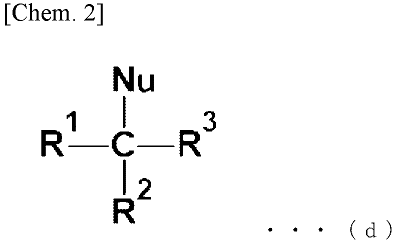 in Formula (d), R¹, R² and R³ are each independently a hydrogen atom, a polar group, a substituted or unsubstituted saturated hydrocarbon group, a substituted or unsubstituted unsaturated hydrocarbon group, or a substituted or unsubstituted aromatic hydrocarbon group, and at least two of R¹, R² and R³ may be bonded to each other to form a ring; and Nu is a hydrogen atom, a hydroxy group, a halogen atom, a substituted or unsubstituted alkoxy group, a substituted or unsubstituted aryloxy group, an azide group, a substituted or unsubstituted amino group, a substituted or unsubstituted aromatic heterocyclic group, a substituted or unsubstituted alkylthio group, a substituted or unsubstituted arylthio group, a cyano group, a substituted or unsubstituted phosphino group, a substituted or unsubstituted amide group, a substituted or unsubstituted sulfonamide group, or a substituted or unsubstituted hydrocarbon group.

5. A reaction product production method, comprising
reacting an organic compound having an amino group with an amino group eliminating agent containing a compound (a) represented by the following Formula (a) and additionally reacting the organic compound with a nucleophile:
Y¹-X¹-Rf-X²-Y² ··· (a)
in Formula (a), Rf is a substituted or unsubstituted divalent hydrocarbon group containing at least one fluorine atom; X¹ and X² are each independently a carbonyl group or a sulfonyl group; and Y¹ and Y² are each independently a halogen atom, a substituted or unsubstituted alkylcarbonyloxy group, a substituted or unsubstituted arylcarbonyloxy group, a substituted or unsubstituted alkylsulfonyloxy group, or a substituted or unsubstituted arylsulfonyloxy group.

6. The reaction product production method according to claim 5, wherein the organic compound is represented by the following Formula (b): 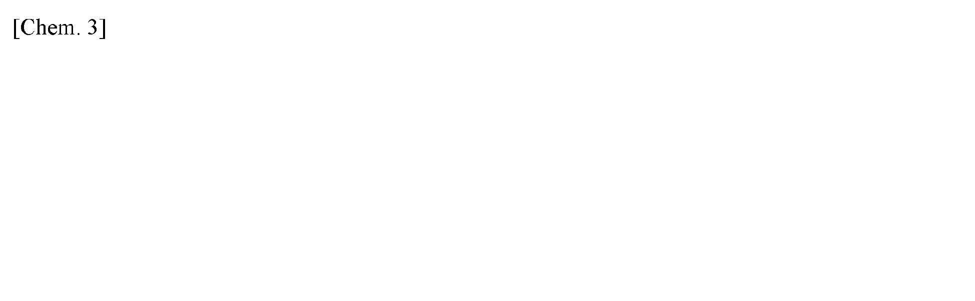 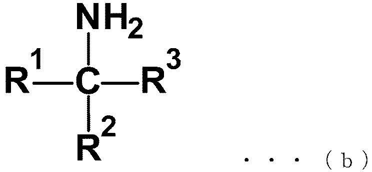 in Formula (b), R¹, R² and R³ are each independently a hydrogen atom, a polar group, a substituted or unsubstituted saturated hydrocarbon group, a substituted or unsubstituted unsaturated hydrocarbon group, or a substituted or unsubstituted aromatic hydrocarbon group, and at least two of R¹, R² and R³ may be bonded to each other to form a ring.

7. The reaction product production method according to claim 5,
wherein the nucleophile is used to substitute the amino group, which has reacted with the amino group eliminating agent, with a chemical species, and
the chemical species includes at least one selected from the group consisting of a hydrogen atom, a hydroxy group, a halogen atom, a substituted or unsubstituted alkoxy group, a substituted or unsubstituted aryloxy group, an azide group, a substituted or unsubstituted amino group, a substituted or unsubstituted aromatic heterocyclic group, a substituted or unsubstituted alkylthio group, a substituted or unsubstituted arylthio group, a cyano group, a substituted or unsubstituted phosphino group, a substituted or unsubstituted amide group, a substituted or unsubstituted sulfonamide group, and a substituted or unsubstituted hydrocarbon group.

8. The reaction product production method according to any one of claims 5 to 7, wherein the reaction product is represented by the following Formula (d): 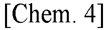 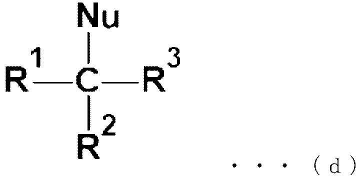 in Formula (d), R¹, R² and R³ are each independently a hydrogen atom, a polar group, a substituted or unsubstituted saturated hydrocarbon group, a substituted or unsubstituted unsaturated hydrocarbon group, or a substituted or unsubstituted aromatic hydrocarbon group, and at least two of R¹, R² and R³ may be bonded to each other to form a ring; and Nu is a hydrogen atom, a hydroxy group, a halogen atom, a substituted or unsubstituted alkoxy group, a substituted or unsubstituted aryloxy group, an azide group, a substituted or unsubstituted amino group, a substituted or unsubstituted aromatic heterocyclic group, a substituted or unsubstituted alkylthio group, a substituted or unsubstituted arylthio group, a cyano group, a substituted or unsubstituted phosphino group, a substituted or unsubstituted amide group, a substituted or unsubstituted sulfonamide group, or a substituted or unsubstituted hydrocarbon group.

9. A reaction precursor containing a compound (e) represented by the following Formula (e) or a compound (f) represented by the following Formula (f), which is used to obtain a reaction product by reacting with a nucleophile: 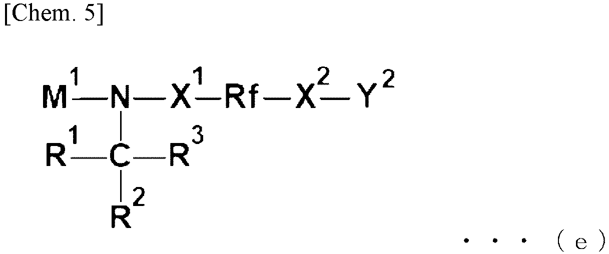 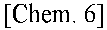 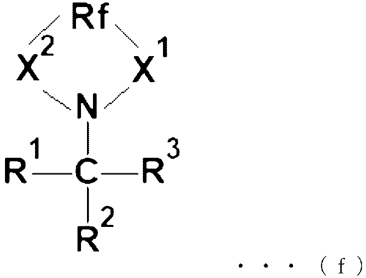 in Formulae (e) and (f), Rf is a substituted or unsubstituted divalent hydrocarbon group containing at least one fluorine atom; X¹ and X² are each independently a carbonyl group or a sulfonyl group; Y² is a halogen atom, a substituted or unsubstituted alkylcarbonyloxy group, a substituted or unsubstituted arylcarbonyloxy group, a substituted or unsubstituted alkylsulfonyloxy group, or a substituted or unsubstituted arylsulfonyloxy group; M¹ is a hydrogen atom or a counter ion; and R¹, R² and R³ are each independently a hydrogen atom, a polar group, a substituted or unsubstituted saturated hydrocarbon group, a substituted or unsubstituted unsaturated hydrocarbon group, or a substituted or unsubstituted aromatic hydrocarbon group, and at least two of R¹, R² and R³ may be bonded to each other to form a ring.

10. The reaction precursor according to claim 9,
wherein the nucleophile is used to substitute a tertiary amino group in Formulae (e) and (f) with a chemical species, and
the chemical species includes at least one chemical species selected from the group consisting of a hydrogen atom, a hydroxy group, a halogen atom, a substituted or unsubstituted alkoxy group, a substituted or unsubstituted aryloxy group, an azide group, a substituted or unsubstituted amino group, a substituted or unsubstituted aromatic heterocyclic group, a substituted or unsubstituted alkylthio group, a substituted or unsubstituted arylthio group, a cyano group, a substituted or unsubstituted phosphino group, a substituted or unsubstituted amide group, a substituted or unsubstituted sulfonamide group, and a substituted or unsubstituted hydrocarbon group.

11. The reaction precursor according to claim 9 or 10, wherein the reaction product is represented by the following Formula (d): 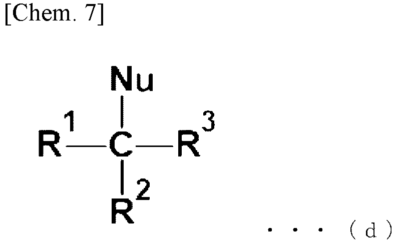 in Formula (d), R¹, R² and R³ are each independently a hydrogen atom, a polar group, a substituted or unsubstituted saturated hydrocarbon group, a substituted or unsubstituted unsaturated hydrocarbon group, or a substituted or unsubstituted aromatic hydrocarbon group, and at least two of R¹, R² and R³ may be bonded to each other to form a ring; and Nu is a hydrogen atom, a hydroxy group, a halogen atom, a substituted or unsubstituted alkoxy group, a substituted or unsubstituted aryloxy group, an azide group, a substituted or unsubstituted amino group, a substituted or unsubstituted aromatic heterocyclic group, a substituted or unsubstituted alkylthio group, a substituted or unsubstituted arylthio group, a cyano group, a substituted or unsubstituted phosphino group, a substituted or unsubstituted amide group, a substituted or unsubstituted sulfonamide group, or a substituted or unsubstituted hydrocarbon group.

12. A method of producing a reaction precursor containing a compound (e) represented by the following Formula (e) or a compound (f) represented by the following Formula (f), which is used to obtain a reaction product by reacting with a nucleophile, comprising
reacting an organic compound having an amino group with an amino group eliminating agent containing a compound (a) represented by the following Formula (a):
Y¹-X¹-Rf-X²-Y² ··· (a)
in Formula (a), Rf is a substituted or unsubstituted divalent hydrocarbon group containing at least one fluorine atom; X¹ and X² are each independently a carbonyl group or a sulfonyl group; and Y¹ and Y² are each independently a halogen atom, a substituted or unsubstituted alkylcarbonyloxy group, a substituted or unsubstituted arylcarbonyloxy group, a substituted or unsubstituted alkylsulfonyloxy group, or a substituted or unsubstituted arylsulfonyloxy group. 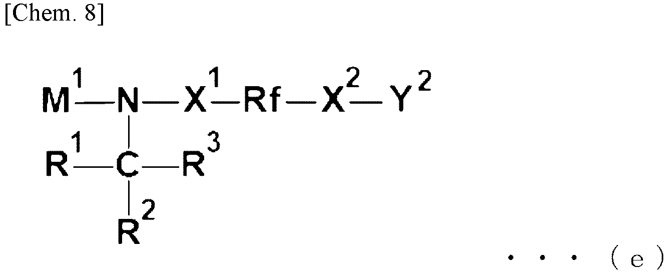 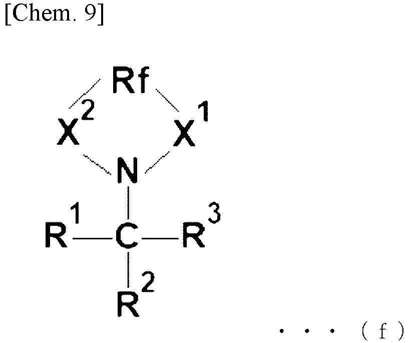 in Formulae (e) and (f), Rf is a substituted or unsubstituted divalent hydrocarbon group containing at least one fluorine atom; X¹ and X² are each independently a carbonyl group or a sulfonyl group; Y² is a halogen atom, a substituted or unsubstituted alkylcarbonyloxy group, a substituted or unsubstituted arylcarbonyloxy group, a substituted or unsubstituted alkylsulfonyloxy group, or a substituted or unsubstituted arylsulfonyloxy group; M¹ is a hydrogen atom or a counter ion; and R¹, R² and R³ are each independently a hydrogen atom, a polar group, a substituted or unsubstituted saturated hydrocarbon group, a substituted or unsubstituted unsaturated hydrocarbon group, or a substituted or unsubstituted aromatic hydrocarbon group, and at least two of R¹, R² and R³ may be bonded to each other to form a ring.

13. The reaction precursor production method according to claim 12, wherein the organic compound is represented by the following Formula (b): 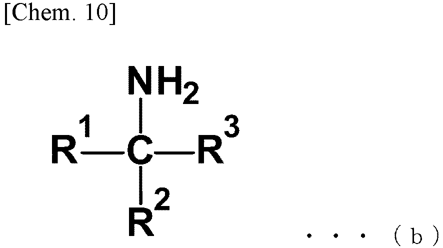 in Formula (b), R¹, R² and R³ are each independently a hydrogen atom, a polar group, a substituted or unsubstituted saturated hydrocarbon group, a substituted or unsubstituted unsaturated hydrocarbon group, or a substituted or unsubstituted aromatic hydrocarbon group, and at least two of R¹, R² and R³ may be bonded to each other to form a ring.

14. The reaction precursor production method according to claim 12,
wherein the nucleophile is used to substitute the amino group, which has reacted with the amino group eliminating agent, with a chemical species, and
the chemical species includes at least one chemical species selected from the group consisting of a hydrogen atom, a hydroxy group, a halogen atom, a substituted or unsubstituted alkoxy group, a substituted or unsubstituted aryloxy group, an azide group, a substituted or unsubstituted amino group, a substituted or unsubstituted aromatic heterocyclic group, a substituted or unsubstituted alkylthio group, a substituted or unsubstituted arylthio group, a cyano group, a substituted or unsubstituted phosphino group, a substituted or unsubstituted amide group, a substituted or unsubstituted sulfonamide group, and a substituted or unsubstituted hydrocarbon group.

15. The reaction precursor production method according to any one of claims 12 to 14, wherein the reaction product is represented by the following Formula (d): 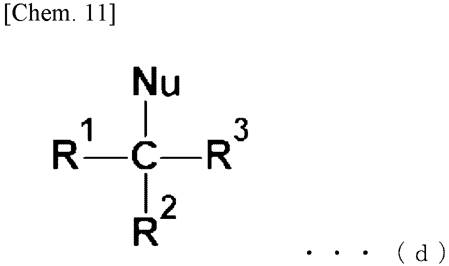 in Formula (d), R¹, R² and R³ are each independently a hydrogen atom, a polar group, a substituted or unsubstituted saturated hydrocarbon group, a substituted or unsubstituted unsaturated hydrocarbon group, or a substituted or unsubstituted aromatic hydrocarbon group, and at least two of R¹, R² and R³ may be bonded to each other to form a ring; and Nu is a hydrogen atom, a hydroxy group, a halogen atom, a substituted or unsubstituted alkoxy group, a substituted or unsubstituted aryloxy group, an azide group, a substituted or unsubstituted amino group, a substituted or unsubstituted aromatic heterocyclic group, a substituted or unsubstituted alkylthio group, a substituted or unsubstituted arylthio group, a cyano group, a substituted or unsubstituted phosphino group, a substituted or unsubstituted amide group, a substituted or unsubstituted sulfonamide group, or a substituted or unsubstituted hydrocarbon group.

16. An optically active reaction product production method, comprising
reacting an optically active organic compound having an amino group with an amino group eliminating agent containing a compound (a) represented by the following Formula (a) and additionally reacting the optically active organic compound with a nucleophile:
Y¹-X¹-Rf-X²-Y² ··· (a)
in Formula (a), Rf is a substituted or unsubstituted divalent hydrocarbon group containing at least one fluorine atom; X¹ and X² are each independently a carbonyl group or a sulfonyl group; and Y¹ and Y² are each independently a halogen atom, a substituted or unsubstituted alkylcarbonyloxy group, a substituted or unsubstituted arylcarbonyloxy group, a substituted or unsubstituted alkylsulfonyloxy group, or a substituted or unsubstituted arylsulfonyloxy group.

17. The optically active reaction product production method according to claim 16, wherein the optically active organic compound is the following Formula (b*): 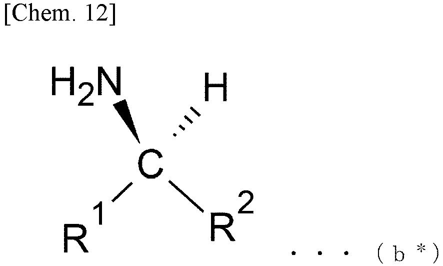 in Formula (b*), R¹ and R² are not the same and each independently a polar group, a substituted or unsubstituted saturated hydrocarbon group, a substituted or unsubstituted unsaturated hydrocarbon group, or a substituted or unsubstituted aromatic hydrocarbon group, and R¹ and R² may be bonded to each other to form a ring.

18. The optically active reaction product production method according to claim 16,
wherein the nucleophile is used to substitute the amino group, which has reacted with the amino group eliminating agent, with a chemical species, and
the chemical species includes at least one chemical species selected from the group consisting of a hydrogen atom, a hydroxy group, a halogen atom, a substituted or unsubstituted alkoxy group, a substituted or unsubstituted aryloxy group, an azide group, a substituted or unsubstituted amino group, a substituted or unsubstituted aromatic heterocyclic group, a substituted or unsubstituted alkylthio group, a substituted or unsubstituted arylthio group, a cyano group, a substituted or unsubstituted phosphino group, a substituted or unsubstituted amide group, a substituted or unsubstituted sulfonamide group, and a substituted or unsubstituted hydrocarbon group.

19. The optically active reaction product production method according to any one of claims 16 to 18, wherein the optically active reaction product is represented by the following Formula (d*): 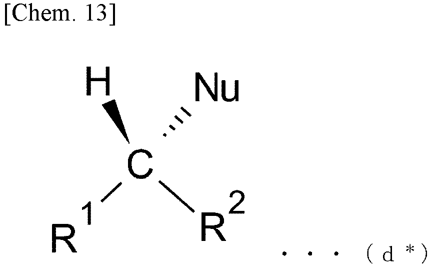 in Formula (d*), R¹ and R² are not the same and each independently a polar group, a substituted or unsubstituted saturated hydrocarbon group, a substituted or unsubstituted unsaturated hydrocarbon group, or a substituted or unsubstituted aromatic hydrocarbon group, and R¹ and R² may be bonded to each other to form a ring; and Nu is a hydrogen atom, a hydroxy group, a halogen atom, a substituted or unsubstituted alkoxy group, a substituted or unsubstituted aryloxy group, an azide group, a substituted or unsubstituted amino group, a substituted or unsubstituted aromatic heterocyclic group, a substituted or unsubstituted alkylthio group, a substituted or unsubstituted arylthio group, a cyano group, a substituted or unsubstituted phosphino group, a substituted or unsubstituted amide group, a substituted or unsubstituted sulfonamide group, or a substituted or unsubstituted hydrocarbon group.
